# FASCICULE DE BREVET EUROPEEN

(11) **EP 1 025 834 B1**
(45) Date de publication et mention de la délivrance du brevet: **04.07.2007**
(21) Numéro de dépôt: 99403290.2
(22) Date de dépôt: 27.12.1999
(51) Int. Cl.: A61K 8/49, A61Q 5/06

(54) **Utilisation pour la teinture directe des fibres kératiniques d'une association de deux colorants cationiques**
Verwendung von eine kombination von zwei kationische farbestoffe zur direktziehende haarfarbung
Use for direct hair dye a combination of two cationic dyes

(30) Priorité: 19.01.1999 FR 9900501
(43) Date de publication de la demande: 09.08.2000
(73) Titulaire: L'ORÉAL, 75008 Paris (FR)
(72) Inventeur: Rondeau, Christine, 78500 Sartrouville (FR)
(74) Mandataire: Miszputen, Laurent

(56) Documents cités:
- EP-A- 0 850 638
- WO-A-95/01772
- WO-A-97/39727
- WO-A-99/20234
- FR-A- 2 757 387
- GB-A- 2 057 019

## Description

L'invention a pour objet l'utilisation d'une association de deux colorants cationiques particuliers, à titre de colorants directs, dans des compositions destinées à la teinture directe des fibres kératiniques, en particulier des fibres kératiniques humaines telles que les cheveux, les compositions de teinture directe la contenant, les procédés de teinture directe correspondants et les dispositifs de teinture mettant en oeuvre lesdites compositions.

Dans le domaine capillaire, on peut distinguer deux types de coloration.

Le premier est la coloration permanente ou coloration d'oxydation qui utilise des colorants d'oxydation qui développent leur pouvoir tinctorial en présence d'agents oxydants.

Le second est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs ; ils peuvent être mis en oeuvre avec ou sans agent oxydant. En présence d'agent oxydant, la coloration est dite éclaircissante ; sans agent oxydant, c'est la teinture directe classique et non éclaircissante.
La présente invention concerne la coloration directe classique et donc sans agent oxydant, moins agressive pour les fibres kératiniques.

Parmi les colorants directs cationiques disponibles dans le domaine de la teinture des fibres kératiniques notamment humaines, on connaît déjà les composés de formules (I) à (III) dont la structure est développée dans le texte qui va suivre; néanmoins, ces colorants généralement appelés colorants ARIANORS, conduisent à des colorations qui présentent des caractéristiques encore insuffisantes, à la fois sur le plan de la puissance et de la chromaticité des nuances obtenues, sur le plan de l'homogénéité de la couleur répartie le long de la fibre (on dit alors que la coloration est trop sélective), et sur le plan de la tenacité, en terme de résistance aux diverses agressions que peuvent subir les cheveux (lumière, intempéries, shampooings). Par ailleurs, le document WO 95/01772 décrit des colorants cationiques de formules (IV) et (VI) telles que définies ci dessous pour la coloration des fibres kératiniques.

Or, la demanderesse vient maintenant de découvrir, de façon totalement inattendue et surprenante, qu'une association d'au moins un colorant cationique de formules (I) à (III) définies ci-après et d'au moins un colorant cationique de formules (IV) à (VII) définies ci-après, convient pour la teinture directe et qu'en outre elle permet d'obtenir des compositions tinctoriales conduisant à des colorations peu sélectives, très chromatiques et résistant bien aux diverses agressions que peuvent subir les cheveux.

Cette découverte est à la base de la présente invention.

La présente invention a donc pour premier objet, l'utilisation à titre de colorant direct dans des, ou pour la fabrication de, compositions de teinture directe pour fibres kératiniques et en particulier des fibres kératiniques humaines telles que les cheveux et qui ne contiennent pas de colorant autooxydable, d'une association comprenant, (i)au moins un colorant cationique choisi parmi (I) le Basic Brown 17, le Basic Brown 16, le Basic Red 76, le Basic Red 118 (II) le Basic Yellow 57, (III) le Basic Blue 99 et (ii)au moins un colorant cationique de formules (IV) et (VI) définies ci-après.

Les appellations du Color Index relatives aux colorants cationiques (I) à (III) recouvrent les structures chimiques suivantes (sous forme de chlorures) :
- 8-[(4-aminophényl)azo]-7-hydroxy-2- triméthylammonio-naphtalène,
- 8-[(2-méthoxyphényl)azo]-7-hydroxy-2-triméthylammonio-naphtalène,
- 8-[(4-amino-3-nitrophényl)azo]-7-hydroxy-2-triméthylammonio-naphtalène,
- 8-[(4-amino-2-nitrophényl)azo]-7-hydroxy-2-triméthylammonio-naphtalène,
- 3-[(3-méthyl-5-hydroxy-1-phényl-1 H-pyrazol-4-yl)azo]-triméthylammoniobenzène,
- 3-[(4-amino-6-bromo-5,8-dihydro-1-hydroxy-8-imino-5-oxo-2-naphtalényl)amino]-triméthylammonio-benzène,
- 3-[(3,7-dibromo-5,8-dihydro-4-hydroxy-5-imino-8-oxo-1-naphtalényl)- amino]triméthylammonio-benzène.
Lesdits composés (I) à (III) se retrouvent seuls ou en association dans les produits correspondant aux appellations commerciales, proposées par la société WARNER JENKINSON, suivantes :
- ARIANOR MAHOGANY ; ARIANOR STEEL BLUE ; ARIANOR MADDER RED ; ARIANOR SIENNA BROWN ; ARIANOR STRAW YELLOW ; ARIANOR BORDEAUX.

(ii) colorant cationique de formules (IV), (VI), suivantes :
a) les composés de formule (IV) suivante : 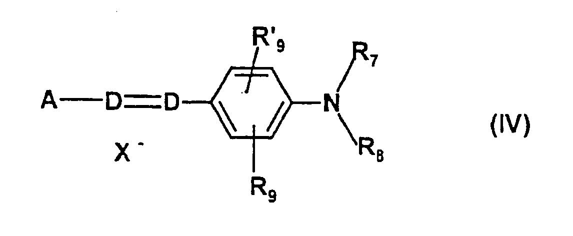 dans laquelle :
   D représente un atome d'azote ou le groupement -CH,
   R₇ et R₈, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₉ et R'₉, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
   X⁻ représente un anion de préférence choisi parmi le chlorure, le méthyl sulfate et l'acétate,
   A représente un groupement choisi par les structures A1 à A19 suivantes : 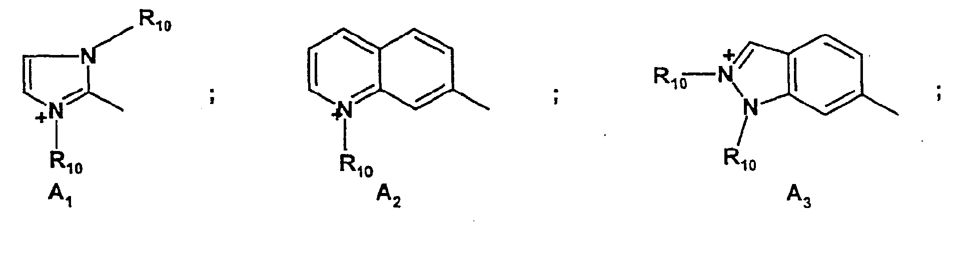 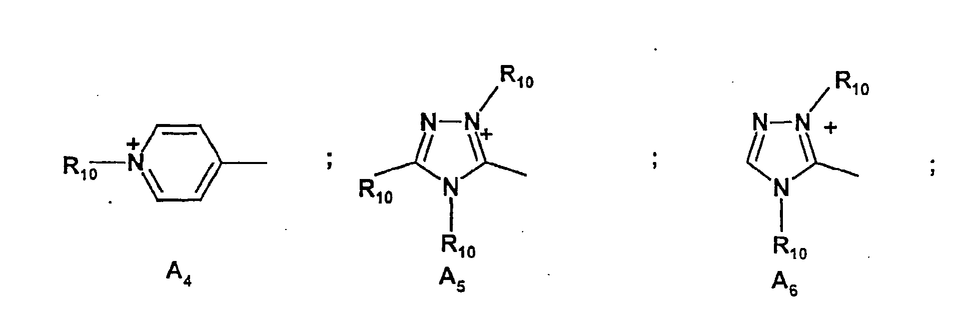 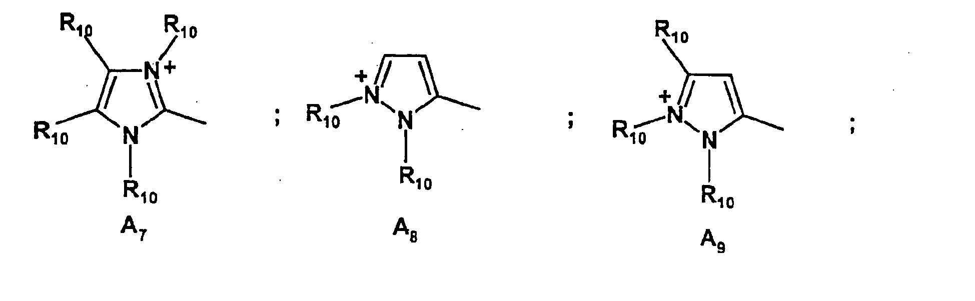 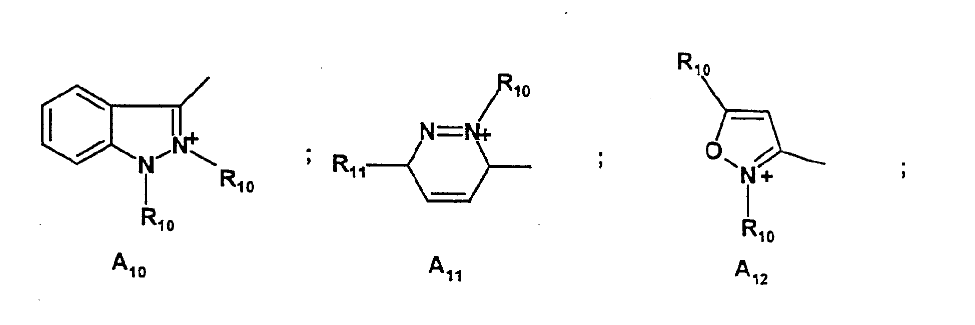 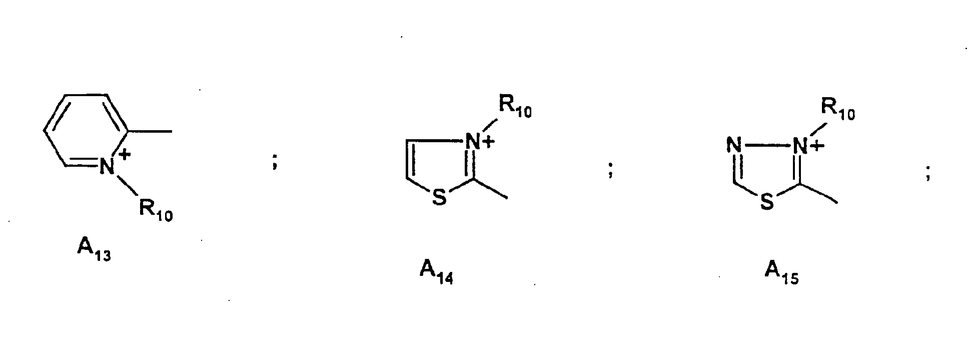 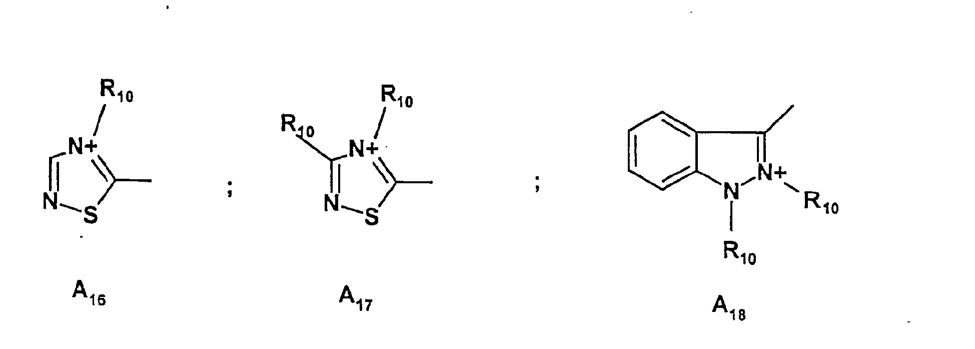 et 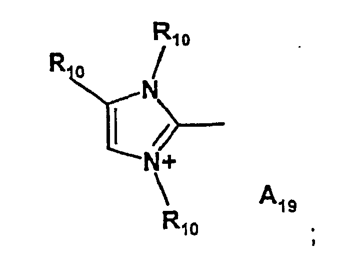
   dans lesquelles R₁₀ représente un radical alkyle en C₁-C₄ pouvant être substitué par un radical hydroxyle et R₁₁ représente un radical alcoxy en C₁-C₄, sous réserve que :
   (i) lorsque D représente -CH, que A représente A₄ ou A₁₃ et que R₉ est différent d'un radical alcoxy, alors R₇ et R₈ ne désignent pas simultanément un atome d'hydrogène ;
   (ii) lorsque D représente un atome d'azote, que A représente A₆, alors R₇ et R₈ ne désignent pas simultanément un radical méthyle.
c) les composés de formules (VI) suivantes : 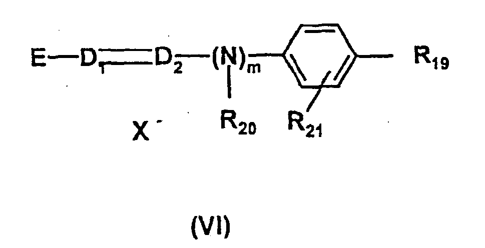
dans laquelle:
R₁₉ représente un atome d'hydrogène, un radical alcoxy en C₁-C₄, un atome d'halogène tel que le brome, le chlore, l'iode ou le fluor ou un radical amino,
R₂₀ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₂₁ représente un atome d'hydrogène ou d'halogène tel que le brome, le chlore, l'iode ou le fluor,
D₁ et D₂, identiques ou différents, représentent un atome d'azote ou le groupement -CH, m = 0 ou 1,
   étant entendu que lorsque R₁₉ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 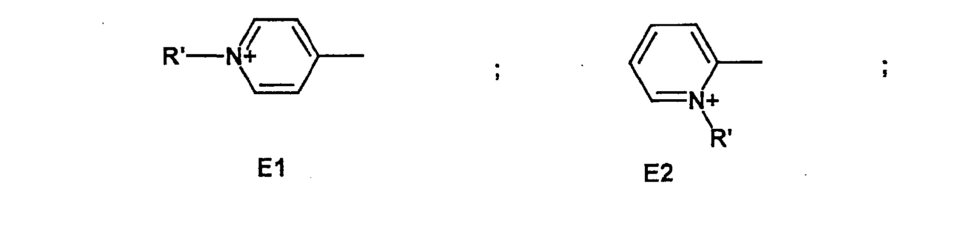 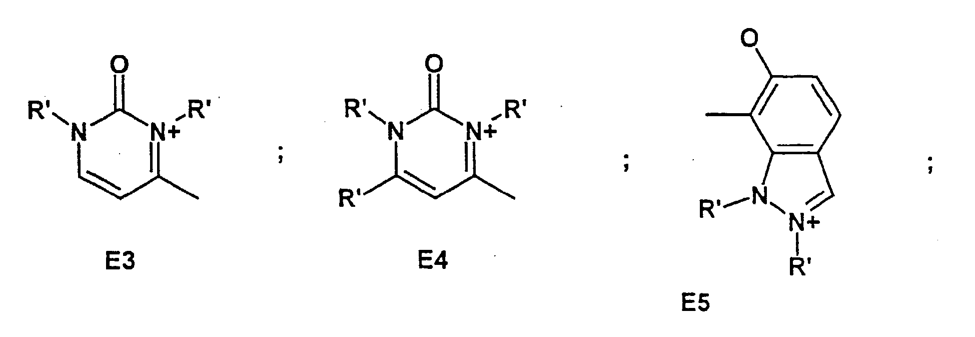 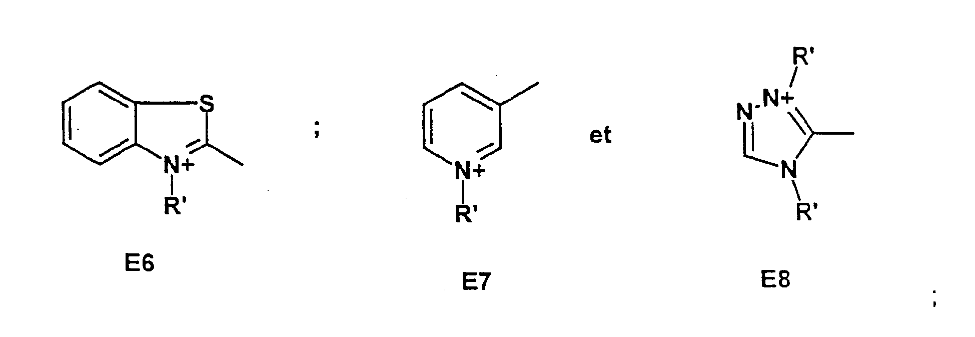
dans lesquelles R' représente un radical alkyle en C₁-C₄ ;
lorsque m = 0 et que D₁ représente un atome d'azote, alors E peut également désigner un groupement de structure E9 suivante : 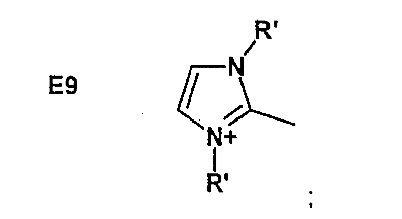 dans laquelle R' représente un radical alkyle en C₁-C₄. On peut également utiliser des composés correspondant aux formes mésomères de ces structures (IV) et (VI).

Dans les structures (IV) et (VI) comme dans les structures (I) à (III) définies ci-dessus le groupement alkyle ou alcoxy en C₁-C₄ désigne de préférence méthyle, éthyle, butyle, méthoxy, éthoxy.

Les colorants directs cationiques de formules (IV), (VI) utilisables dans les compositions tinctoriales conformes à l'invention, sont des composés connus et sont décrits par exemple dans les demandes de brevets WO 95/01772, WO 95/15144 et EP-A-0 714 954.

Parmi les colorants directs cationiques de formule (IV) utilisables dans les compositions tinctoriales conformes à l'invention, on peut plus particulièrement citer les composés répondant aux structures (IV1) à (IV54) suivantes :

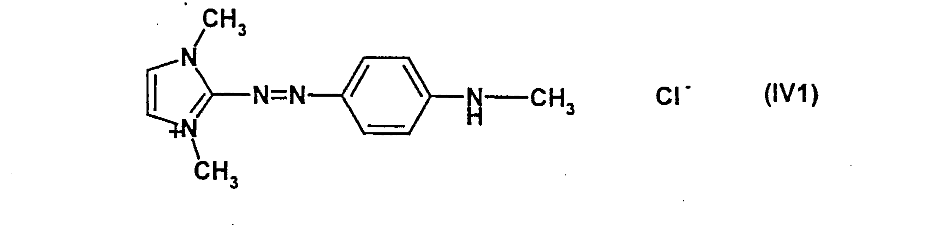

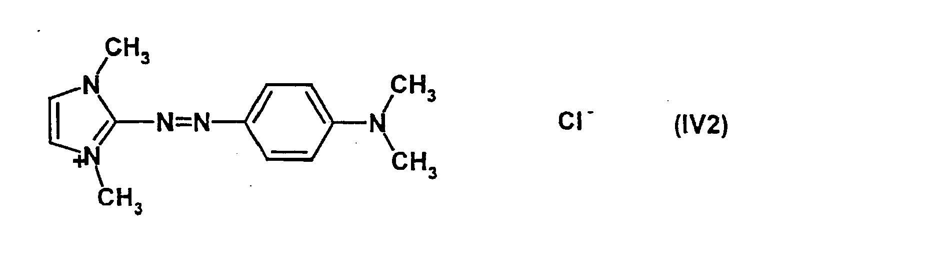

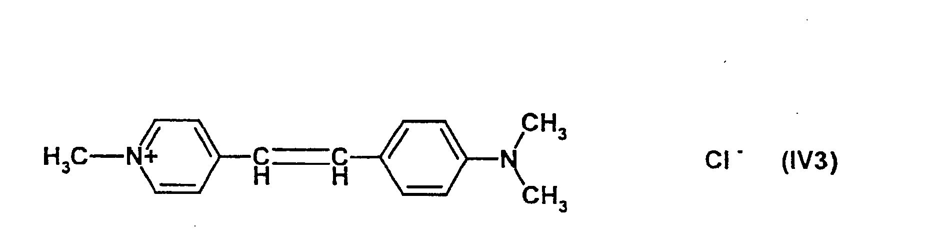

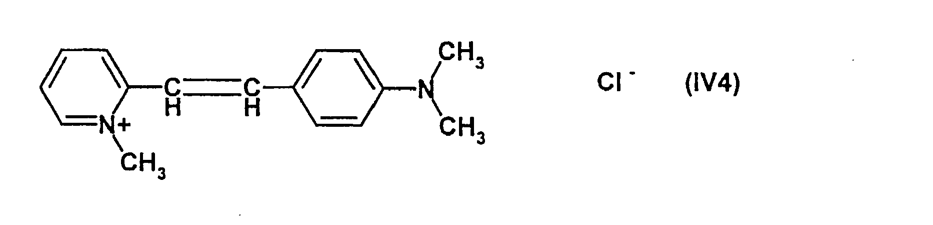

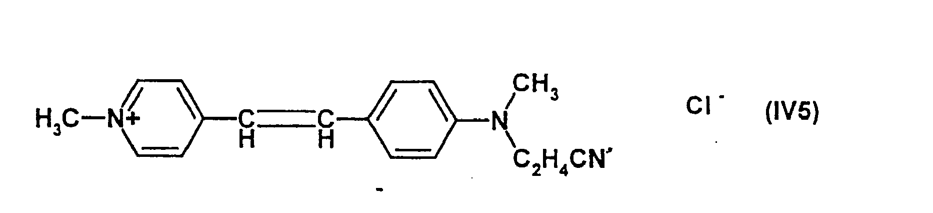

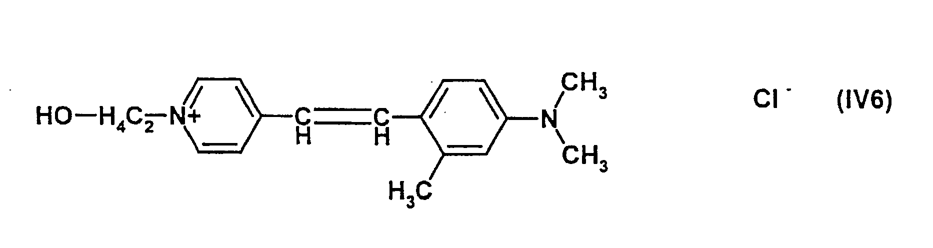

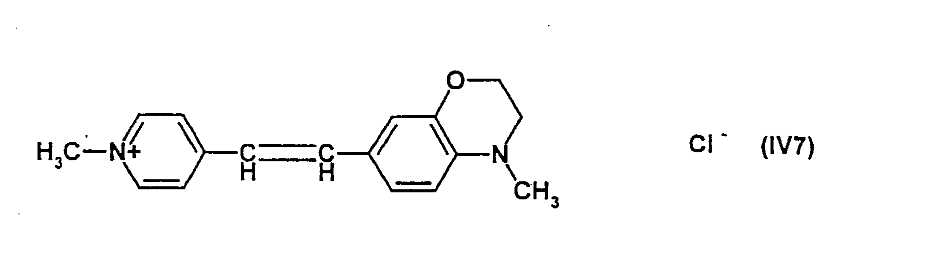

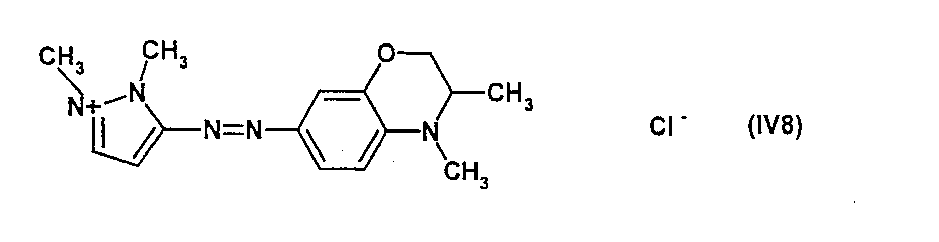

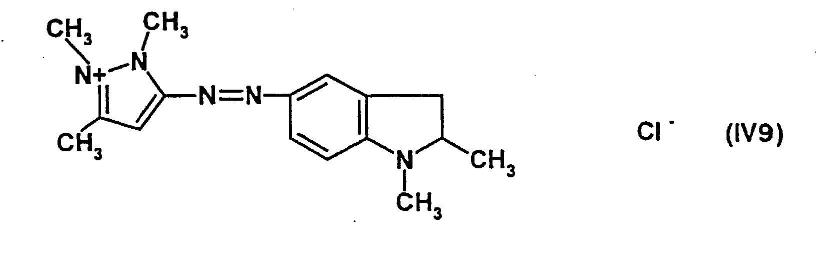

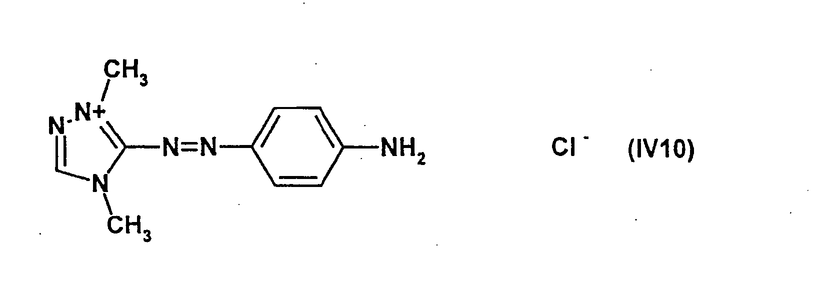

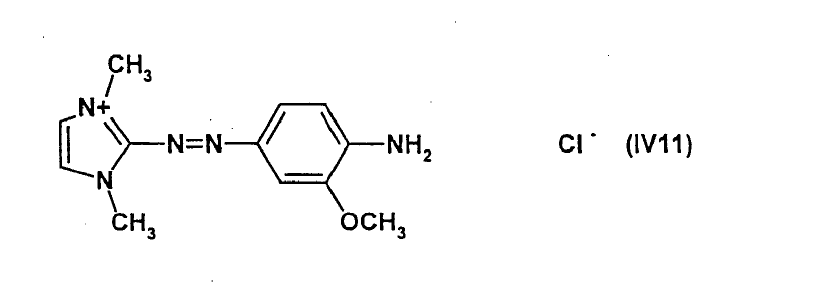

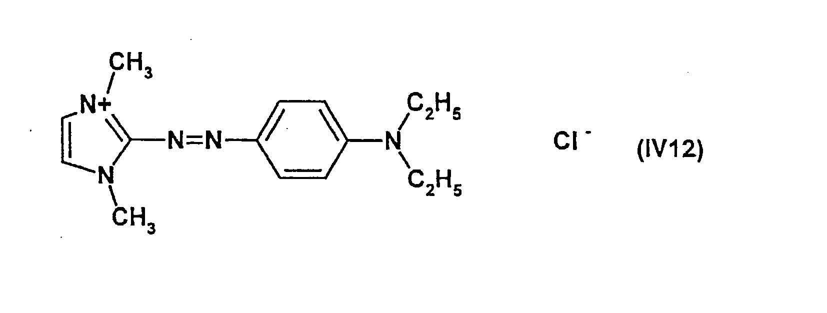

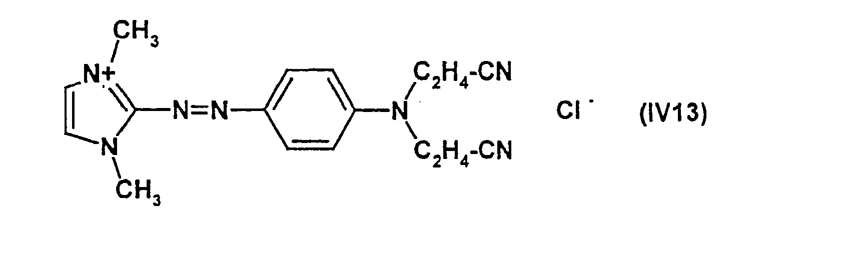

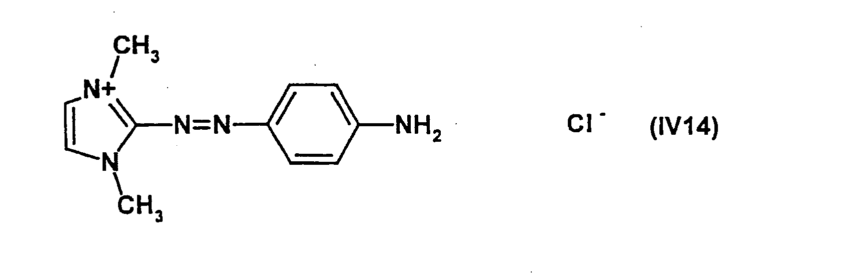

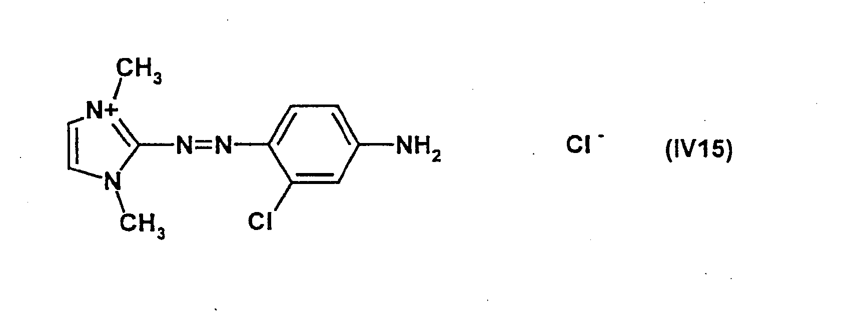

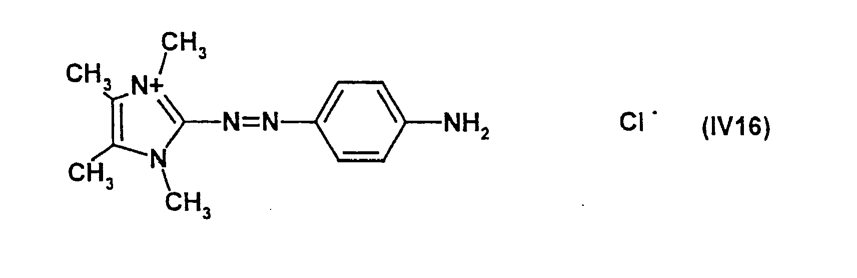

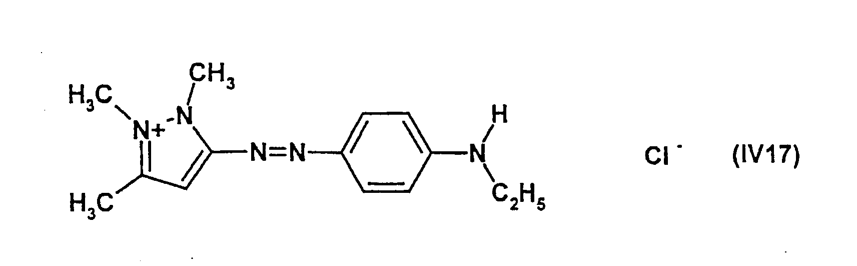

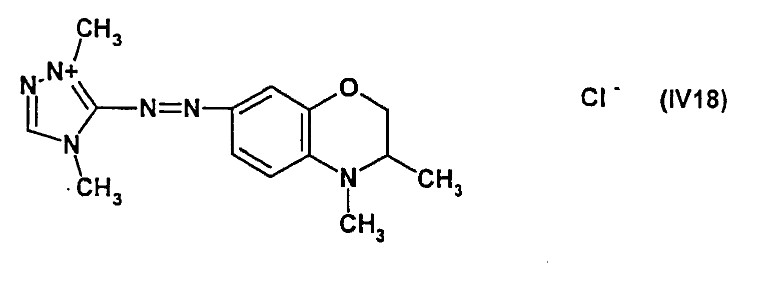

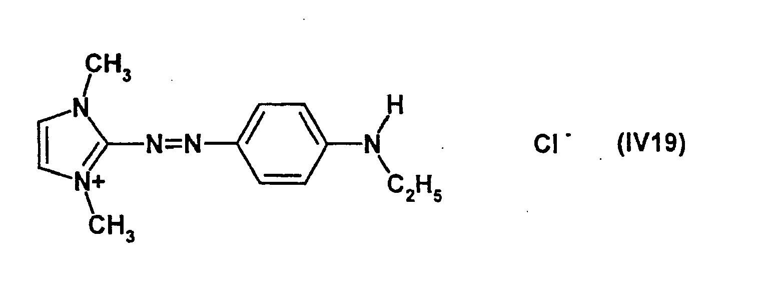

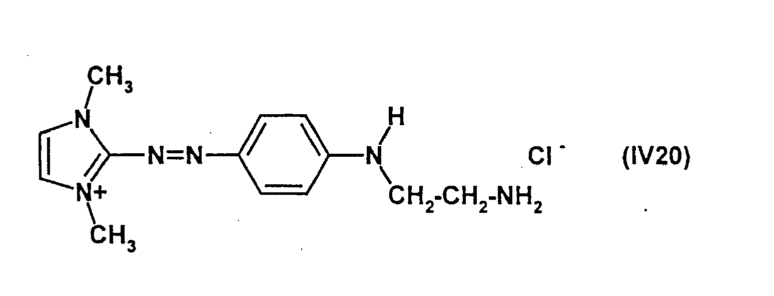

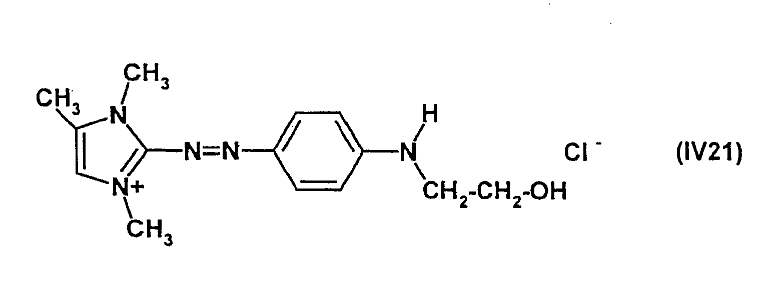

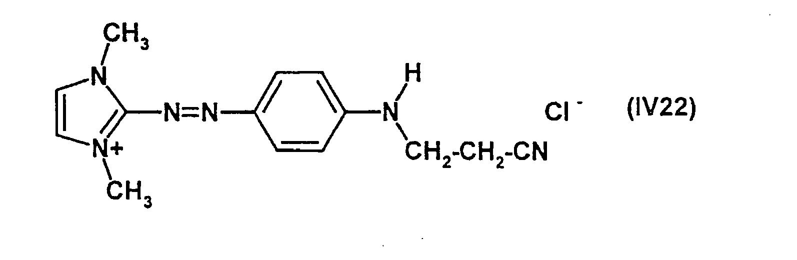

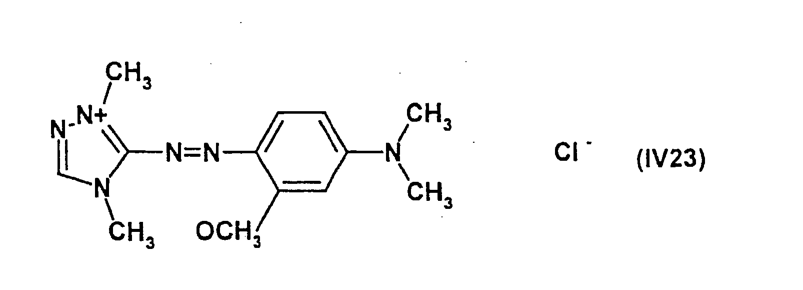

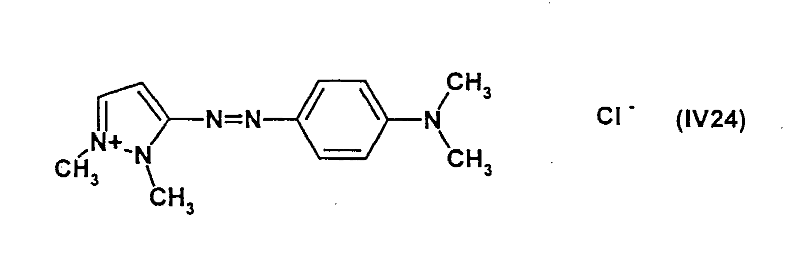

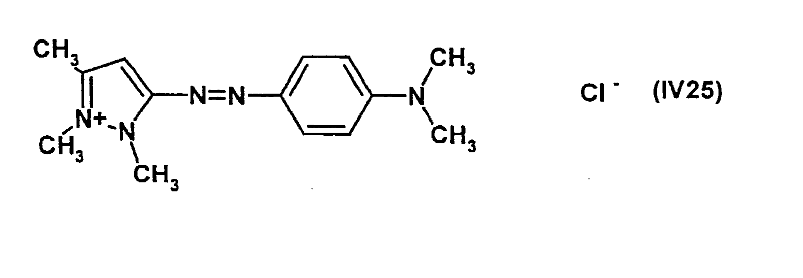

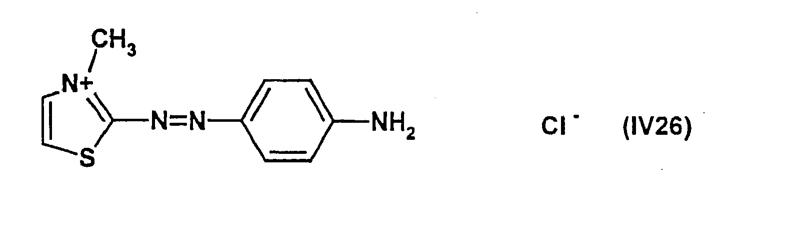

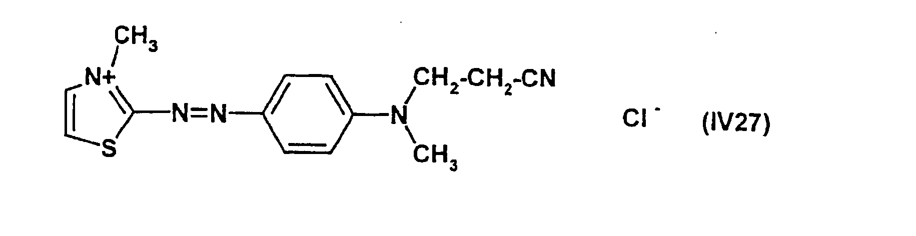

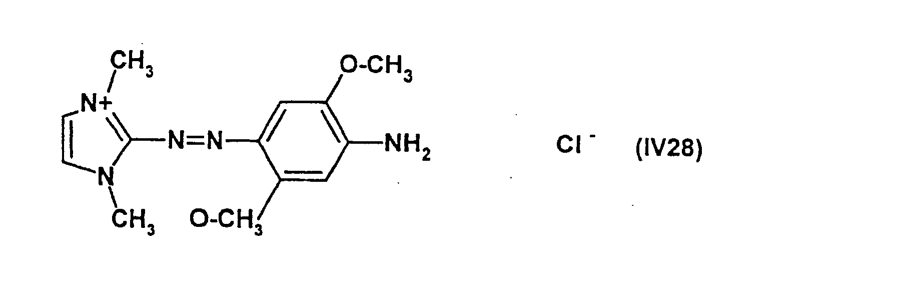

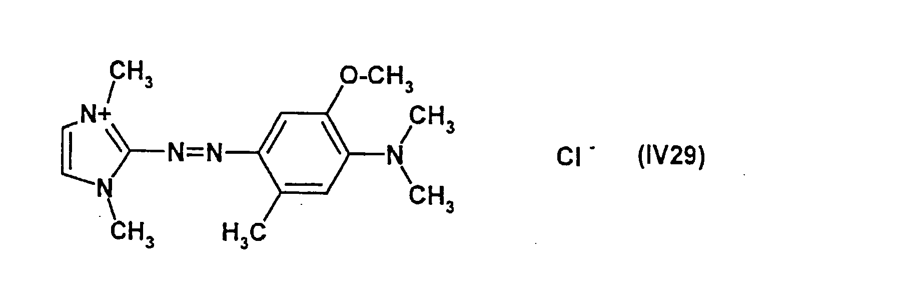

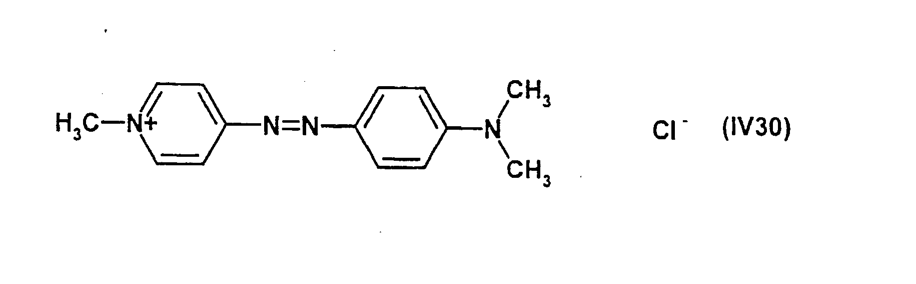

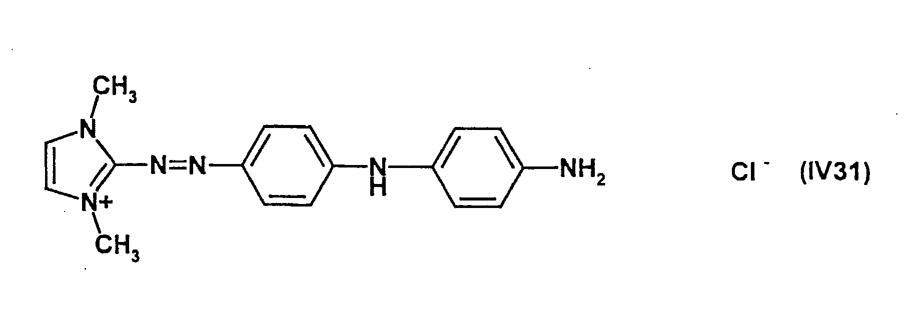

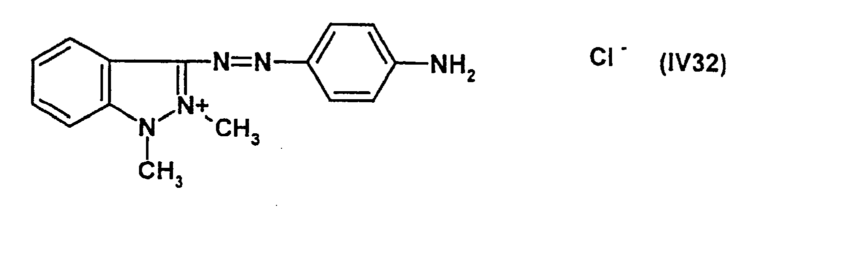

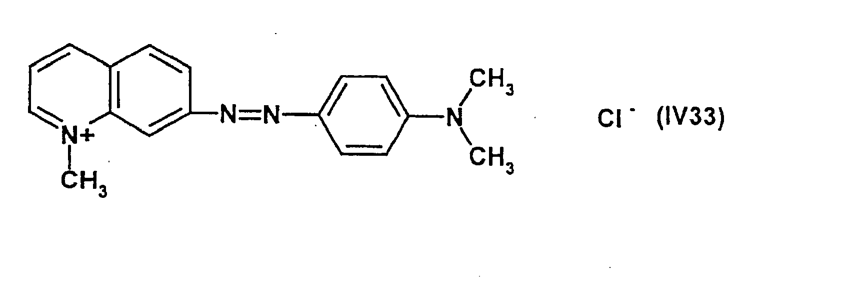

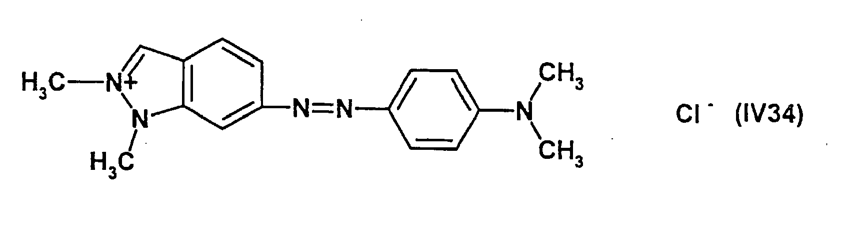

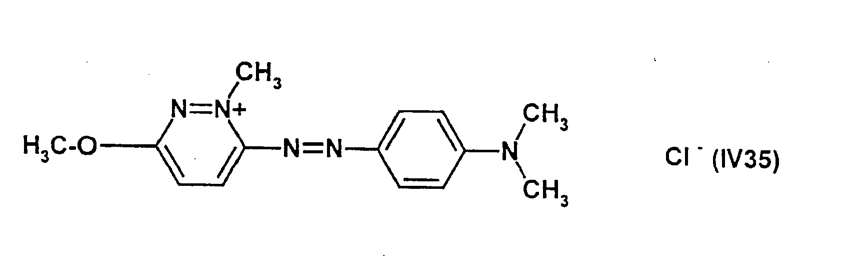

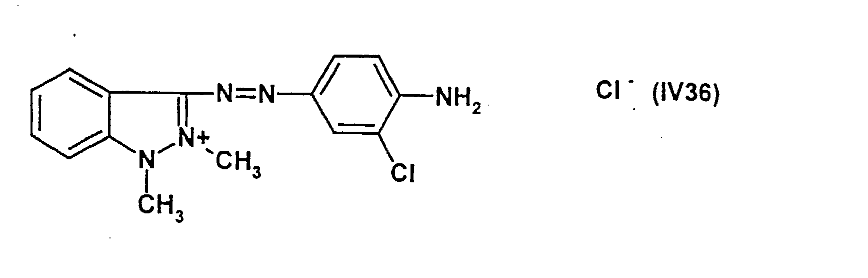

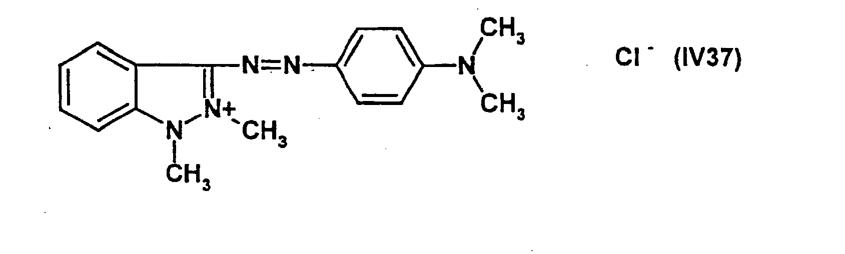

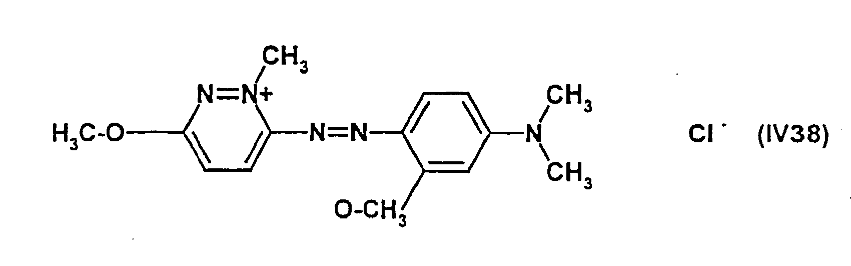

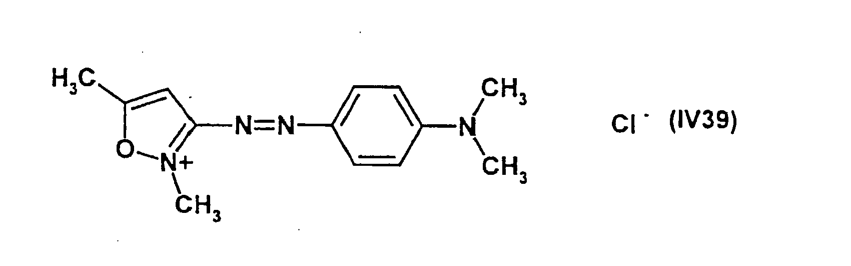

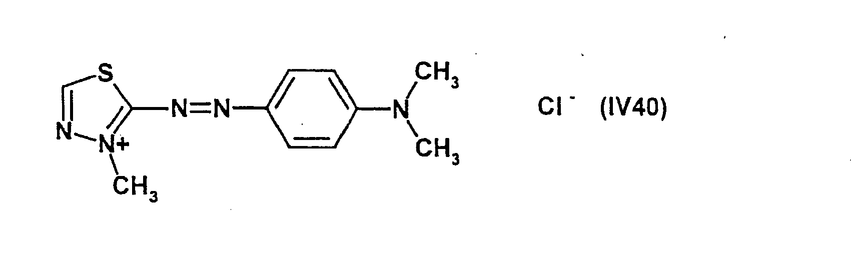

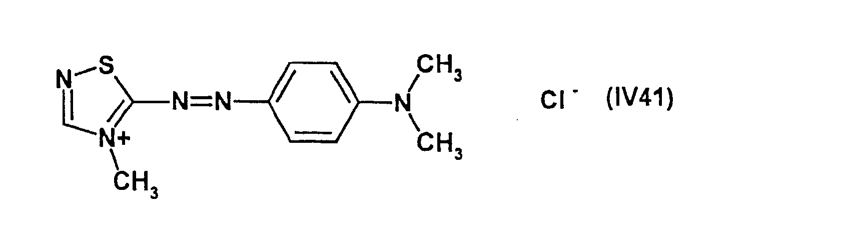

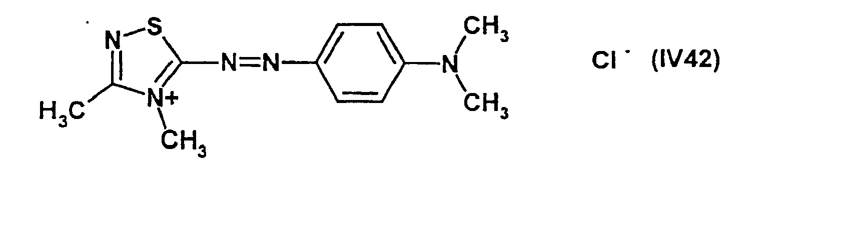

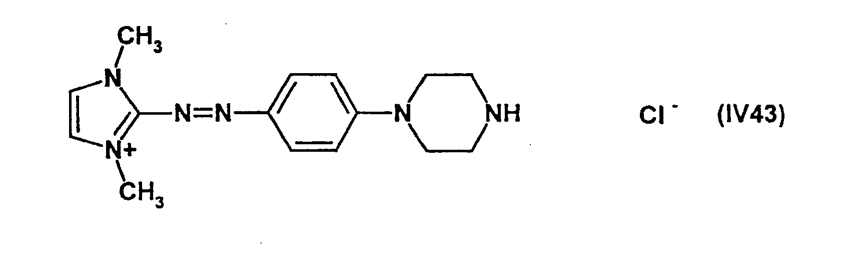

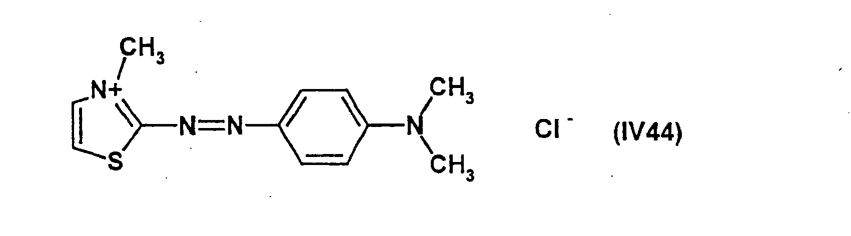

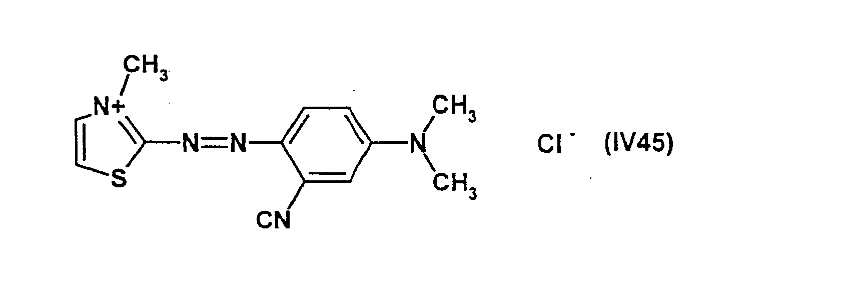

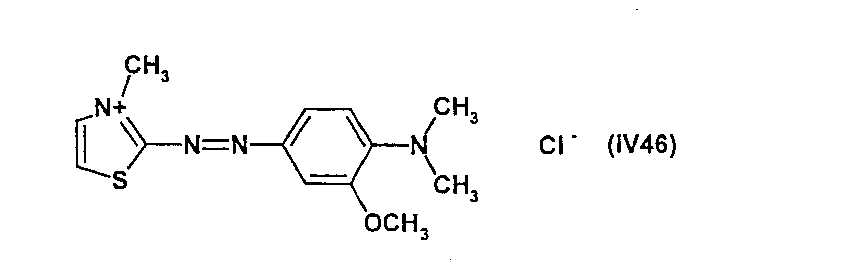

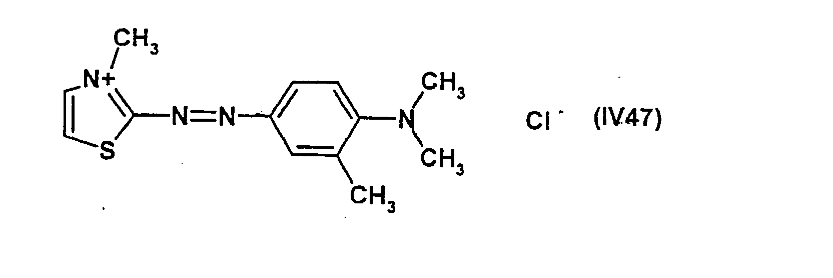

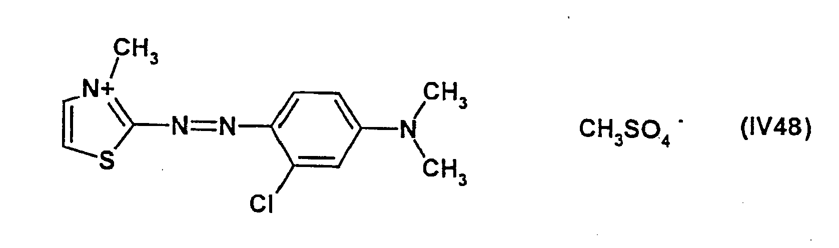

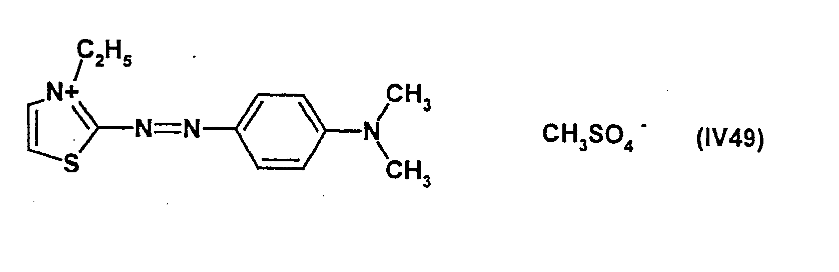

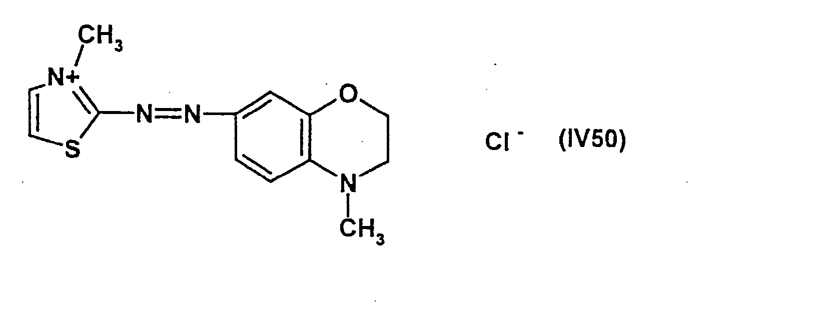

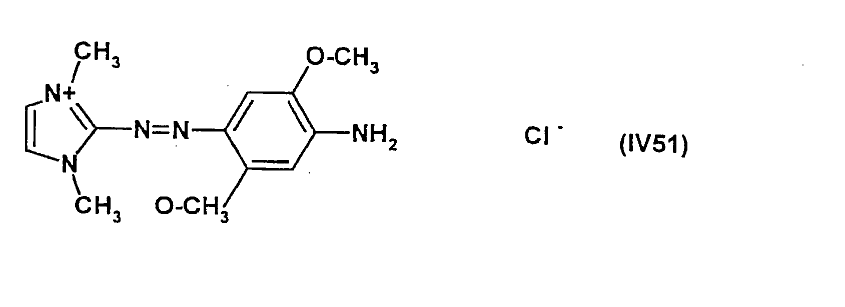

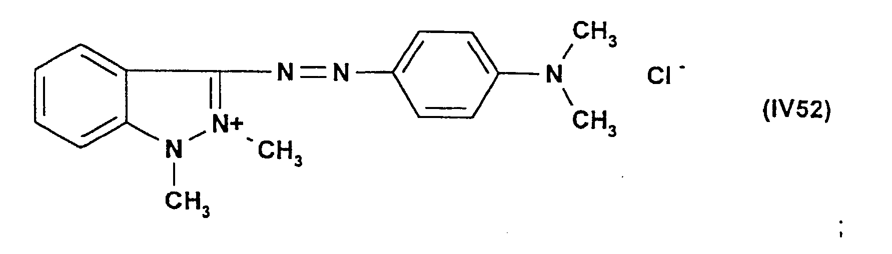

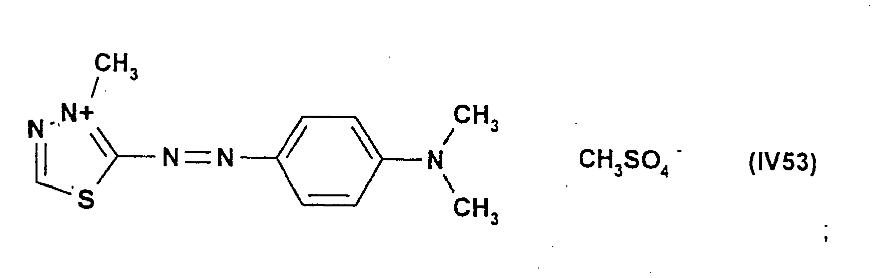

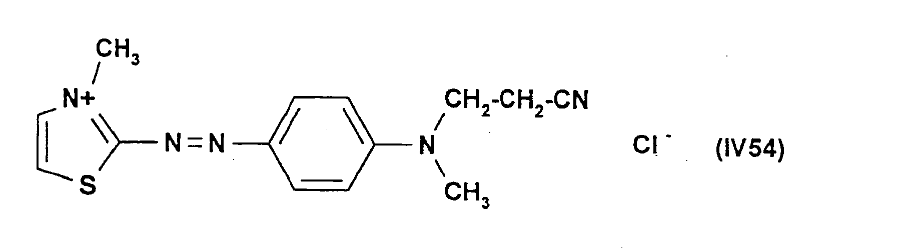

Parmi les composés de structures (IV1) à (IV54) décrits ci-dessus, on préfère tout particulièrement les composés répondant aux structures (IV1), (IV2), (IV14) et (IV31).

Parmi les colorants directs cationiques de formule (VI), utilisables dans les compositions tinctoriales conformes à l'invention, on peut plus particulièrement citer les composés répondant aux structures (VI1) à (VI18) suivantes :

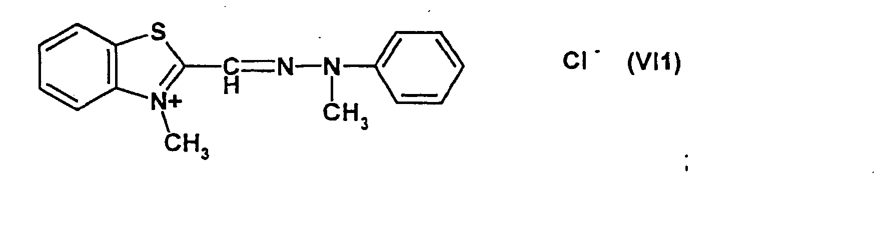

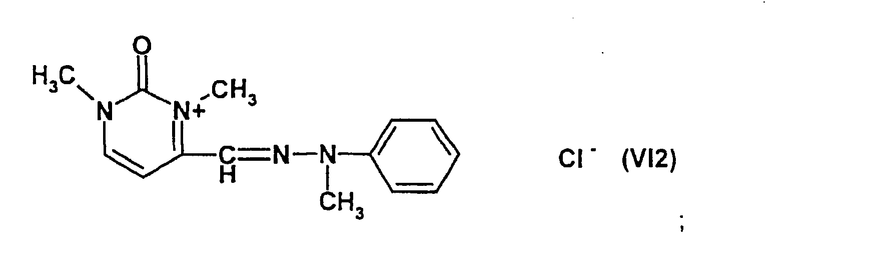

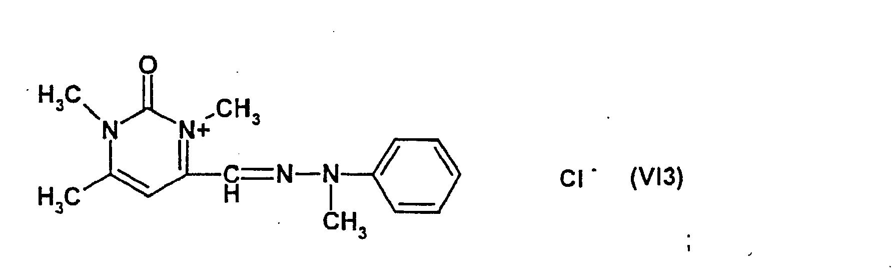

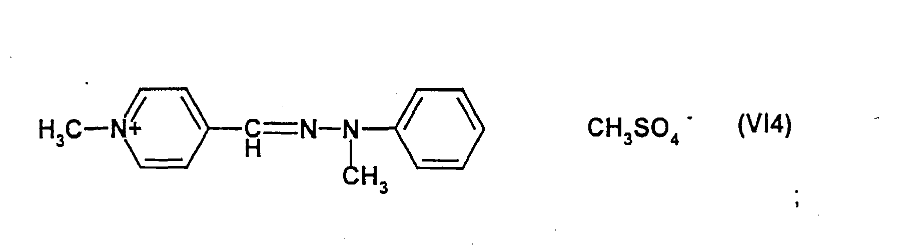

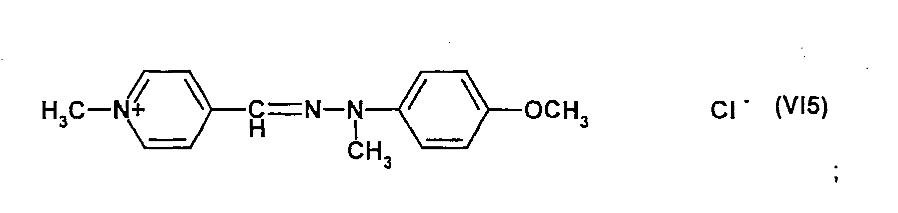

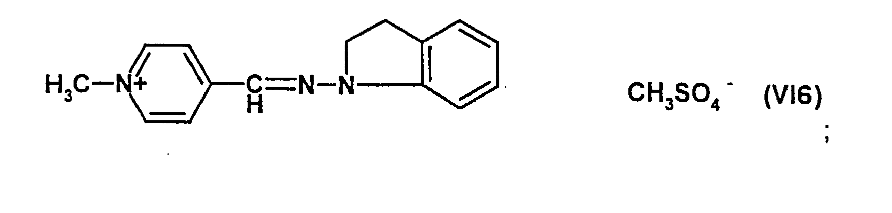

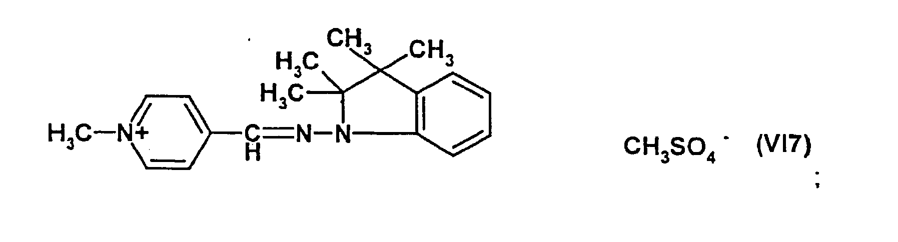

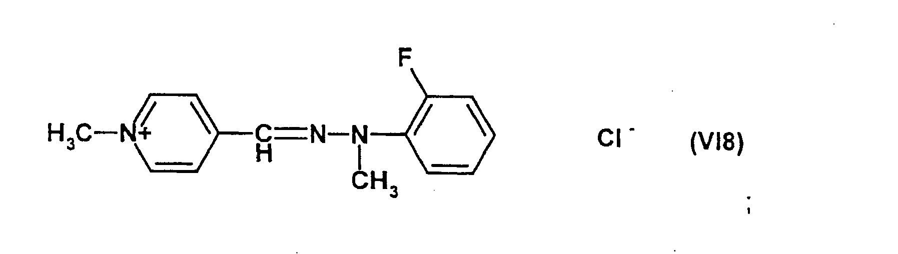

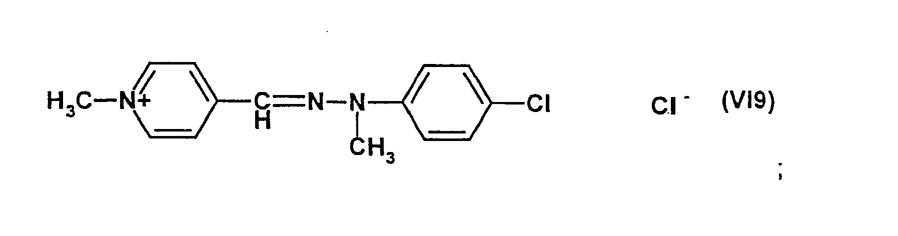

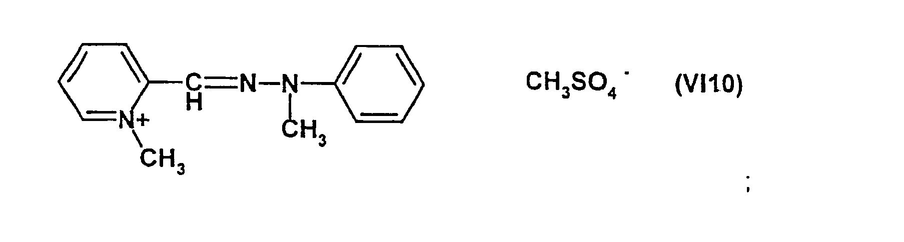

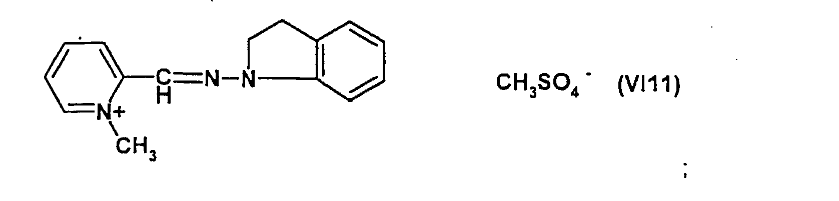

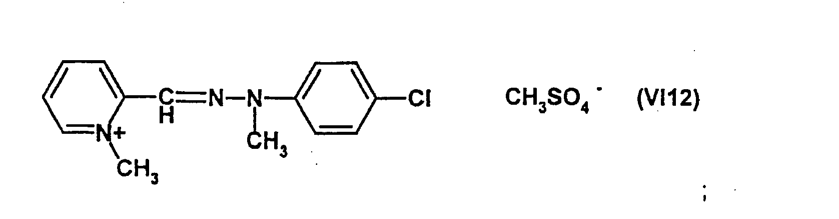

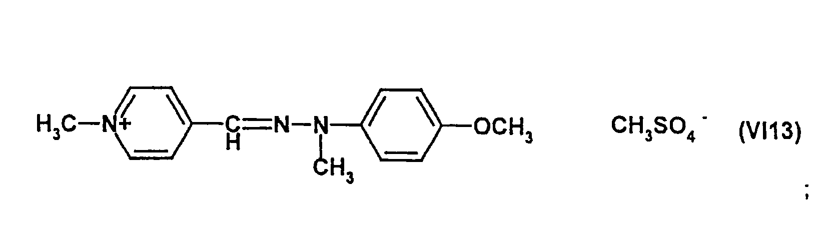

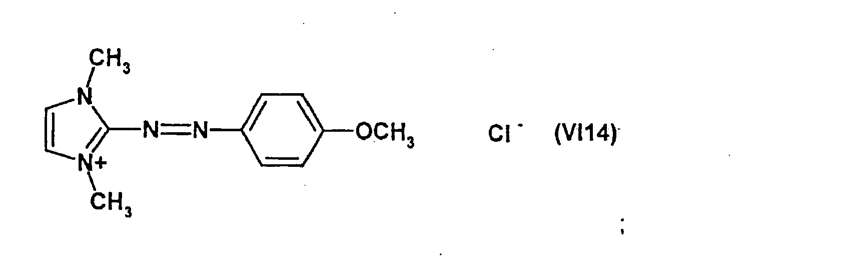

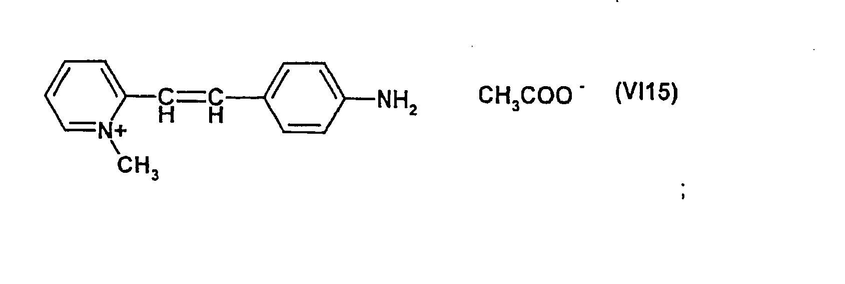

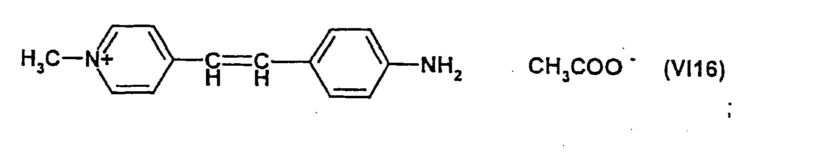

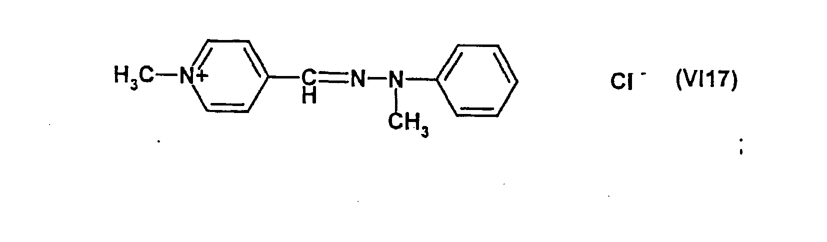

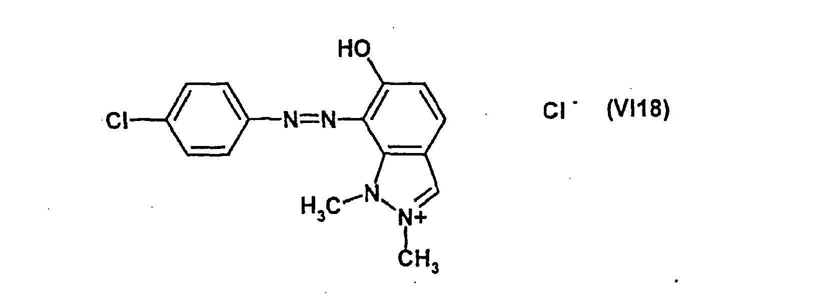

Parmi les composés particuliers de structures (VI1) à (VI18) décrits ci-dessus, on préfère tout particulièrement les composés répondant aux structures (VI4), (VI5) et (VI13).

L'invention a également pour objet une composition de teinture directe des fibres kératiniques, en particulier des fibres kératiniques humaines telles que les cheveux qui ne contient pas de colorant autooxydable, caractérisée par le fait qu'elle comprend, dans un milieu approprié pour la teinture, une association de colorants cationiques telle que définie ci-dessus.

Le ou les colorants cationiques (I) à (III) utilisés selon l'invention, représentent de préférence de 0,001 à 10 % en poids environ du poids total de la composition de teinture directe et encore plus préférentiellement de 0,005 à 5 % en poids environ de ce poids.

Le ou les colorants cationiques de formules (IV) et (VI) utilisés selon l'invention, représentent de préférence de 0,001 à 10 % en poids environ du poids total de la composition de teinture directe et encore plus préférentiellement de 0,005 à 5 % en poids environ de ce poids.
L'association de colorants cationiques selon l'invention peut également servir, à titre de colorant direct, dans les procédés bien connus de teinture d'oxydation, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre l'association de colorants cationiques selon l'invention, d'autres colorants directs classiquement utilisés, et notamment, des colorants nitrés benzéniques, des colorants anthraquinoniques, des colorants naphtoquinoniques, des colorants triarylméthaniques, dles colorants xanthéniques, des colorants azoïques non cationiques.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le PH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 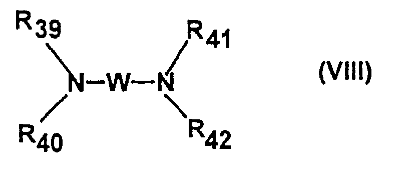 dans laquelle W est un reste propylène éventuellement substitué par un groupement hydroxyle ou un radical alkyle en C₁-C₆ ; R₃₉, R₄₀, R₄₁ et R₄₂, identiques ou différents, représentent un atome d'hydrogène, un radical alkyle en C₁-C₆ ou hydroxyalkyle en C₁-C₆.

La composition de teinture directe conforme à l'invention peut également renfermer divers adjuvants utilisés classiquement dans les compositions pour la teinture des cheveux, tels que des agents antioxydants, des agents de pénétration, des agents séquestrants, des agents épaississants, des parfums, des tampons, des agents dispersants, des agents tensioactifs anioniques, non ioniques, cationiques ou amphotères, des polymères cationiques, anioniques, non ioniques ou amphotères, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition de teinture directe selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant l'association de colorants cationiques définie ci-dessus avec une composition aqueuse.

Un autre objet de l'invention est un procédé de teinture directe des fibres kératiniques et en particulier des fibres kératiniques humaines telles que les cheveux mettant en oeuvre la composition de teinture telle que définie précédemment.

Selon une première variante de ce procédé de teinture directe conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture directe conforme à l'invention, on applique sur les fibres au moins une composition tinctoriale telle que définie précédemment, pendant un temps suffisant pour développer la coloration désirée, sans rinçage final.

Un autre objet de l'invention est un dispositif à plusieurs compartiments ou "kit" de teinture ou tout autre système de conditionnement à plusieurs compartiments dont un premier compartiment renferme une composition contenant le colorant cationique (I) à (III) défini ci-dessus et un second compartiment renferme une composition contenant le colorant cationique de formules (IV) et (VI) défini ci-dessus. Dans une variante de l'invention, le premier compartiment renferme une composition pulvérulente contenant l'association d'au moins un colorant cationique (I) à (III) et d'au moins un colorant cationique de formules (IV) et (VI) défini, selon l'invention et le second compartiment une composition aqueuse servant de véhicule pour la teinture. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 4 :

On a préparé les quatre compositions de teinture directe réunies dans le tableau suivant :
*(toutes teneurs exprimées en grammes)*

| | | **Exemple 1** | **Exemple 2** | **Exemple 3** | **Exemple 4** |
|---|---|---|---|---|---|
| Arianor Madder Red (WARNER JENKINSON) | | 0,1 | | | |
| Arianor Steel Blue (WARNER JENKINSON) | | | 0,1 | 0,12 | |
| Arianor Straw Yellow (WARNER JENKINSON) | | | | | 0,12 |
| Colorant direct cationique de formule (IV₃₁) | | | 0,1 | | |
| Colorant direct cationique de formule (VII₂₇) | | | | 0,12 | |
| Colorant direct cationique de formule (VI₄) | | | | | 0,15 |
| Colorant direct cationique de formule (IV₁) | | 0,1 | | | |
| Hydroxyéthylcellulose | | 1,0 MA* | 1,0 MA* | 1,0 MA* | 1,0 MA* |
| Ethanol | | 10 | 10 | 10 | 10 |
| 2-amino-2-méthyl-1-propanol qs | | pH 9 | pH 9 | pH 9 | pH 9 |
| Eau déminéralisée qsp | | 100 | 100 | 100 | 100 |
| | MA* désigne Matière Active | | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| **Exemples** | **Nuances obtenues** |
|---|---|
| 1 | Rouge puissant |
| 2 | Volet bleu puissant |
| 3 | Violet puissant |
| 4 | Jaune puissant |

## Revendications

1. Utilisation à titre de colorants directs dans des, ou pour la fabrication de, compositions de teinture directe pour fibres kératiniques en particulier pour fibres kératiniques humaines et notamment les cheveux et qui ne contiennent pas de colorant autooxydable, d'une association comprenant (i) au moins un colorant cationique choisi parmi (I) le basic brown 17, le basic brown 16, le basic red 16, le basic red 118, (II) le basic yellow 57, (III) le basic blue 99 et (ii) au moins un colorant cationique de formules (IV) ou VI) suivantes :
(ii) colorant cationique de formules (IV), (VI), suivantes:
a) les composés de formule (IV) suivante : 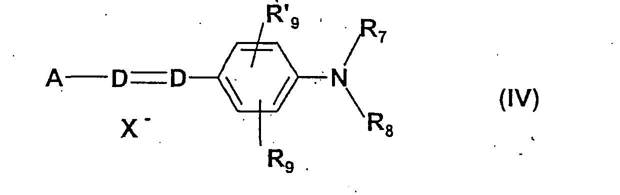 dans laquelle :
D représente un atome d'azote ou le groupement -CH,
R₇ et R₈, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₉ et R'₉, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy
X⁻ représente un anion de préférence choisi parmi le chlorure, le méthyl sulfate et l'acétate,
A représente un groupement choisi par les structures A1 à A19 suivantes : 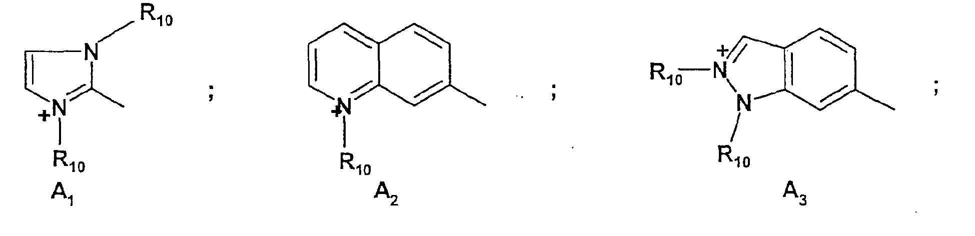 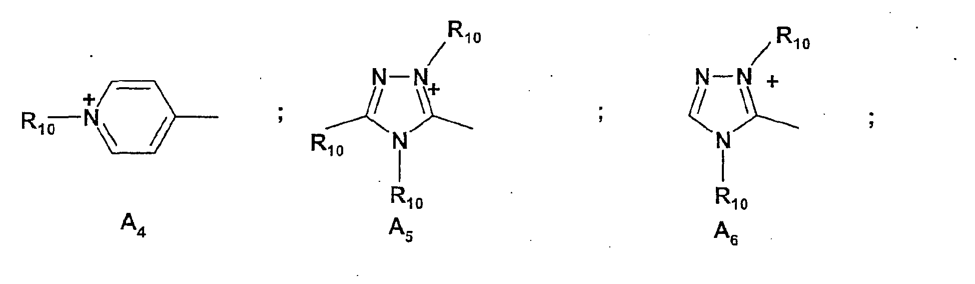 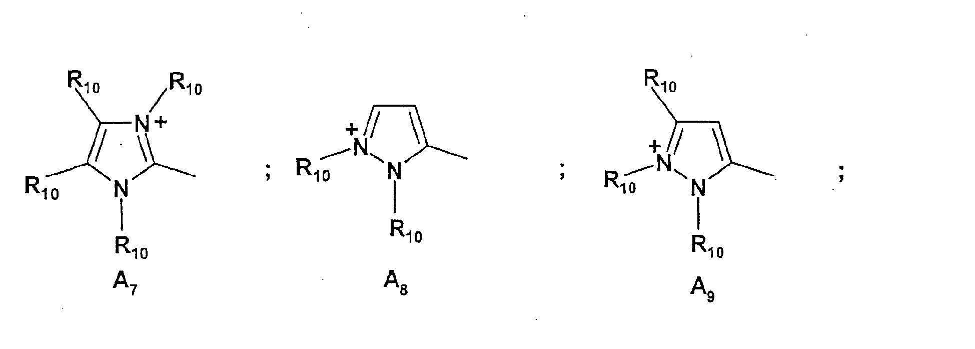 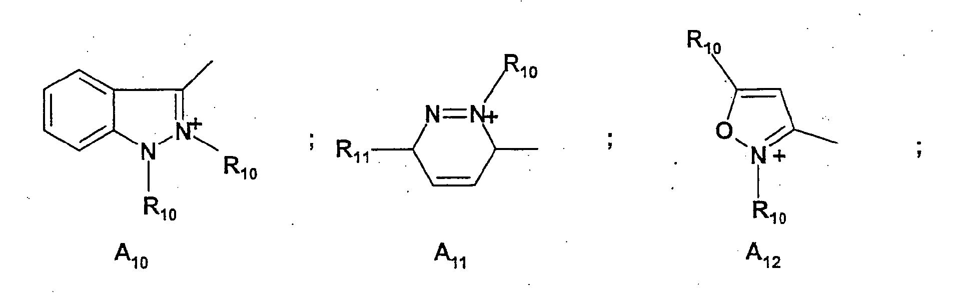 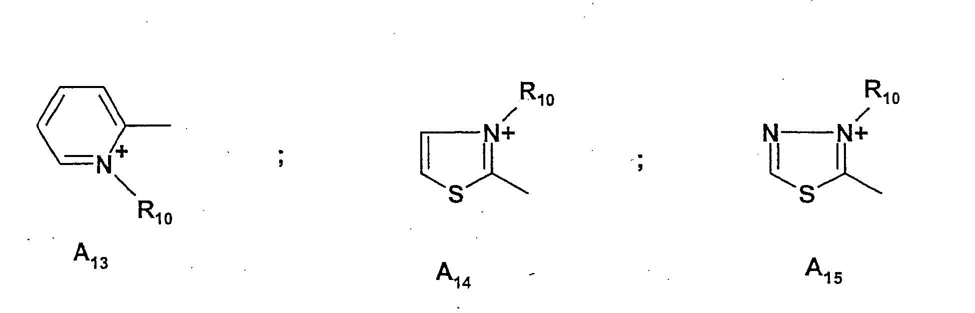 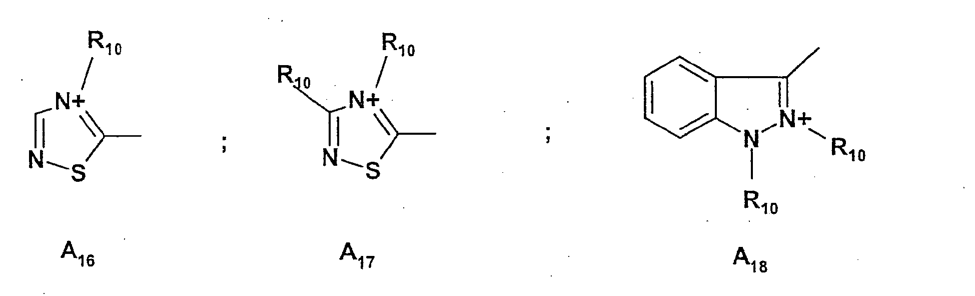 et 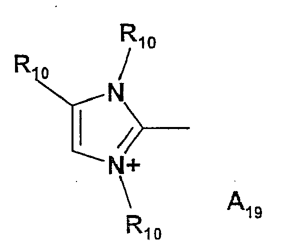
dans lesquelles R₁₀ représente un radical alkyle en C₁-C₄ pouvant être substitué par un radical hydroxyle et R₁₁ représente un radical alcoxy en C₁-C₄, sous réserve que
(i) lorsque D représente -CH, que A représente A₄ ou A₁₃ et que R₉ est différent d'un radical alcoxy, alors R₇ et R₈ ne désignent pas simultanément un atome d'hydrogène ;
(ii) lorsque D représente un atome d'azote, que A représente A₆, alors R₇ et R₈ ne désignent pas simultanément un radical méthyle.
c) les composés de formules (VI) suivantes : 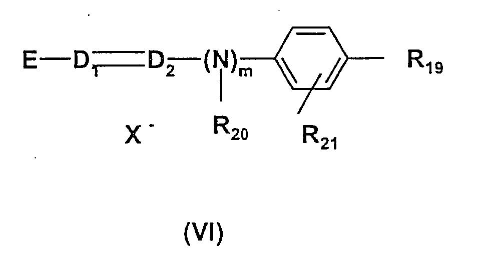 dans laquelle :
R₁₉ représente un atome d'hydrogène, un radical alcoxy en C₁-C₄, un atome d'halogène tel que le brome, le chlore, l'iode ou le fluor ou un radical amino,
R₂₀ représente un atome d'hydrogène, un radical alkyle en C₁-C₄ ou forme avec un atome de carbone du cycle benzénique un hétérocycle éventuellement oxygéné et/ou substitué par un ou plusieurs groupement alkyle en C₁-C₄,
R₂₁ représente un atome d'hydrogène ou d'halogène tel que le brome, le chlore, l'iode ou le fluor,
D₁ et D₂, identiques ou différents, représentent un atome d'azote ou le groupement -CH,
m = 0 ou 1,
étant entendu que lorsque R₁₉ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 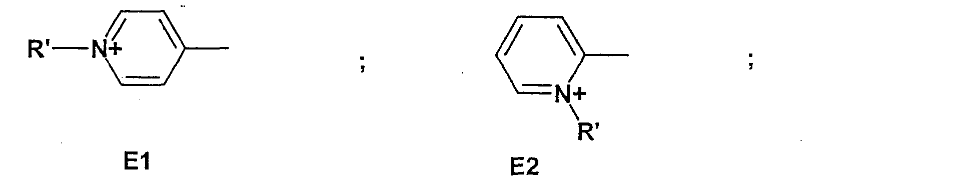 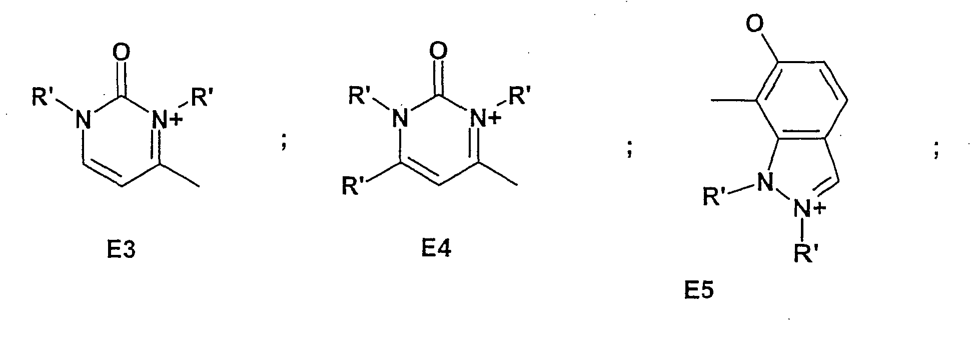 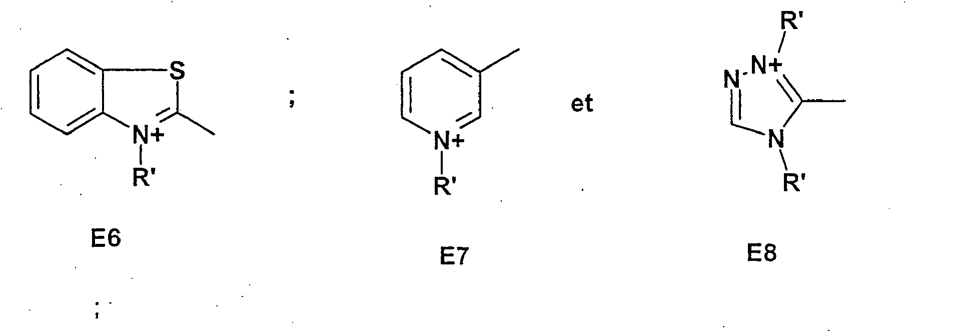
dans lesquelles R' représente un radical alkyle en C₁-C₄ ;
lorsque m = 0 et que D₁₃ représente un atome d'azote, alors E peut également désigner un groupement de structure E9 suivante : 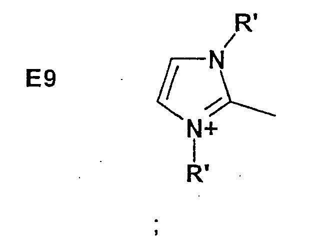 dans laquelle R' représente un radical alkyle en C₁-C₄.

2. Utilisation selon la revendication 1, **caractérisée par le fait que** le colorant cationique est le Basic Blue 99.

3. Utilisation selon la revendication 1, **caractérisée par le fait que** les colorants cationiques de formule (IV) sont choisis parmi les composés de formules (IV1) à (IV54) suivantes :

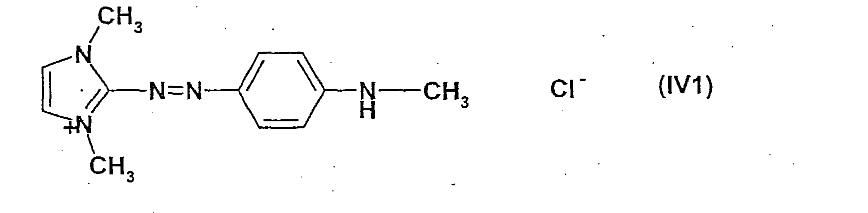

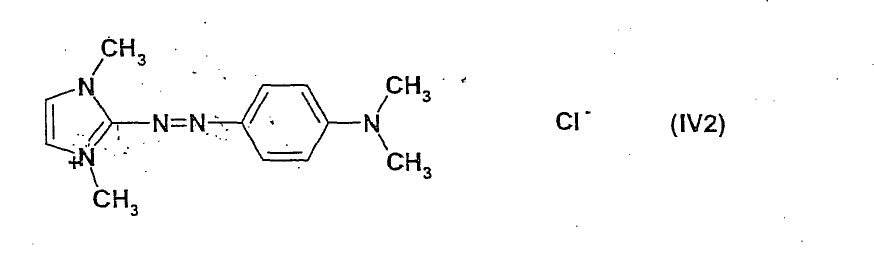

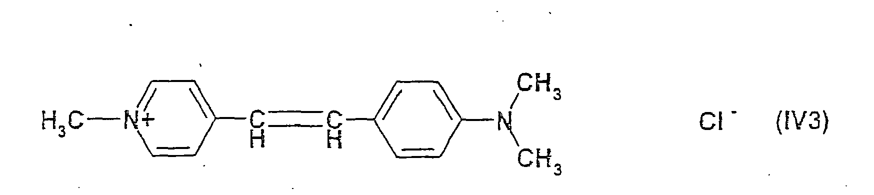

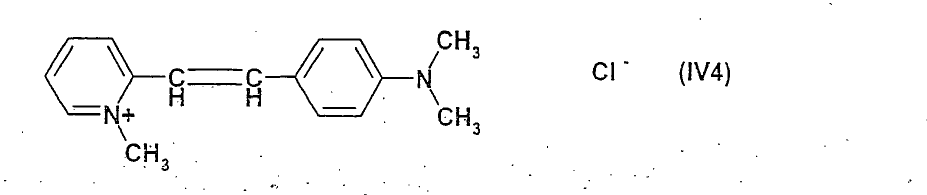

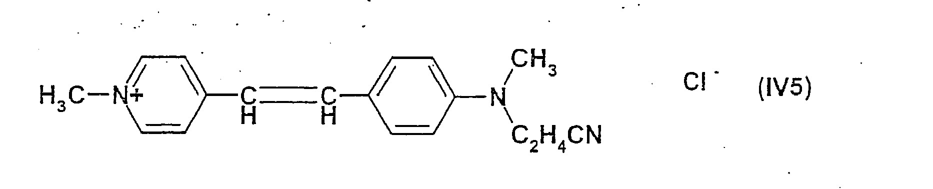

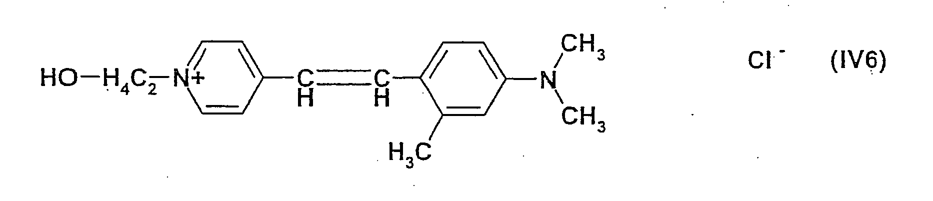

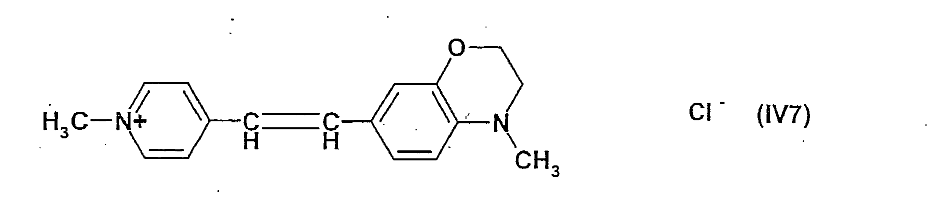

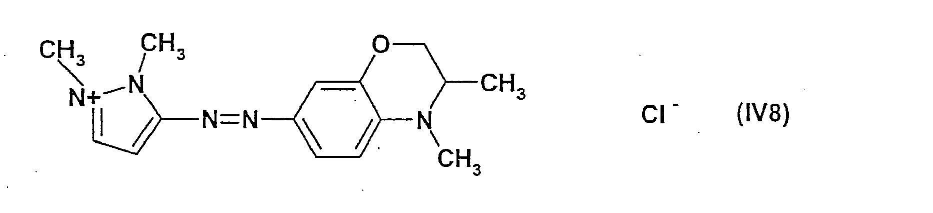

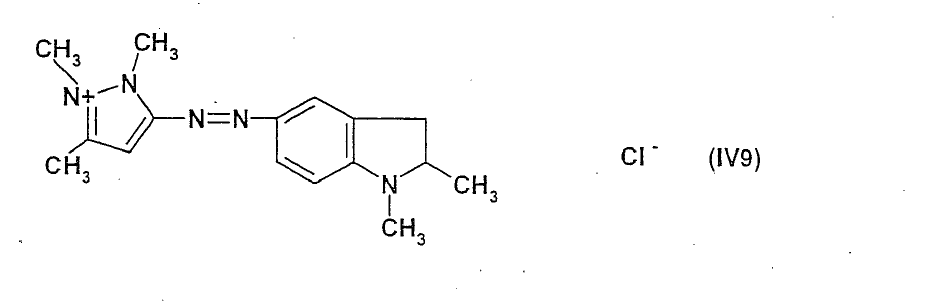

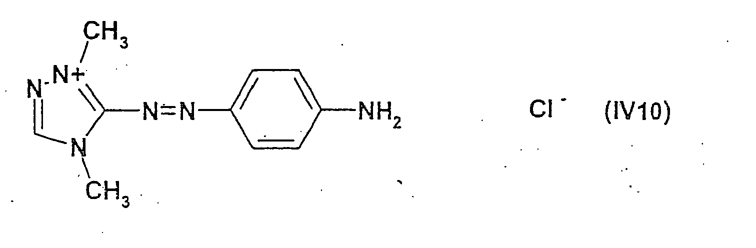

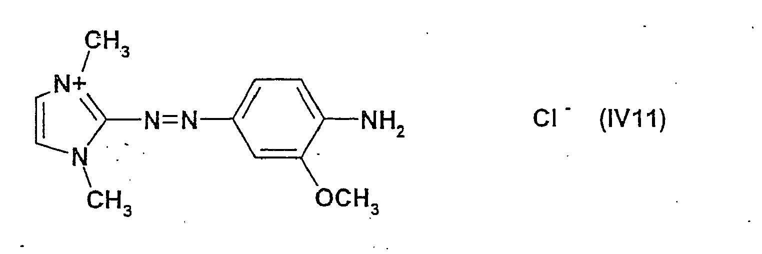

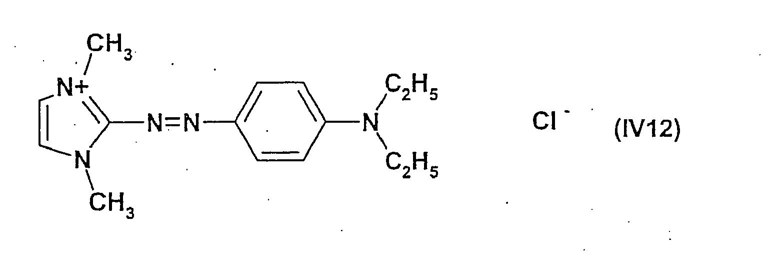

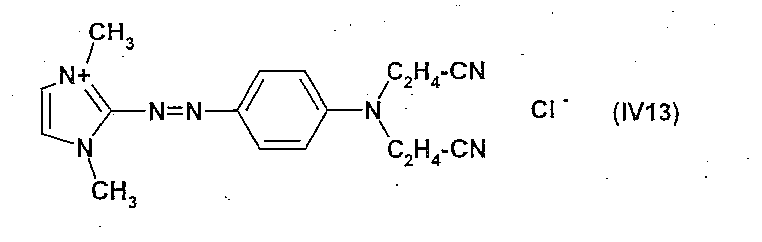

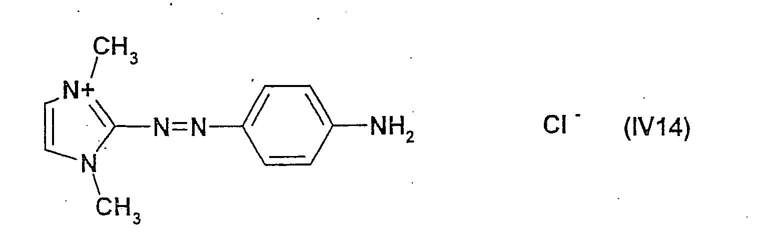

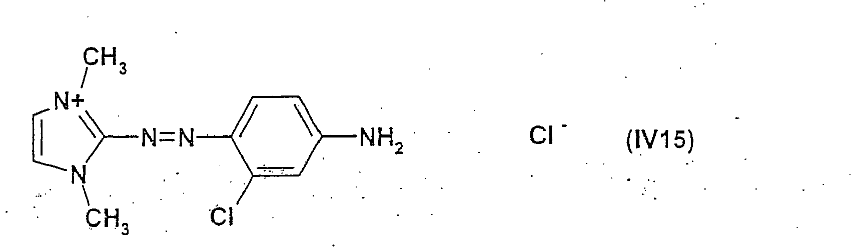

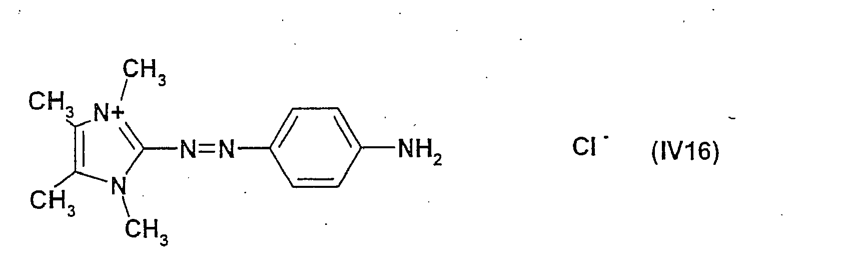

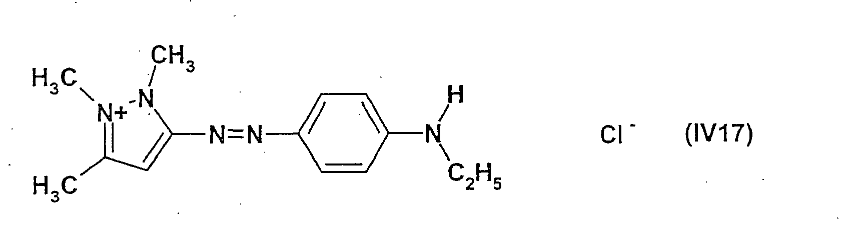

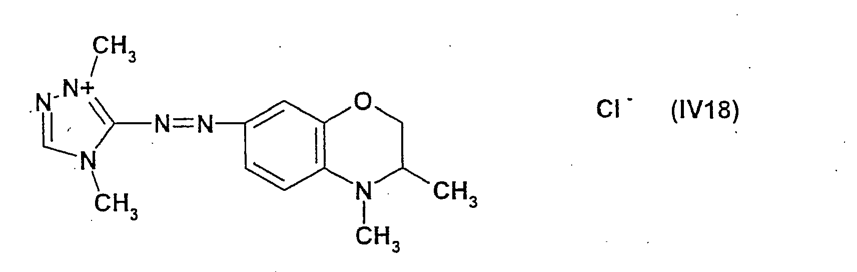

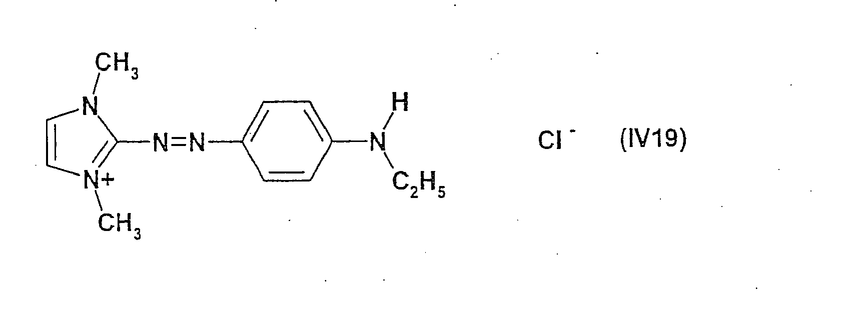

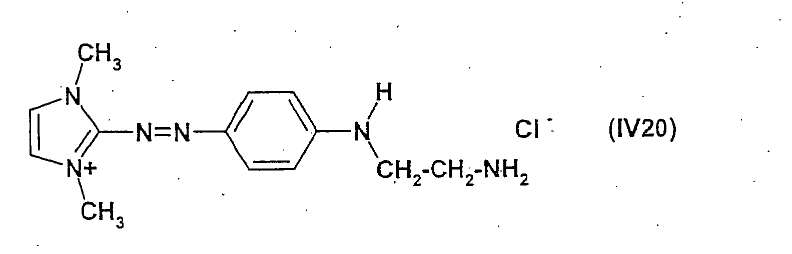

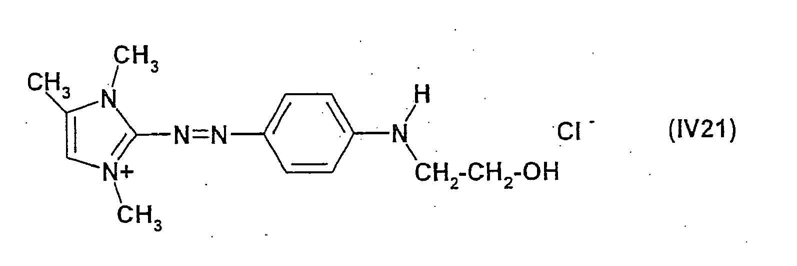

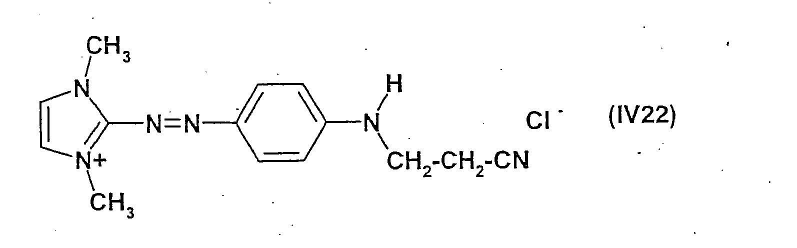

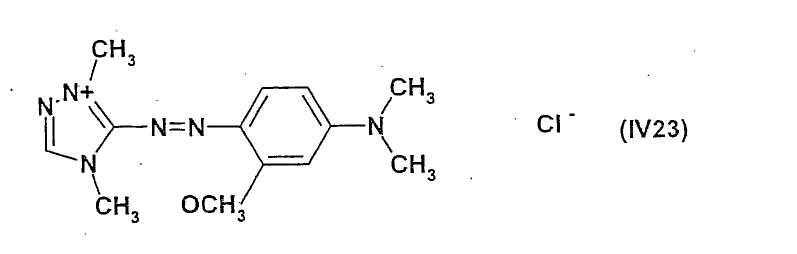

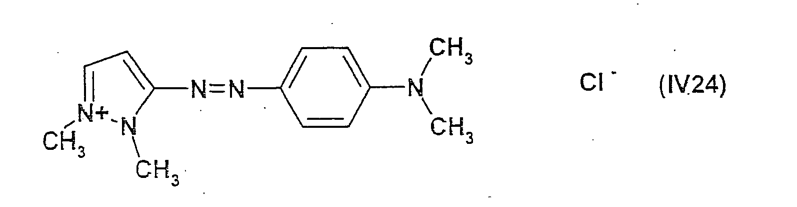

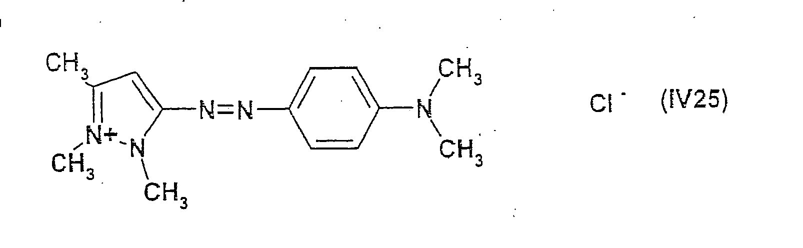

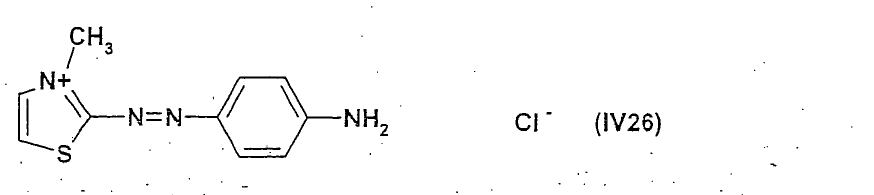

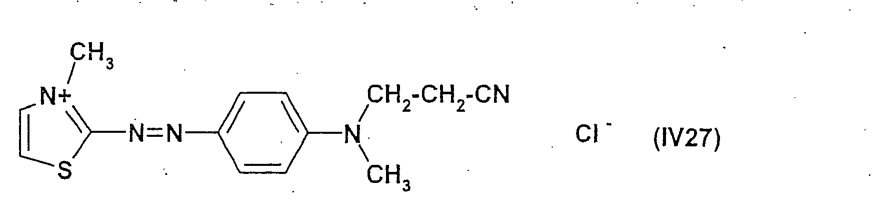

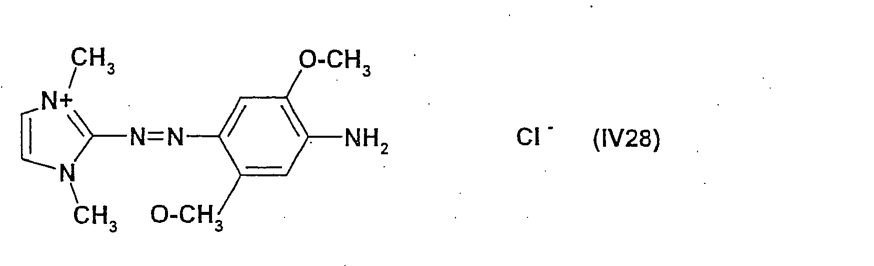

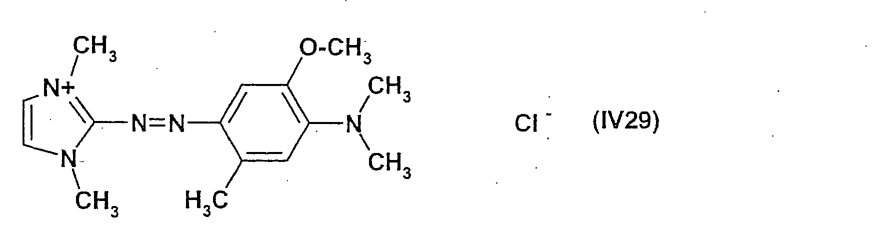

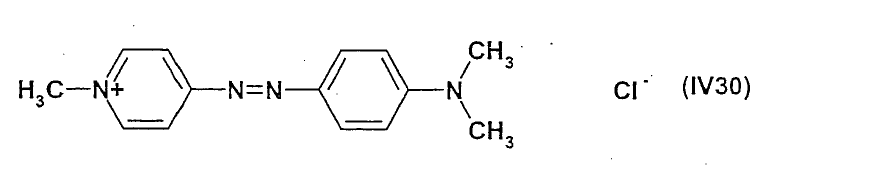

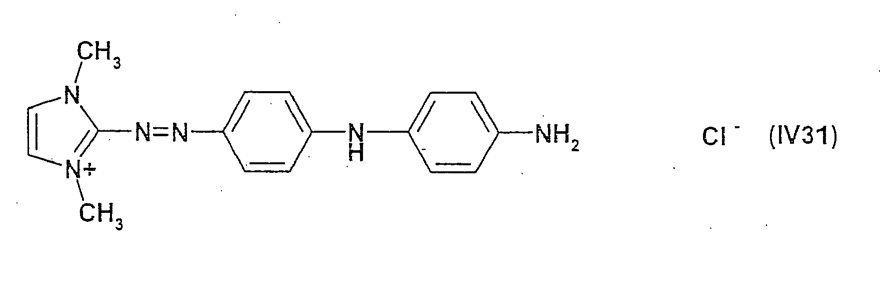

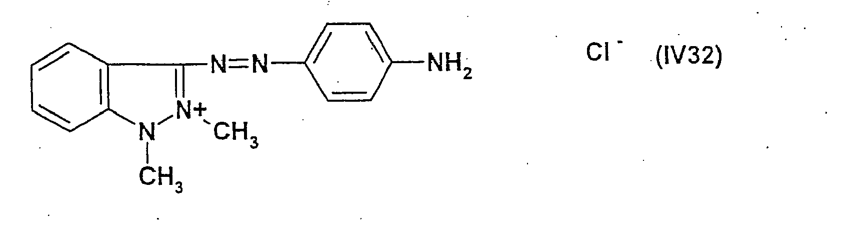

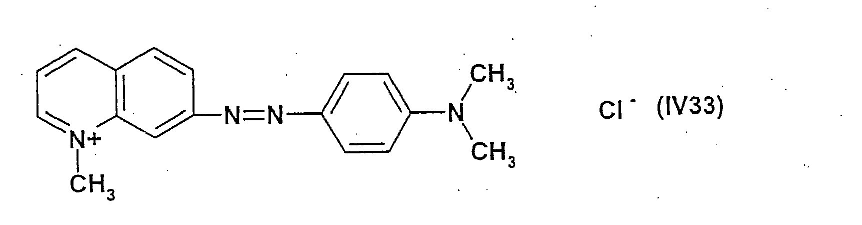

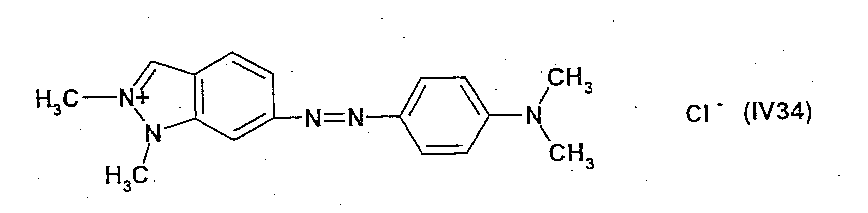

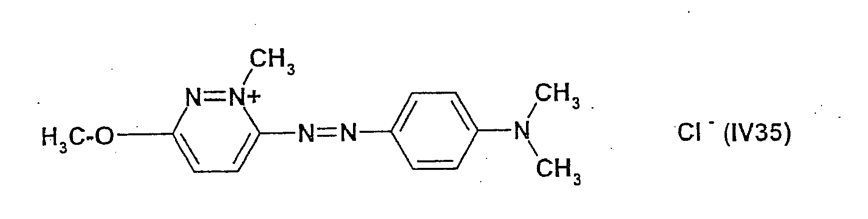

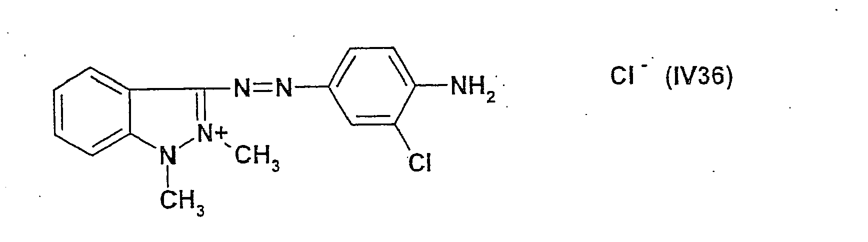

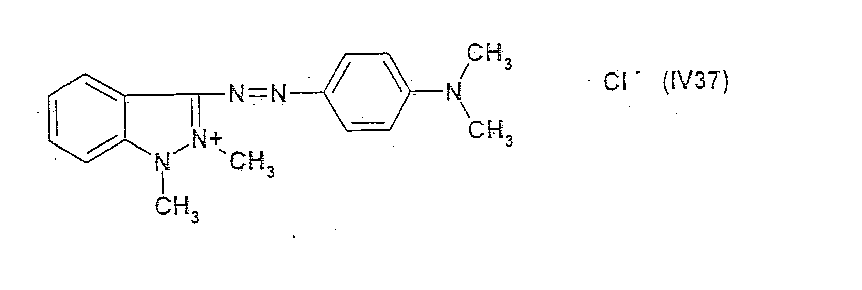

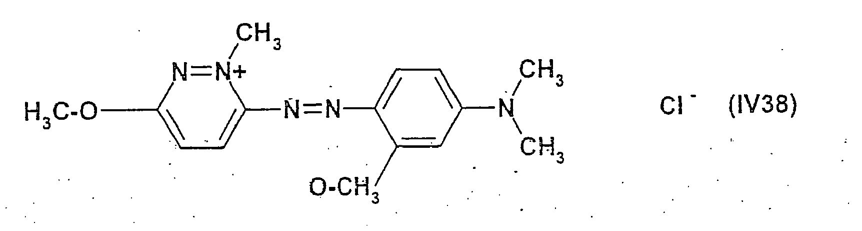

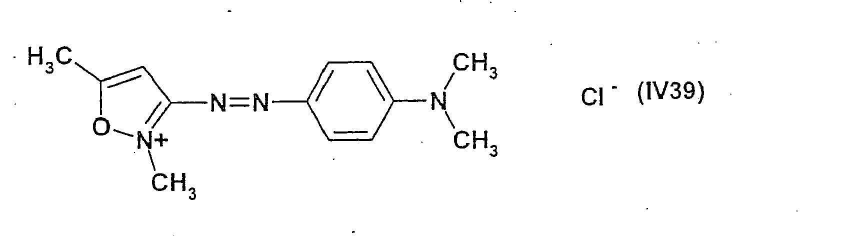

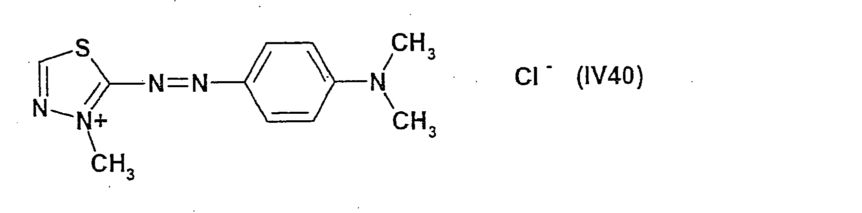

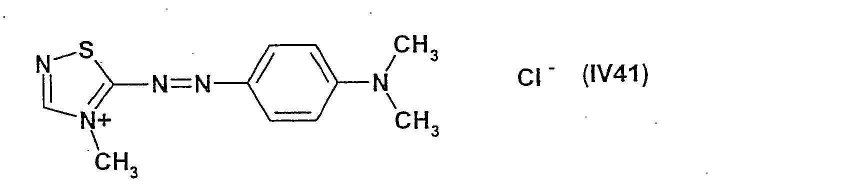

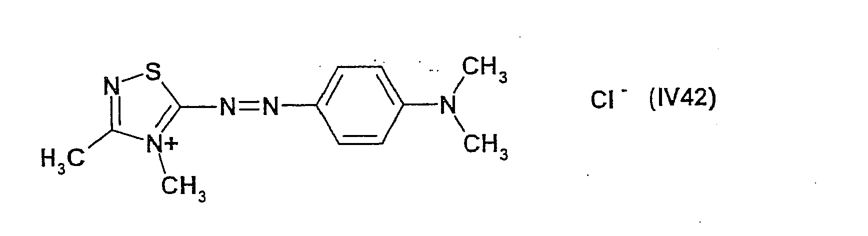

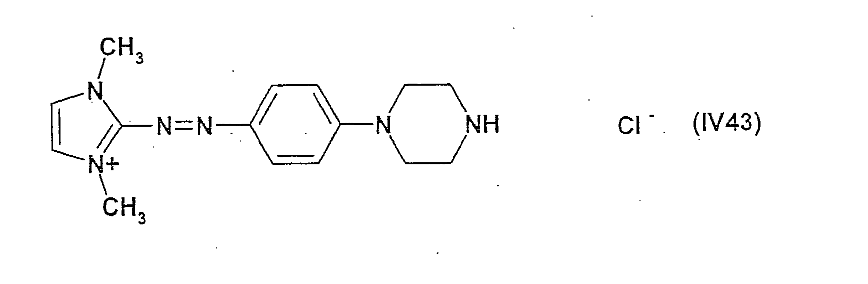

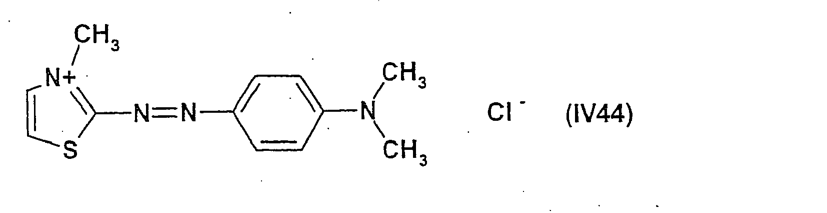

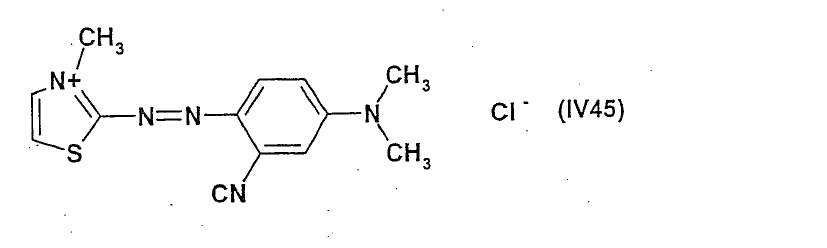

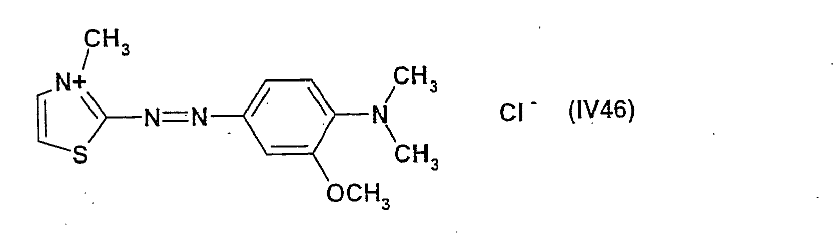

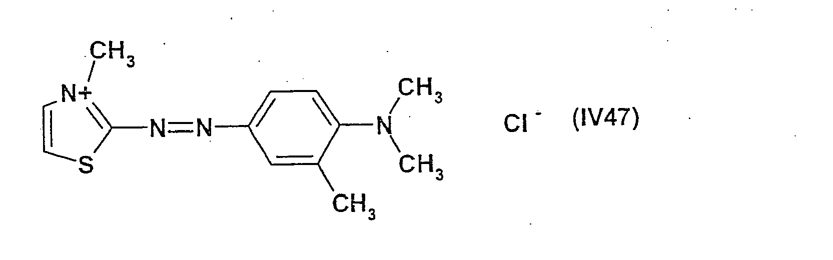

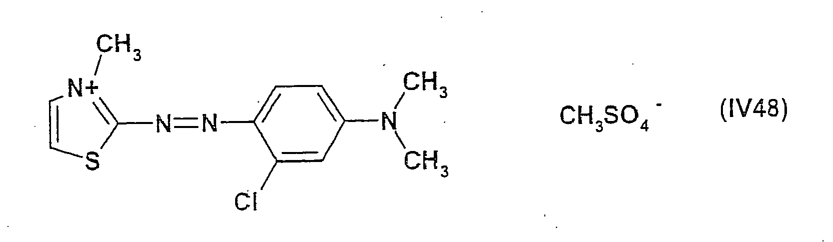

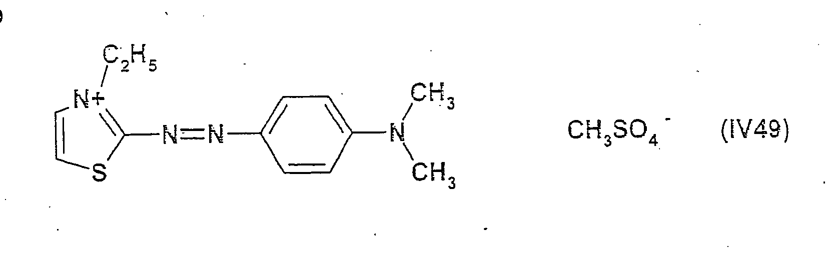

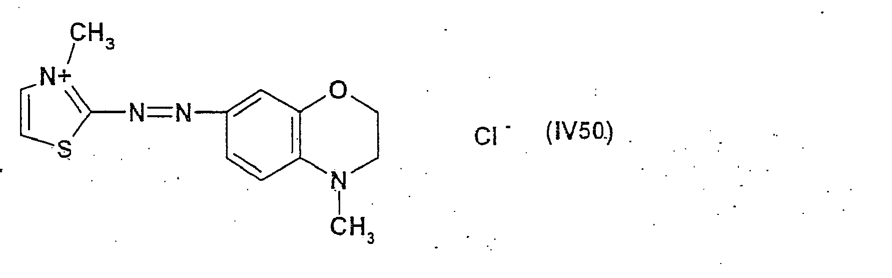

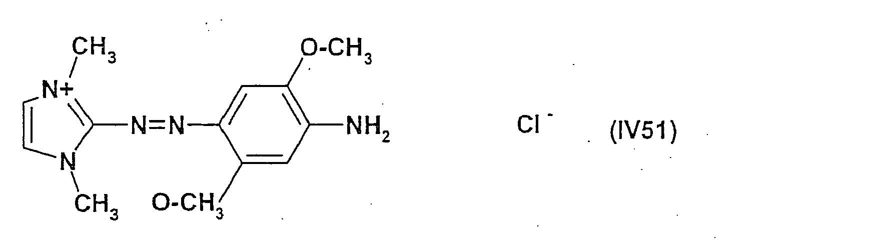

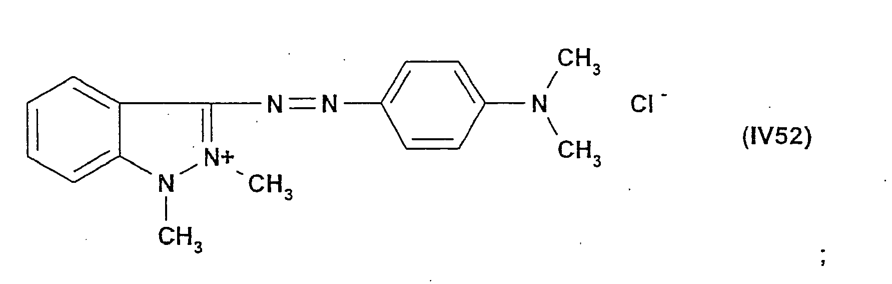

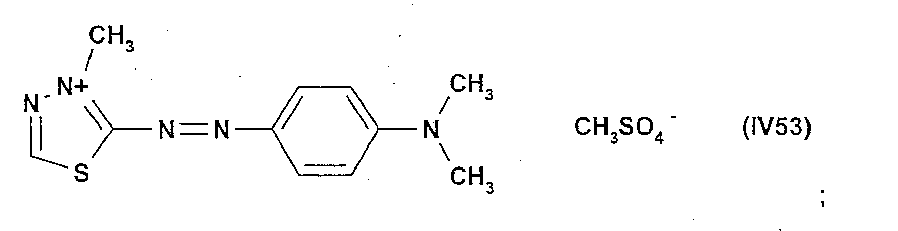

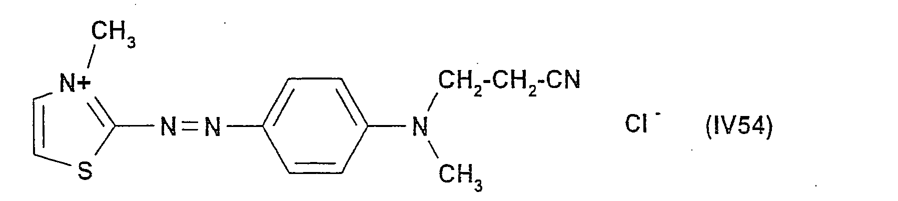

4. Utilisation selon la revendication 3, **caractérisée par le fait que** les colorants cationiques de formule (IV) sont choisis parmi les composés de formules (IV1) (IV2), (IV14), et (IV31).

5. Utilisation selon la revendication 1, **caractérisée par le fait que** les colorants cationiques de formule (VI) sont choisis parmi les composés de formules (VI1) à (VI18) suivantes : 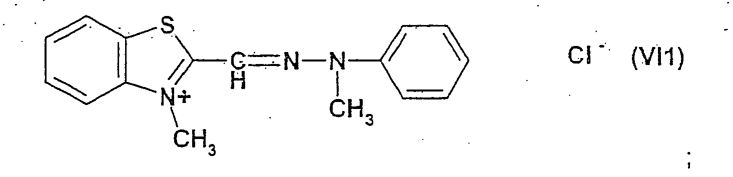 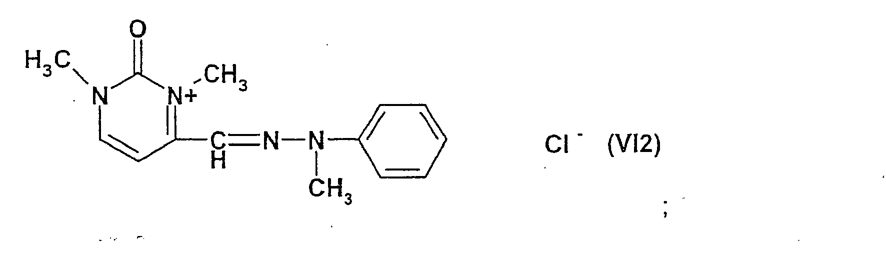 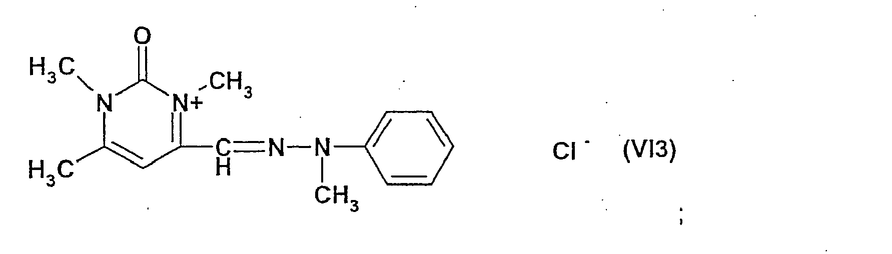 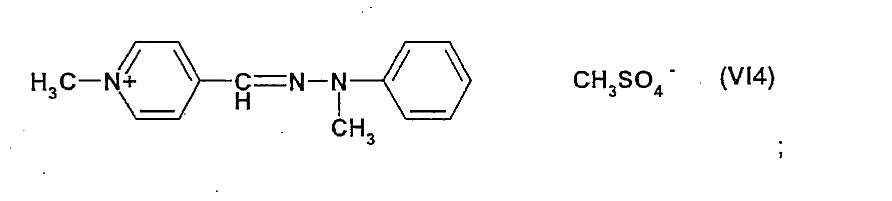 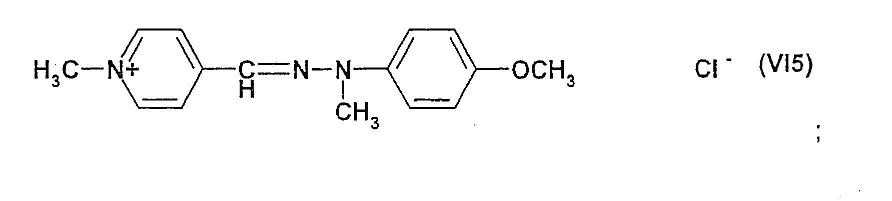 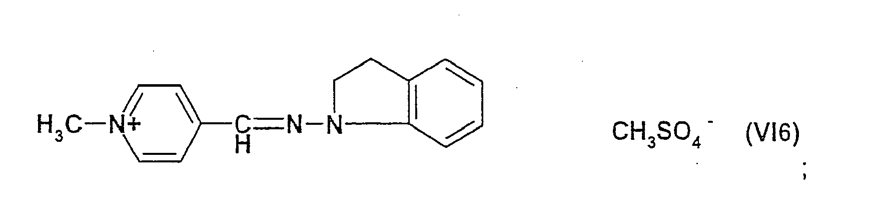 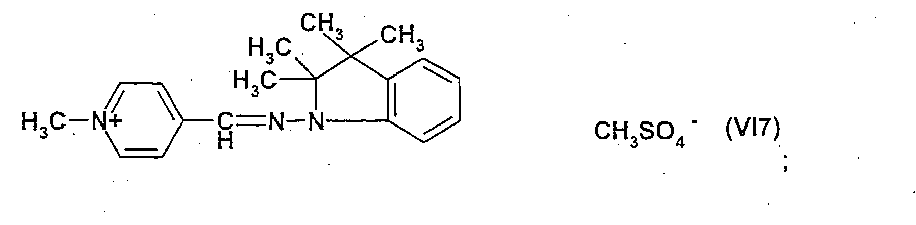 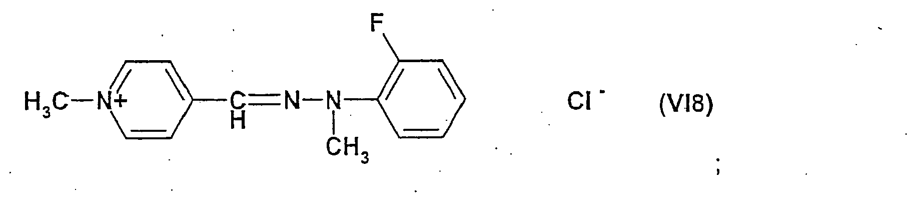 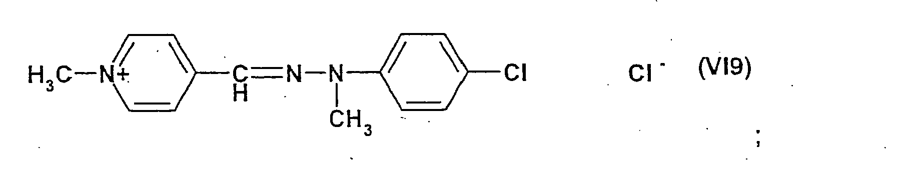 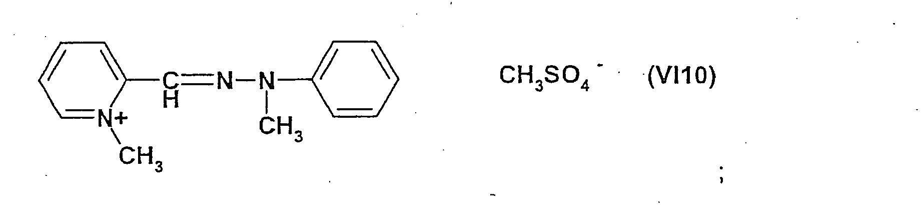 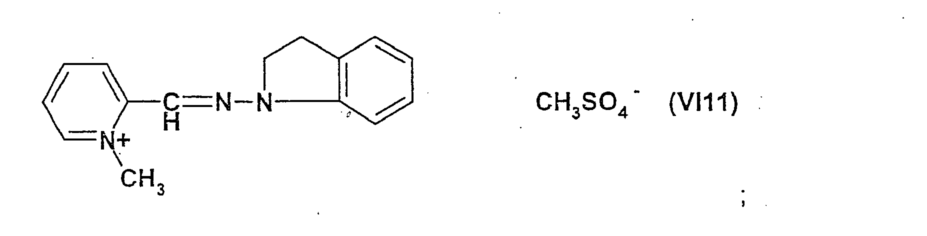 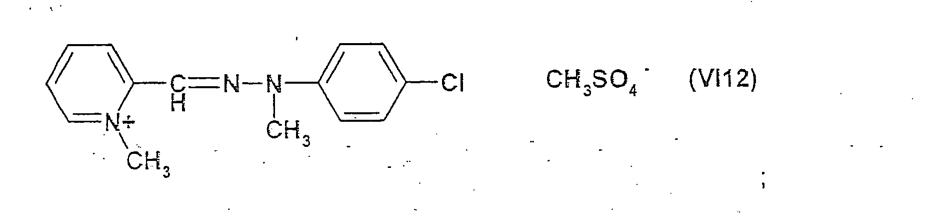 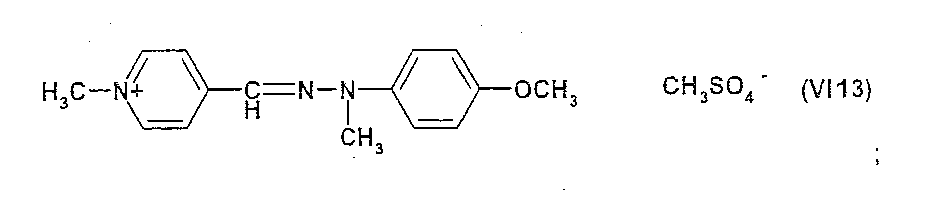 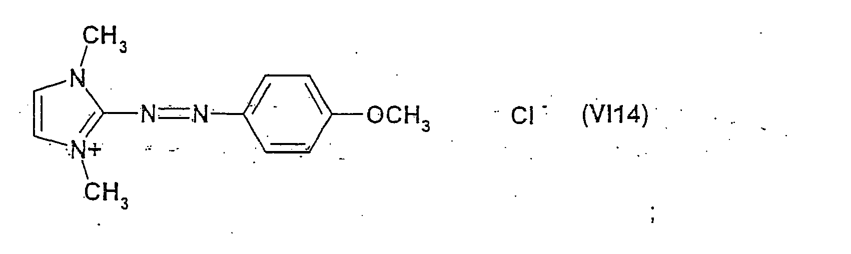 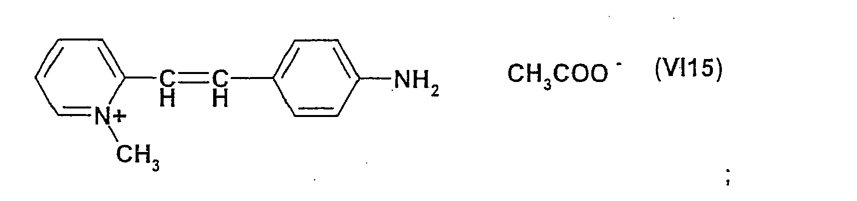 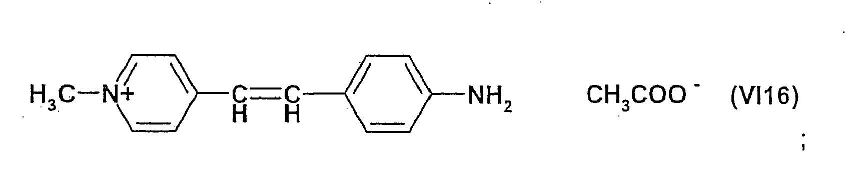 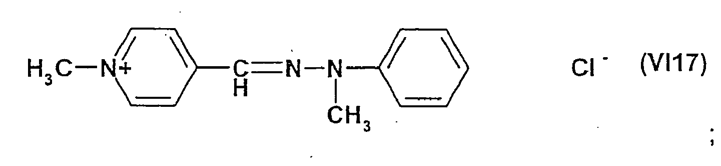 et

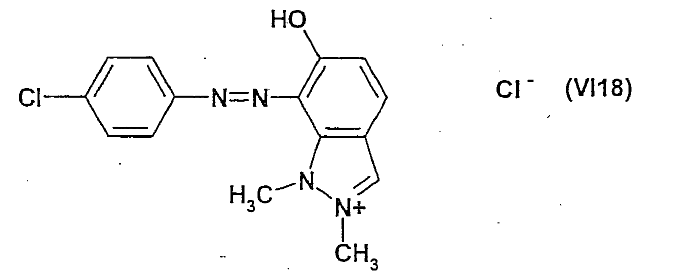

6. Utilisation selon la revendication 5, **caractérisée par le fait que** les colorants cationiques de formule (VI) sont choisis parmi les composés de formules (VI4), (VI5) et (VI13).

7. Composition de teinture directe pour fibres kératiniques et en particulier pour fibres kératiniques humaines et notamment les cheveux qui ne contient pas de colorant autooxydable, **caractérisée par le fait qu'**elle comprend, dans un milieu approprié pour la teinture, une association d'au moins un colorant de formules (I) à (III) et d'au moins un colorant de formules (IV) ou (VI) définies à l'une quelconque des revendications 1 à 6.

8. Composition selon la revendication 7, **caractérisée par le fait que** le où les colorants cationiques (I), (II), (III) sont présents dans une concentration allant de 0,001 à 10 % en poids du poids total de la composition.

9. Composition selon la revendication 8, **caractérisée par le fait que** le ou les colorants cationiques (I), (II), (III) sont présents dans une concentration allant de 0,005 à 5 % en poids du poids total de la composition.

10. Composition selon la revendications 7, **caractérisée par le fait que** le ou les colorants cationiques de formules (IV), (VI), représentent de 0,001 à 10 % en poids du poids total de la composition.

11. Composition selon la revendication 10, **caractérisée par le fait que** le ou les colorants cationiques de formules (IV), (VI), représentent de 0,001 à 10 % en poids du poids total de la composition.

12. Composition selon l'une quelconque des revendications 7 à 11, **caractérisée par le fait que** le milieu approprié pour la teinture (ou support) est constitué par de l'eau ou par un mélange d'eau et d'au moins un solvant organique.

13. Composition selon l'une quelconque des revendications 7 à 12, **caractérisée par le fait qu'**elle présente un pH compris entre 2 et 11, et de préférence entre 5 et 10.

14. Composition selon l'une quelconque des revendications 7 à 13, **caractérisée par le fait qu'**elle renferme des adjuvants choisis parmi des agents antioxydants, des agents de pénétration, des agents séquestrants, des agents épaississants, des parfums, des tampons, des agents dispersants, des agents tensioactifs, des polymères, des agents filmogènes, des céramides, des agents conservateurs, des agents filtrants, des agents opacifiants.

15. Procédé de teinture directe des fibres kératiniques et en particulier des fibres kératiniques humaines telles que les cheveux, **caractérisé par le fait que** qu'on applique sur les fibres au moins une composition tinctoriale telle que définie à l'une quelconque des revendications 7 à 14, pendant un temps suffisant pour développer la coloration désirée, après quoi on rince, on lave éventuellement au shampooing, on rince à nouveau et on sèche.

16. Procédé de teinture directe des fibres kératiniques et en particulier des fibres kératiniques humaines telles que les cheveux, **caractérisé par le fait que** qu'on applique sur les fibres au moins une composition tinctoriale telle que définie à l'une quelconque des revendications 17 à 14, pendant un temps suffisant pour développer la coloration désirée, sans rinçage final.

17. Dispositif à plusieurs compartiments ou "kit" de teinture à plusieurs compartiments, **caractérisé par le fait qu'**un premier compartiment renferme au moins un colorant cationique (I) à (III) défini à la revendication 1 et un second compartiment renferme au moins un colorant cationique de formules (IV) ou (VI) définies à l'une quelconque des revendications 1, et 3 à 6.

18. Dispositif à plusieurs compartiments ou « kit « de teinture à plusieurs compartiments, **caractérisé par le fait qu'**un premier compartiment renferme une composition pulvérulente contenant l'association d'au moins un colorant cationique (I) à (III) défini à la revendication 1 et d'au moins un colorant de formules (IV) ou (VI) définies à l'une quelconque des revendications 1, 3 à 6, et qu'un second compartiment renferme une composition aqueuse servant de véhicule pour la teinture.

## Claims

1. Use as direct dyes, in or for the manufacture of direct dye compositions for keratin fibres, in particular for human keratin fibres and especially the hair, and which contain no self-oxidizing dye, of a combination comprising (i) at least one cationic dye chosen from (I) Basic Brown 17, Basic Brown 16, Basic Red 76, Basic Red 118, (II) Basic Yellow 57, and (III) Basic Blue 99 and (ii) at least one cationic dye of formula (IV) or (VI) below:
a) the compounds of formula (IV) below: 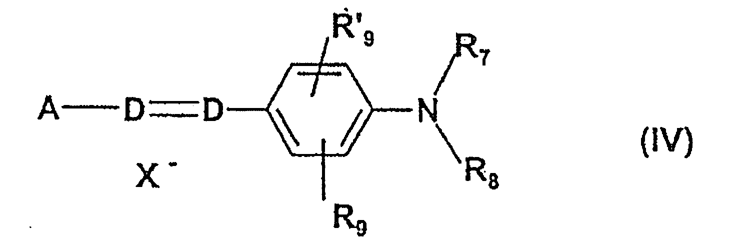 in which:
D represents a nitrogen atom or a -CH group,
R₇ and R₈, which may be identical or different, represent a hydrogen atom; a C₁-C₄ alkyl radical possibly substituted with a -CN, -OH or -NH₂ radical or form, with a carbon atom of the benzene ring, an optionally oxygenated or nitrogenous heterocycle, which may be substituted with one or more C₁-C₄ alkyl radicals; a 4'-aminophenyl radical, R₉ and R'₉, which may be identical or different, represent a hydrogen atom or a halogen atom chosen from chlorine, bromine, iodine and fluorine, or a cyano, C₁-C₄ alkyl, C₁-C₄ alkoxy or acetyloxy radical,
X⁻ represents an anion preferably chosen from chloride, methyl sulfate and acetate,
A represents a group chosen from the structures A1 to A19 below: 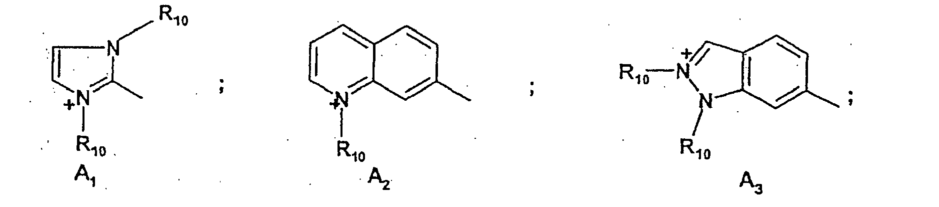 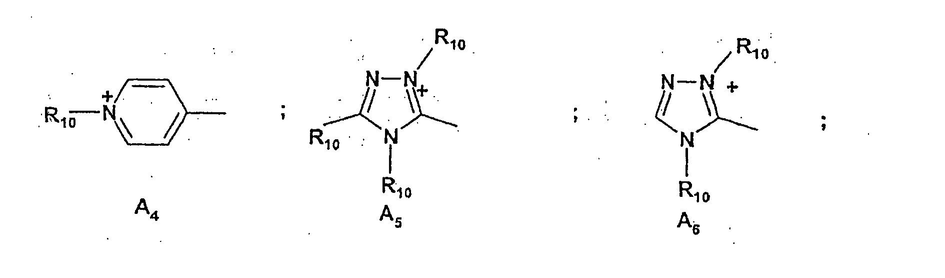 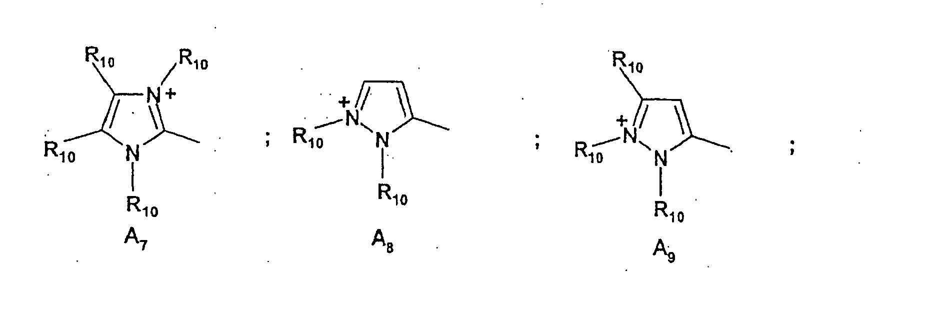 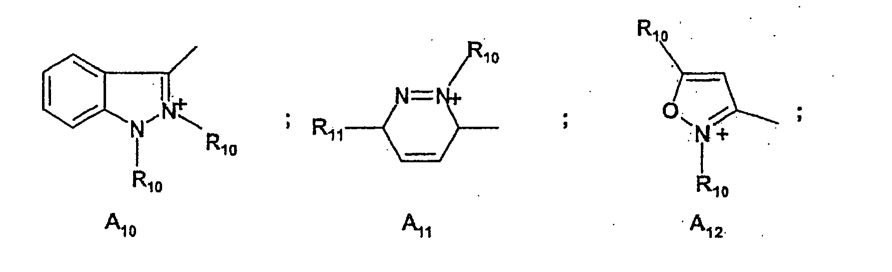 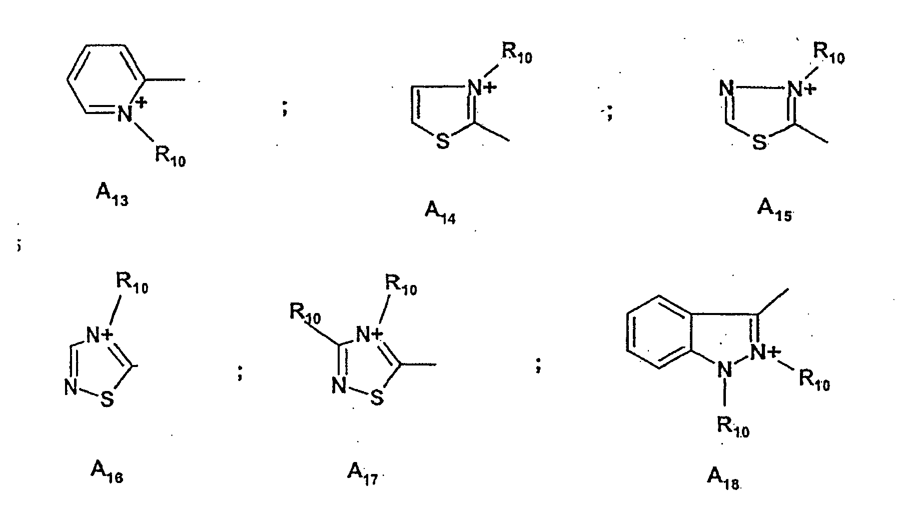 and 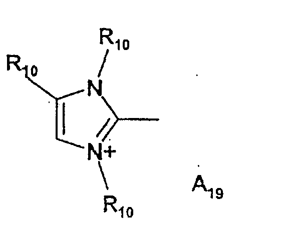
in which R₁₀ represents a C₁-C₄ alkyl radical possibly substituted with a hydroxyl radical and R₁₁ represents a C₁-C₄ alkoxy radical, with the proviso that
(i) when D represents -CH, when A represents A₄ or A₁₃ and when R₉ is other than an alkoxy radical, then R₇ and R₈ do not simultaneously denote a hydrogen atom;
(ii) when D represents a nitrogen atom and when A represents A₆, then R₇ and R₈ do not simultaneously denote a methyl radical,
c) the compounds of formula (VI) below: 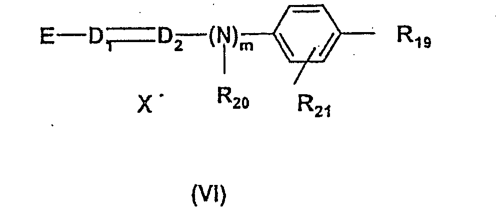
in which:
R₁₉ represents a hydrogen atom, a C₁-C₄ alkoxy radical, a halogen atom such as bromine, chlorine, iodine or fluorine, or an amino radical,
R₂₀ represents a hydrogen atom, a C₁-C₄ alkyl radical or forms, with a carbon atom of the benzene ring, a heterocycle that is optionally oxygenated and/or substituted with one or more C₁-C₄ alkyl groups,
R₂₁ represents a hydrogen or halogen atom such as bromine, chlorine, iodine or fluorine,
D₁ and D₂, which may be identical or different, represent a nitrogen atom or a -CH group,
m = 0 or 1,
it being understood that when R₁₉ represents an unsubstituted amino group, then D₁ and D₂ simultaneously represent a -CH group and m = 0,
X⁻ represents an anion preferably chosen from chloride, methyl sulfate and acetate,
E represents a group chosen from the structures E1 to E8 below: 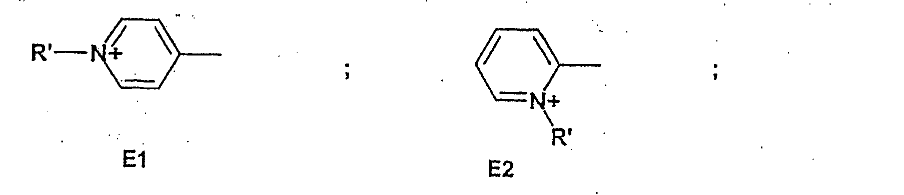 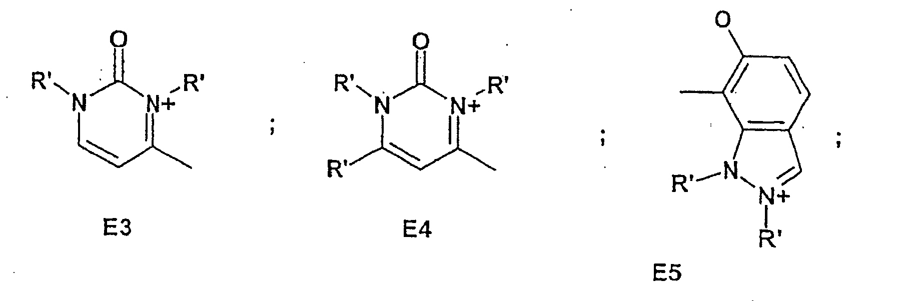 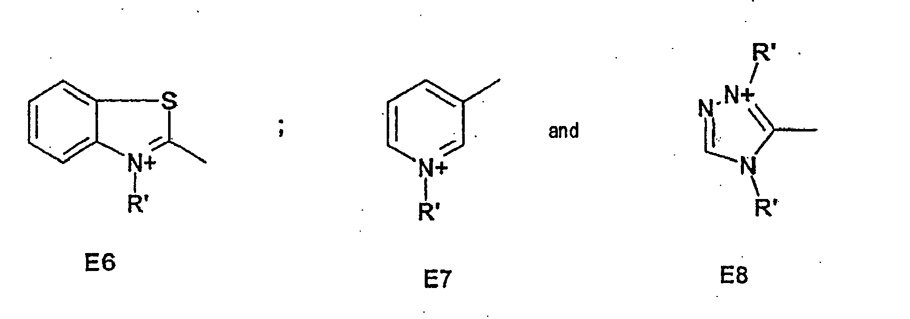
in which R' represents a C₁-C₄ alkyl radical;
when m = 0 and when D₁₃ represents a nitrogen atom, then E may also denote a group of structure E9 below: 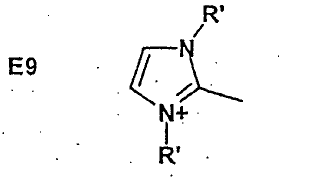 in which R' represents a C₁-C₄ alkyl radical.

2. Use according to Claim 1, **characterized in that** the cationic dye is Basic Blue 99.

3. Use according to Claim 1, **characterized in that** the cationic dyes of formula (IV) are chosen from the compounds of formulae (IV1) to (IV54) below:

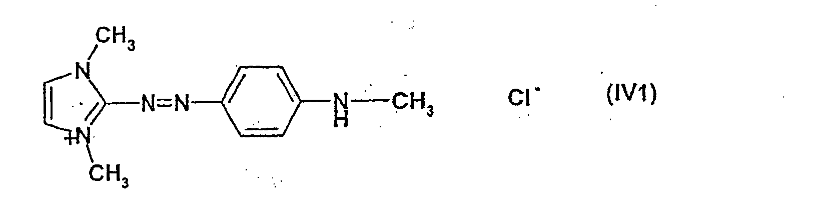

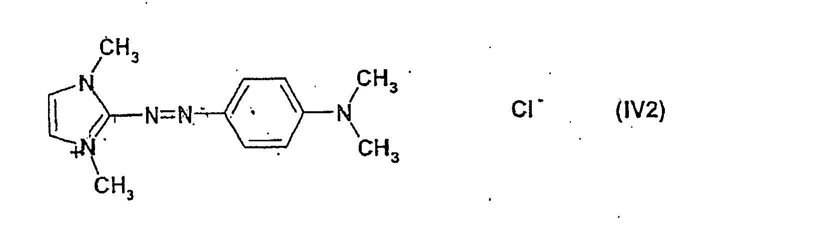

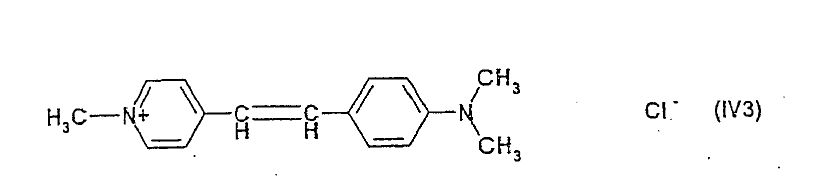

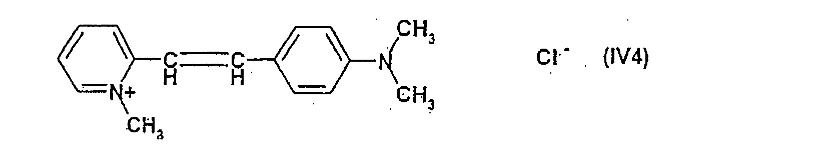

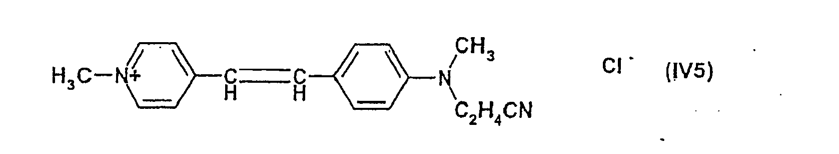

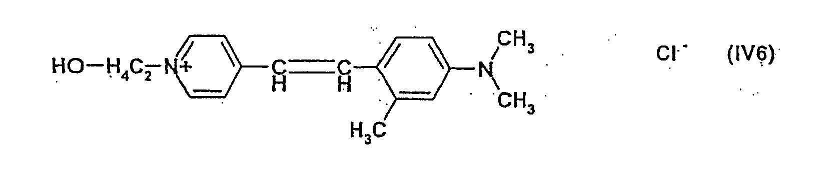

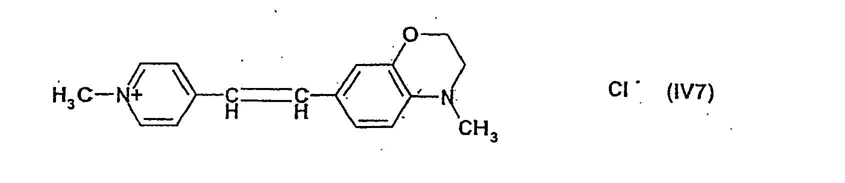

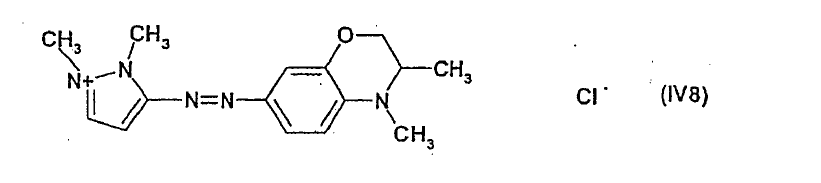

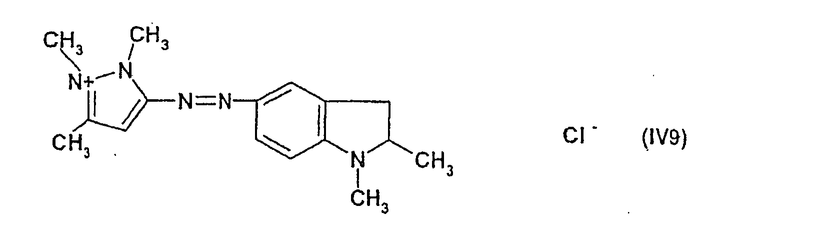

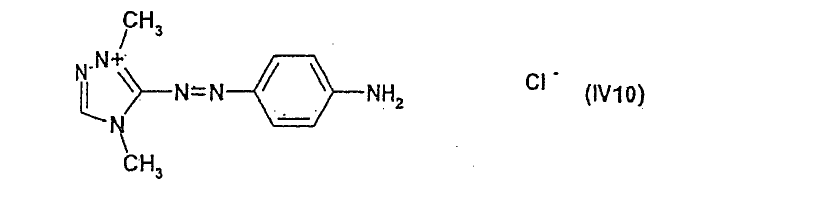

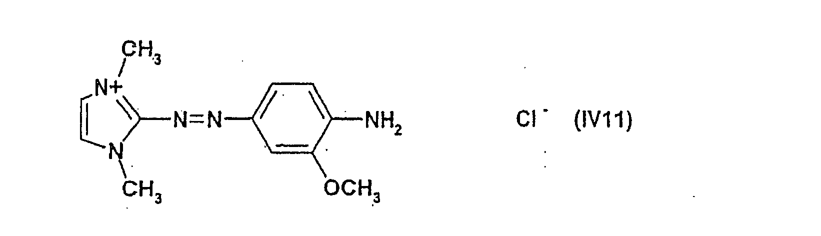

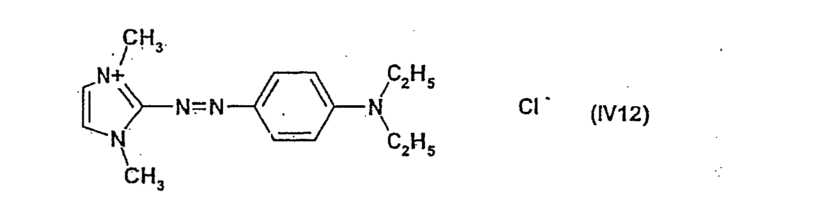

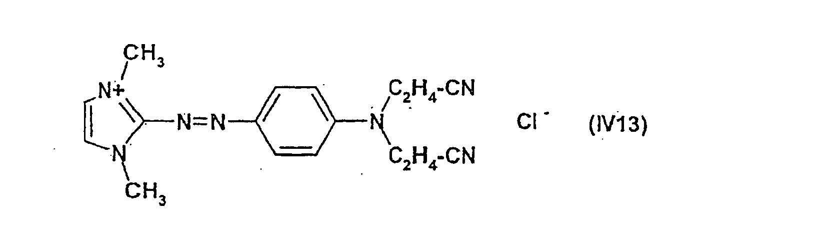

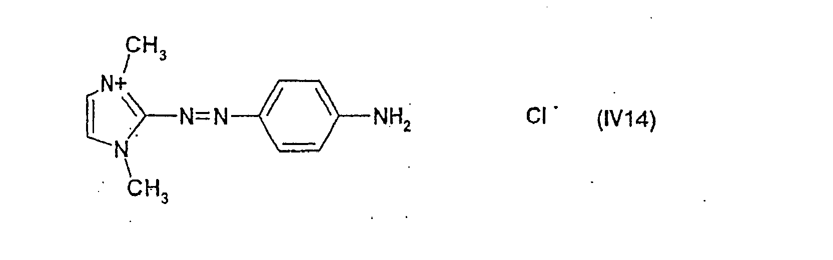

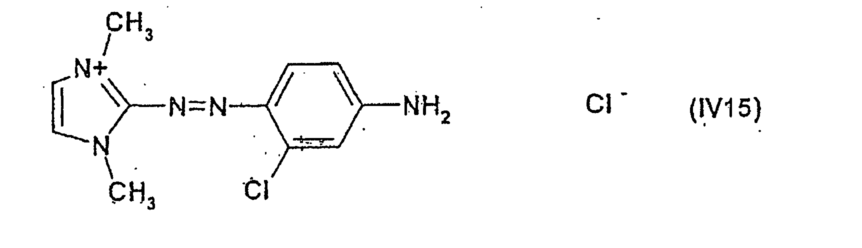

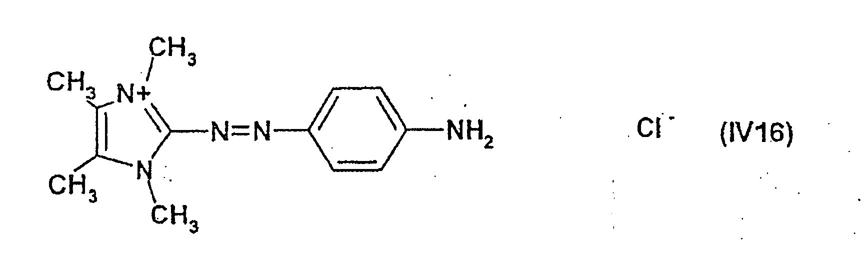

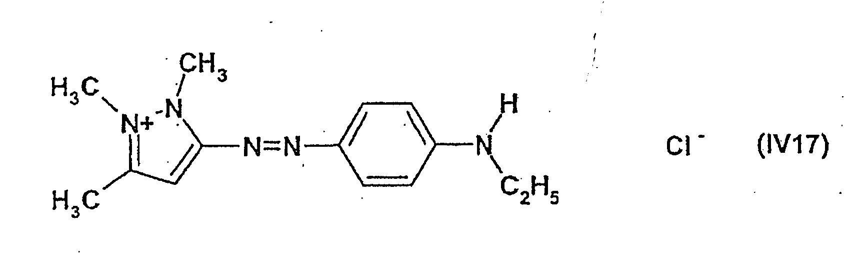

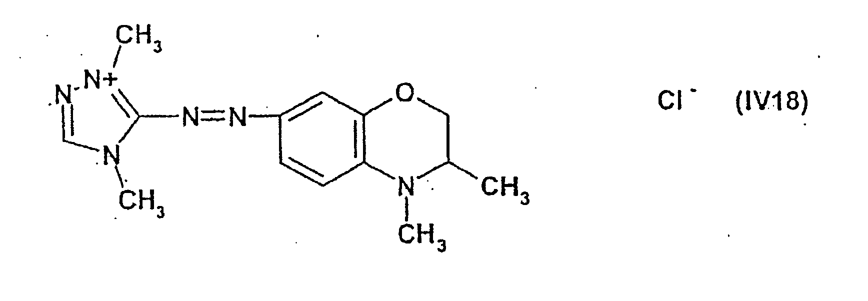

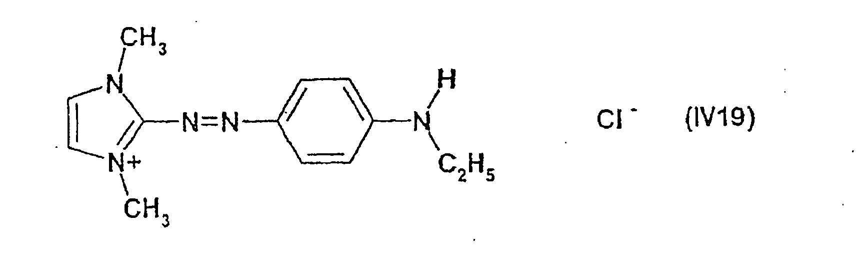

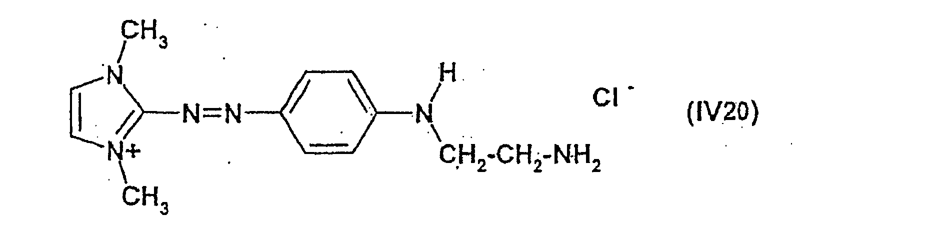

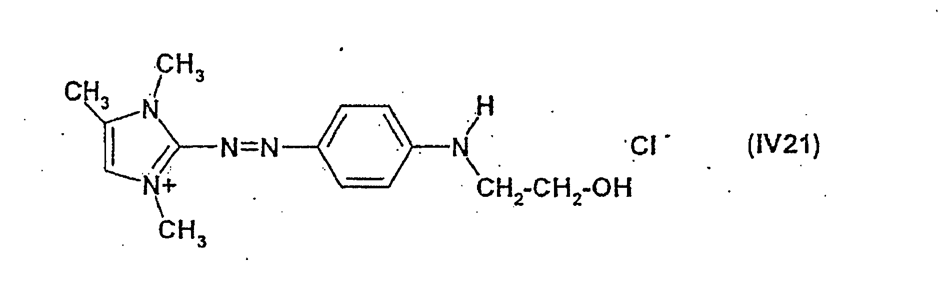

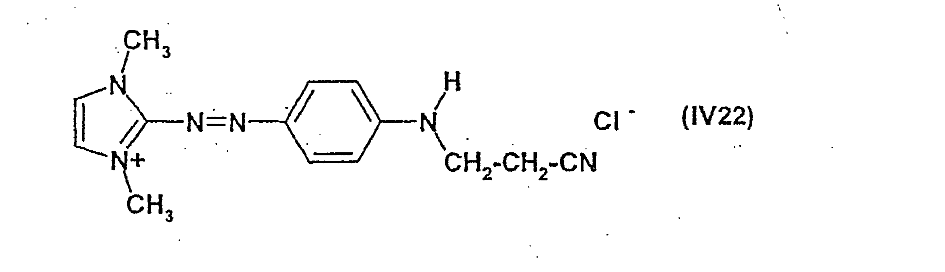

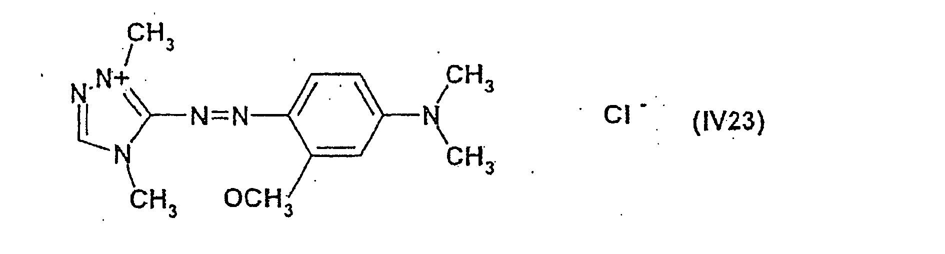

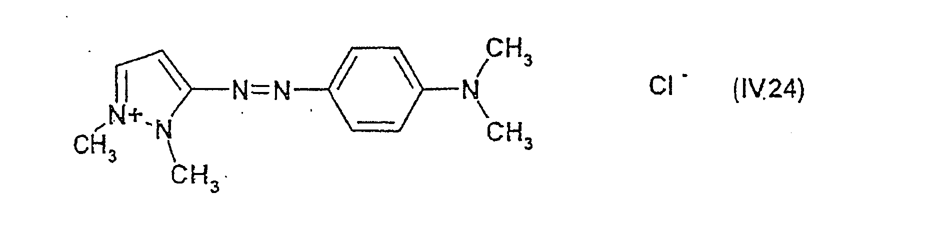

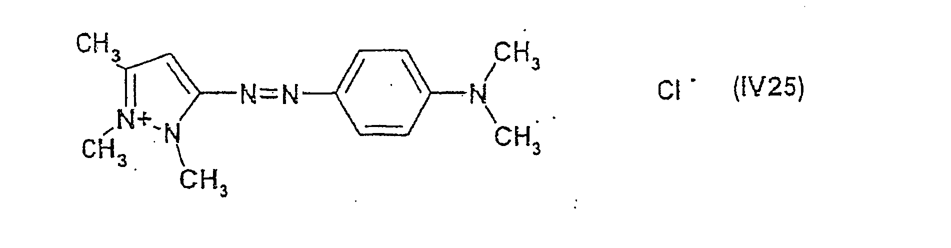

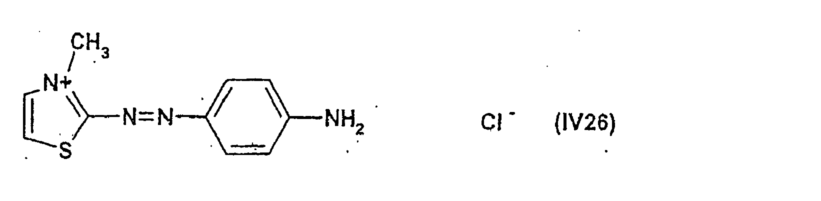

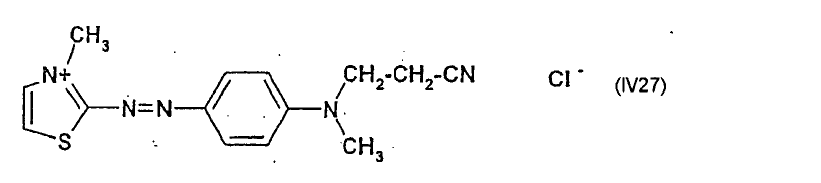

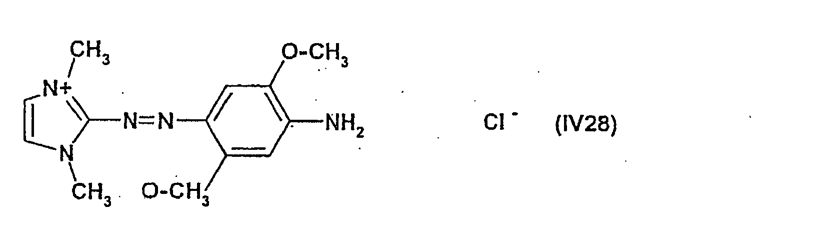

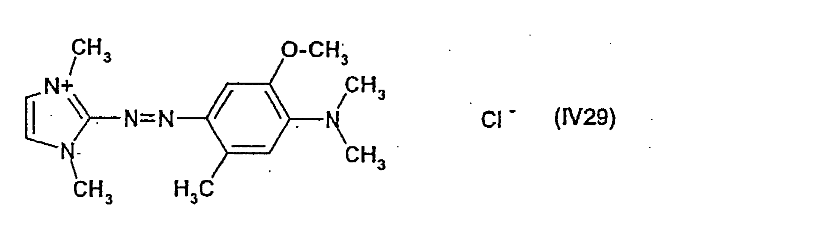

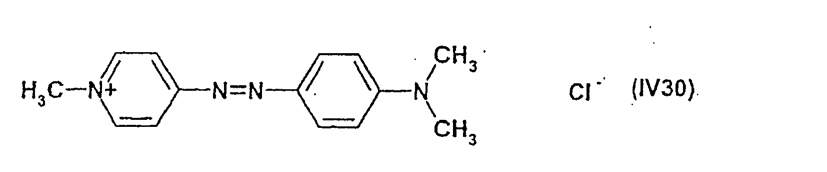

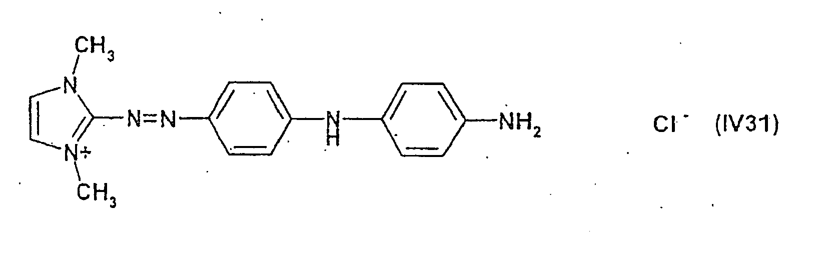

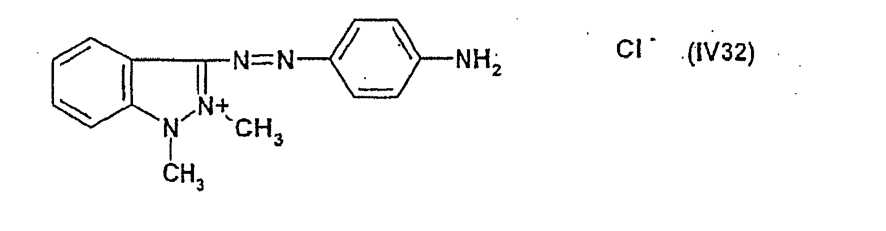

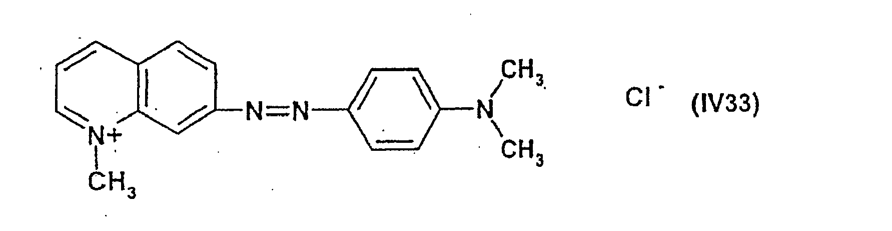

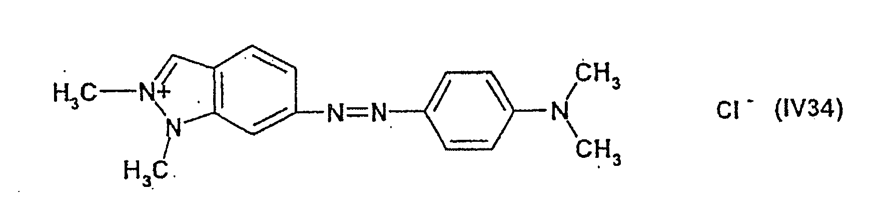

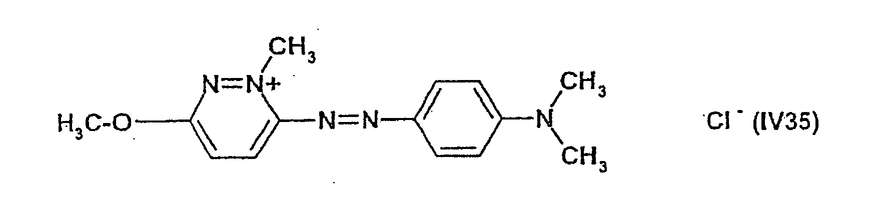

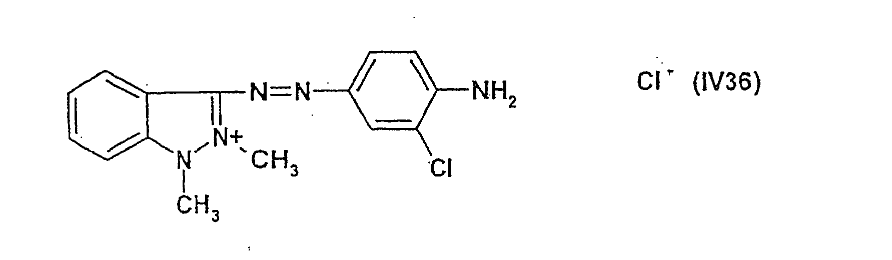

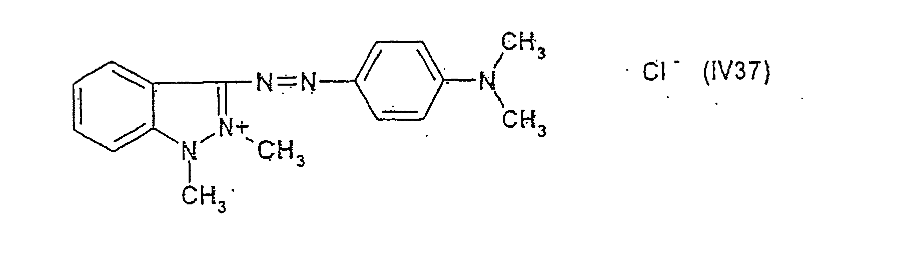

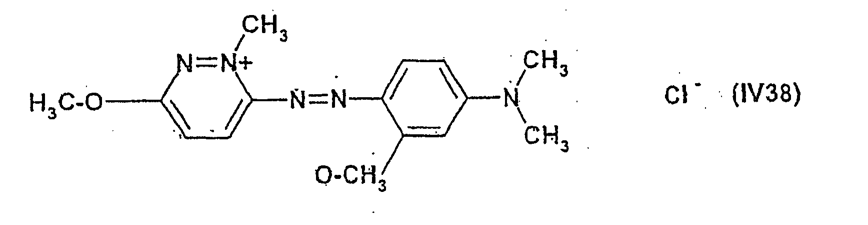

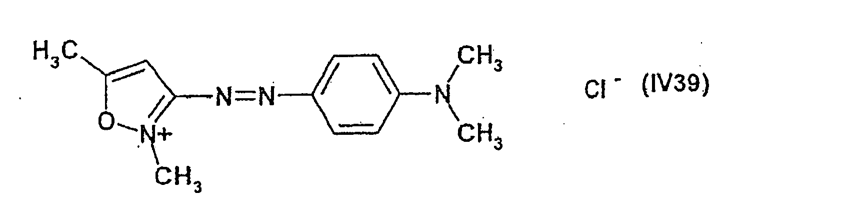

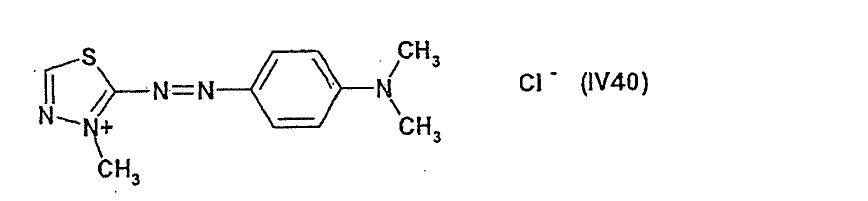

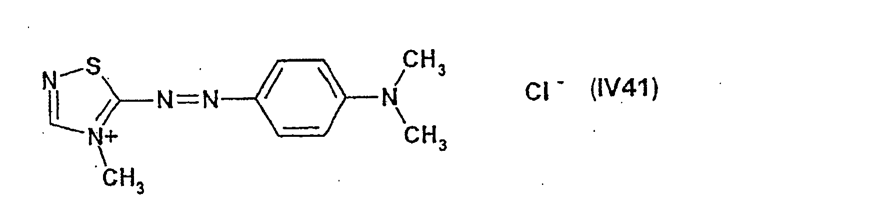

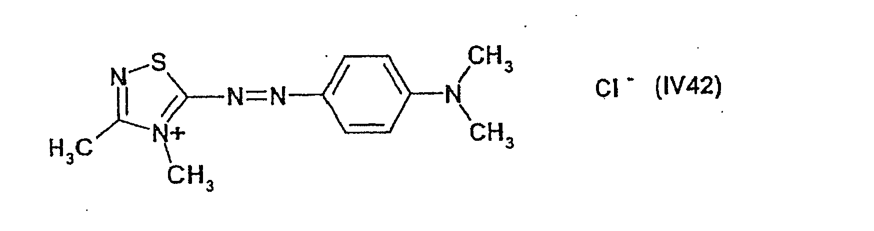

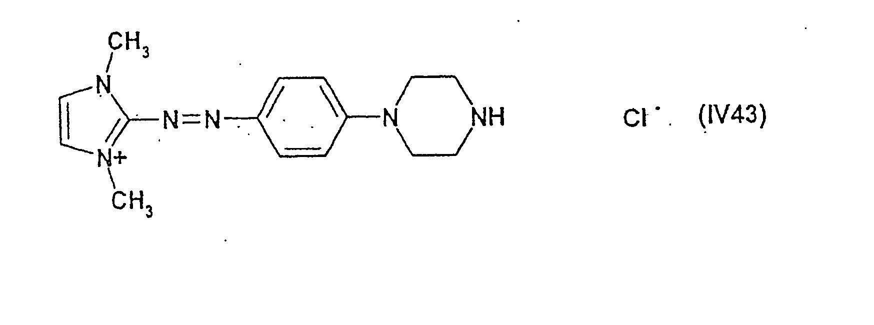

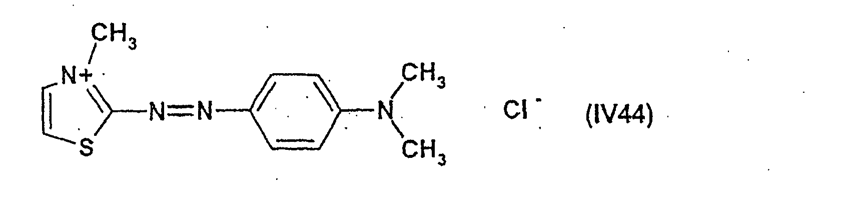

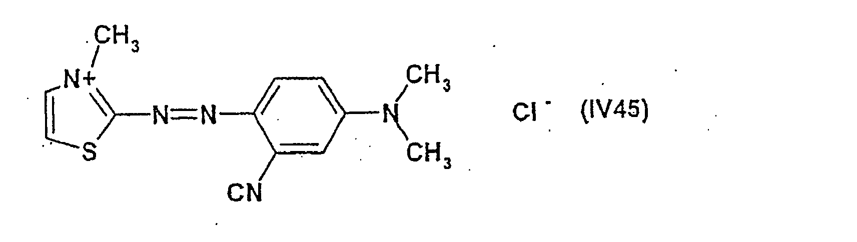

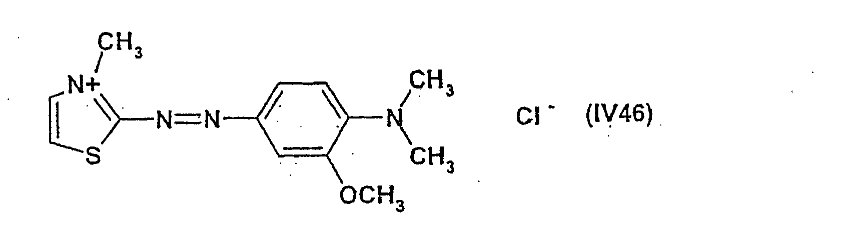

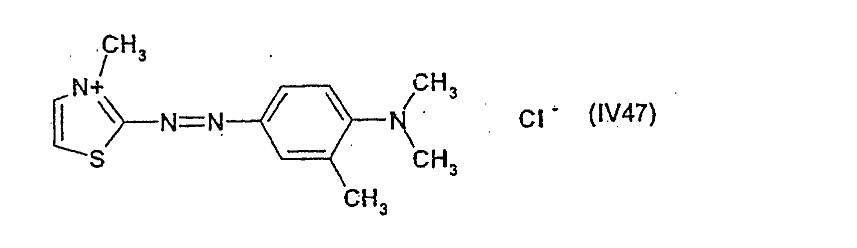

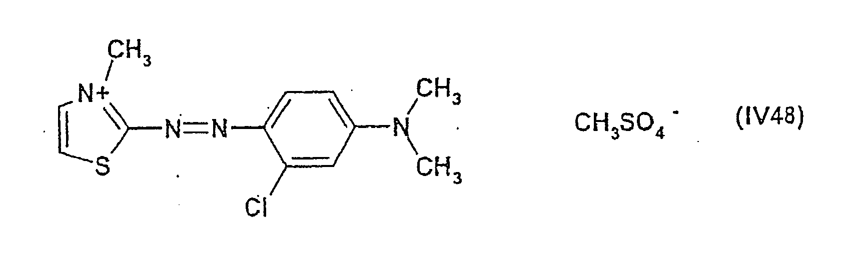

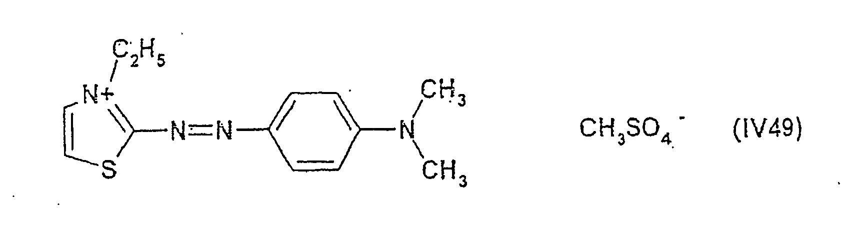

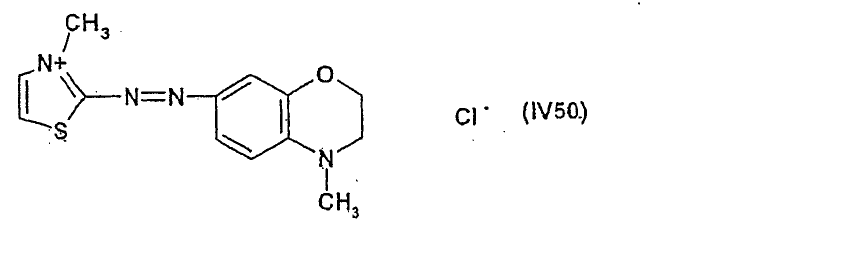

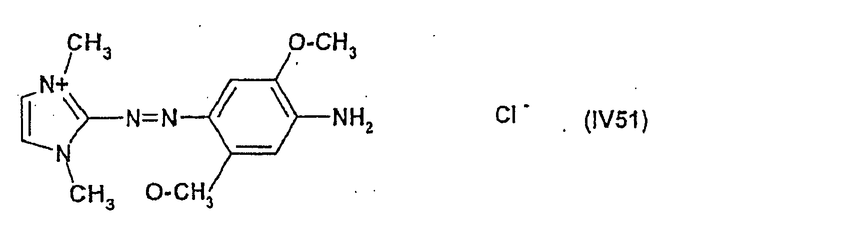

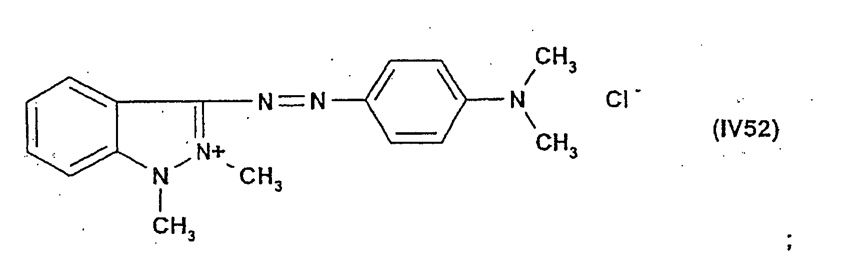

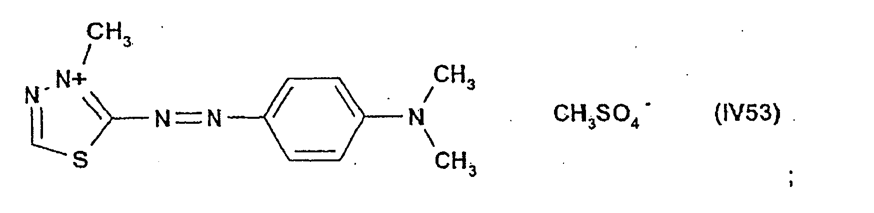

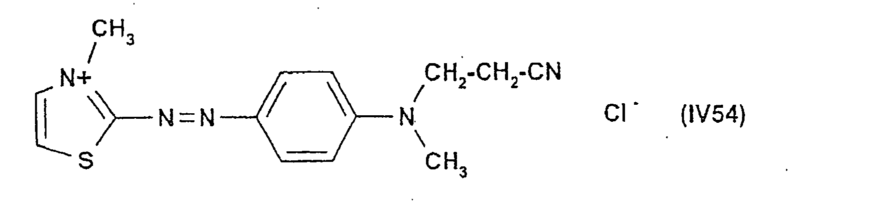

4. Use according to Claim 3, **characterized in that** the cationic dyes of formula (IV) are chosen from the compounds of formulae (IV1), (IV2), (IV14) and (IV31).

5. Use according to Claim 1, **characterized in that** the cationic dyes of formula (VI) are chosen from the compounds of formulae (VII) to (VI18) below: 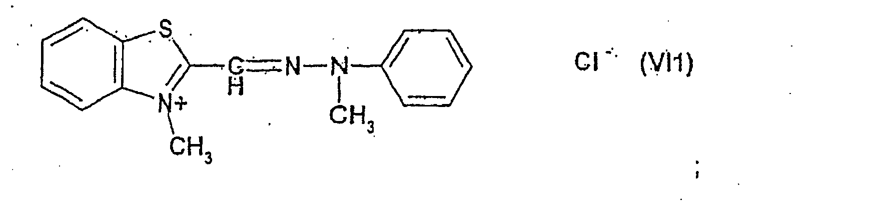 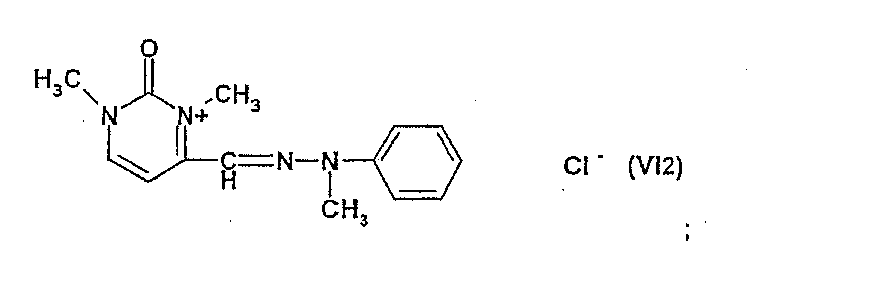 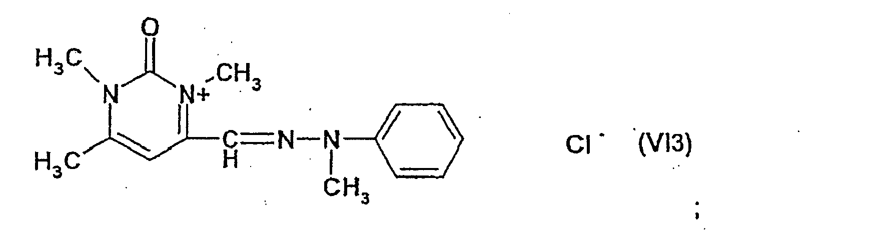 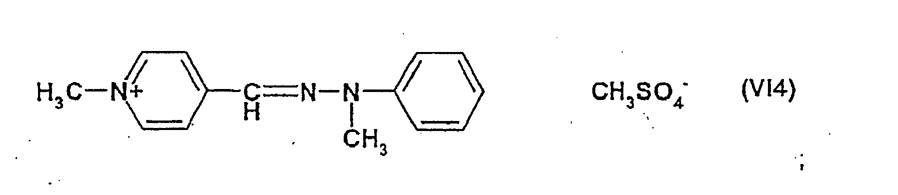 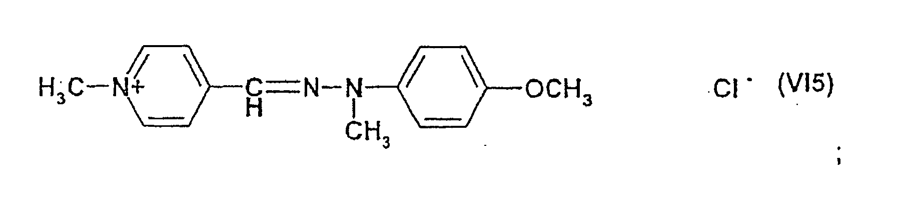 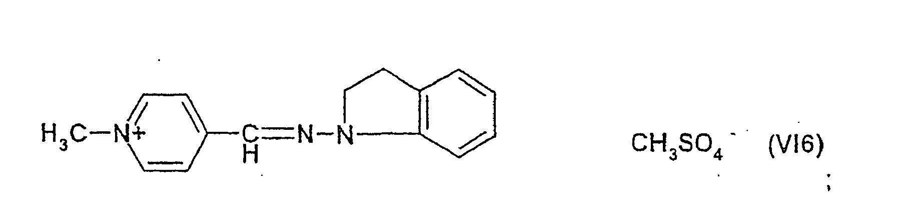 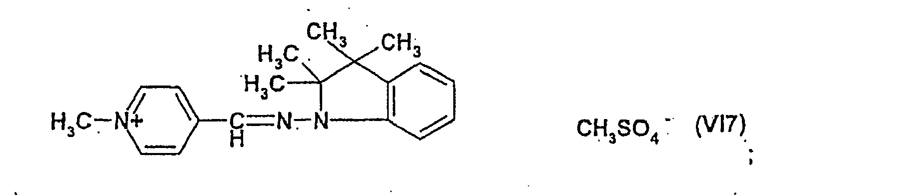 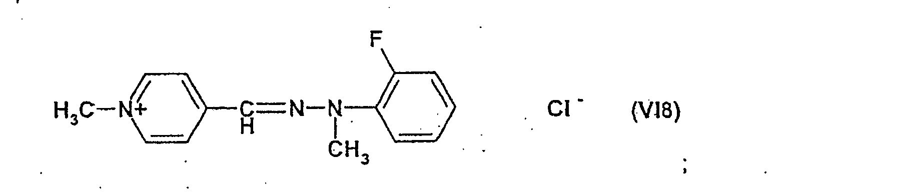 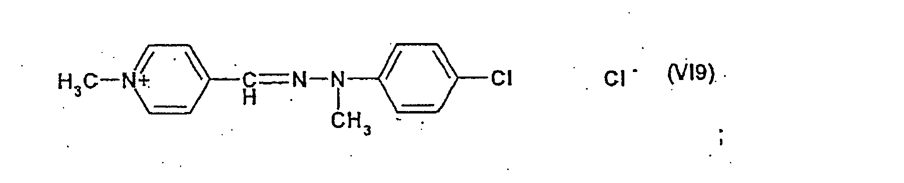 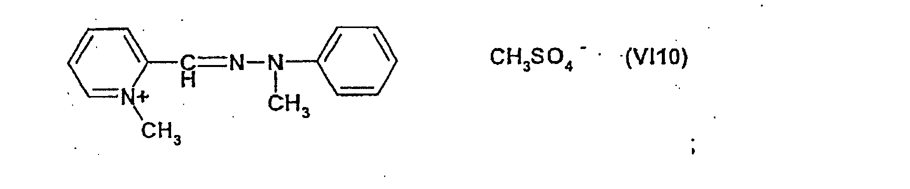 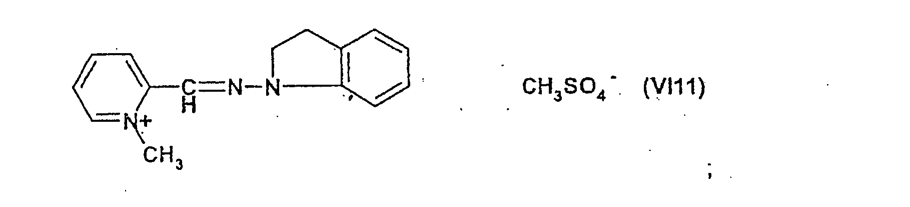 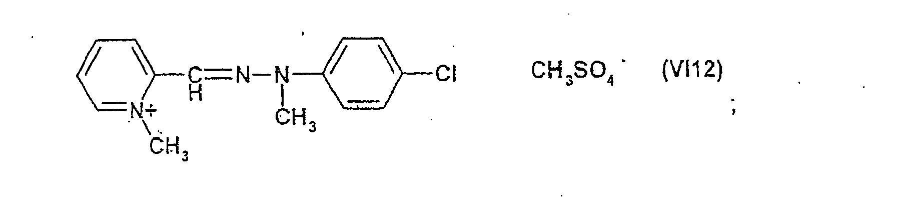 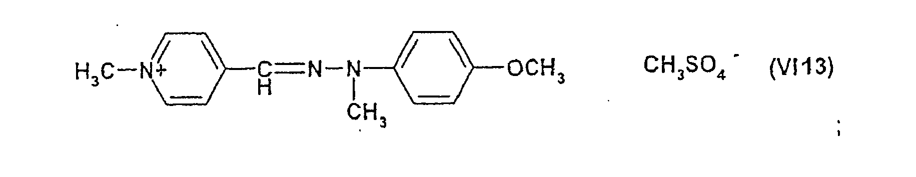 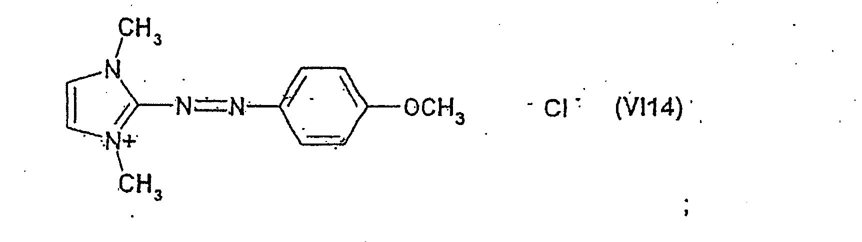 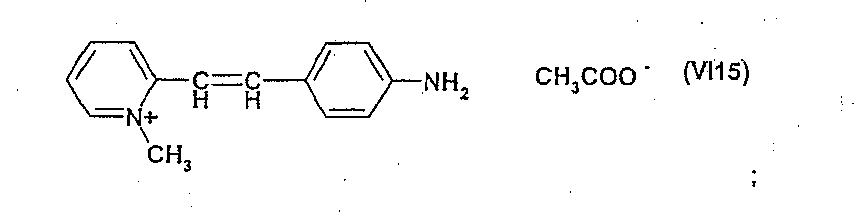 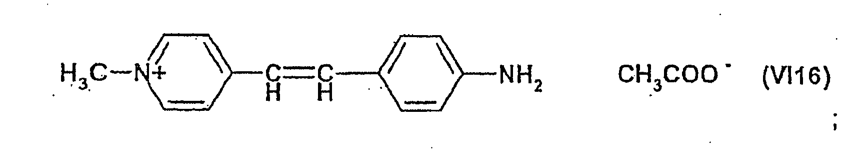 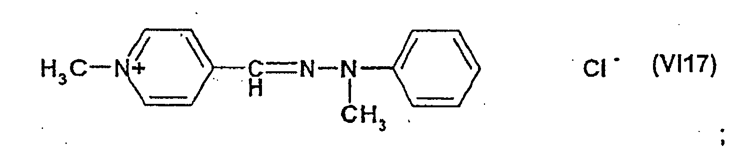 and

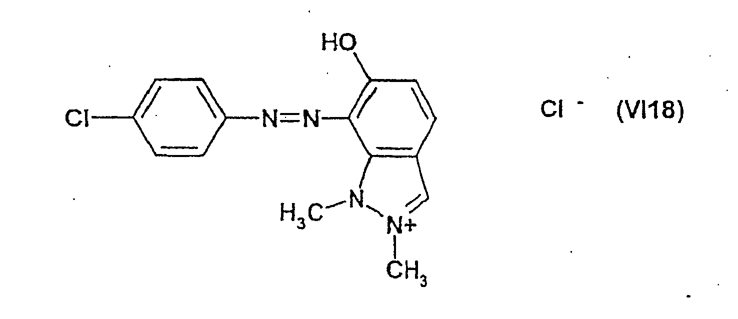

6. Use according to Claim 5, **characterized in that** the cationic dyes of formula (VI) are chosen from the compounds of formulae (VI4), (VI5) and (VI13).

7. Direct dye composition for keratin fibres, in particular for human keratin fibres and especially the hair, which contains no self-oxidizing dye, **characterized in that** it comprises, in a suitable dyeing medium, a combination of at least one dye of formulae (I) to (III) and of at least one dye of formula (IV) or (VI) defined in any one of Claims 1 to 6.

8. Composition according to Claim 7, **characterized in that** the cationic dye(s) (I), (II) and (III) are present in a concentration ranging from 0.001% to 10% by weight relative to the total weight of the composition.

9. Composition according to Claim 8, **characterized in that** the cationic dye(s) (I), (II) and (III) are present in a concentration ranging from 0.005% to 5% by weight relative to the total weight of the composition.

10. Composition according to Claim 7, **characterized in that** the cationic dye(s) of formulae (IV) and (VI) represent from 0.001% to 10% by weight relative to the total weight of the composition.

11. Composition according to Claim 10, **characterized in that** the cationic dye(s) of formulae (IV) and (VI) represent from 0.001% to 10% by weight relative to the total weight of the composition.

12. Composition according to any one of Claims 7 to 11, **characterized in that** the suitable dyeing medium (or support) consists of water or of a mixture of water and of at least one organic solvent.

13. Composition according to any one of Claims 7 to 12, **characterized in that** it has a pH of between 2 and 11 and preferably between 5 and 10.

14. Composition according to any one of Claims 7 to 13, **characterized in that** it contains additives chosen from antioxidants, penetrants, sequestrants, thickeners, fragrances, buffers, dispersants, surfactants, polymers, film-forming agents, ceramides, preserving agents, screening agents and opacifiers.

15. Process for the direct dyeing of keratin fibres and in particular of human keratin fibres such as the hair, **characterized in that** at least one dye composition as defined in any one of Claims 7 to 14 is applied to the fibres for a time that is sufficient to develop the desired coloration, after which the fibres are rinsed, optionally washed with shampoo, rinsed again and dried.

16. Process for the direct dyeing of keratin fibres and in particular of human keratin fibres such as the hair, **characterized in that** at least one dye composition as defined in any one of Claims 7 to 14 is applied to the fibres for a time that is sufficient to develop the desired coloration, without final rinsing.

17. Multi-compartment device or multi-compartment dyeing "kit", **characterized in that** a first compartment contains at least one cationic dye (I) to (III) defined in Claim 1 and a second compartment contains at least one cationic dye of formula (IV) or (VI) defined in any one of Claims 1 and 3 to 6.

18. Multi-compartment device or multi-compartment dyeing "kit", **characterized in that** a first compartment contains a pulverulent composition containing the combination of at least one cationic dye (I) to (III) defined in Claim 1 and of at least one dye of formula (IV) or (VI) defined in any one of Claims 1 and 3 to 6, and **in that** a second compartment contains an aqueous composition serving as vehicle for the dye.

## Patentansprüche

1. Verwendung einer Kombination, die (i) mindestens einen kationischen Farbstoff, der unter (I) Basic Brown 17, Basic Brown 16, Basic Red 76, Basic Red 118, (II) Basic Yellow 57 und (III) Basic Blue 99 ausgewählt ist, und (ii) mindestens einen kationischen Farbstoff der folgenden Formeln (IV) oder (VI) enthält, in Zusammensetzungen für die Direktfärbung von Keratinfasern und insbesondere menschlichen Keratinfasern, wie Haaren, die keinen selbstoxidierfähigen Farbstoff enthalten, oder zur Herstellung solcher Zusammensetzungen:
(ii) kationischer Farbstoff der folgenden Formeln (IV) und (VI):
(a) Verbindungen der folgenden Formel (IV): 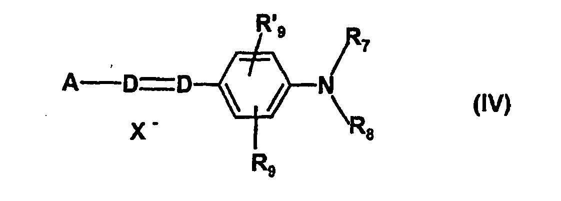 worin bedeuten:
D ein Stickstoffatom oder die Gruppe -CH,
R₇ und R₈, die gleich oder verschieden sind, ein Wasserstoffatom; eine C₁₋₄-Alkylgruppe, die mit einer Gruppe -CN, -OH oder -NH₂ substituiert sein kann, oder sie bilden mit einem Kohlenstoffatom des Benzolrings einen gegebenenfalls sauerstoff- oder stickstoffhaltigen Heterocyclus, der mit einer oder mehreren C₁₋₄-Alkylgruppen substituiert sein kann; oder eine 4'-Aminophenylgruppe,
R₉ und R'₉, die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder Acetyloxy,
X ein Anion, das vorzugsweise unter Chlorid, Methylsulfat und Acetat ausgewählt ist,
A eine Gruppe, die unter den folgenden Strukturen A1 bis A19 ausgewählt ist: 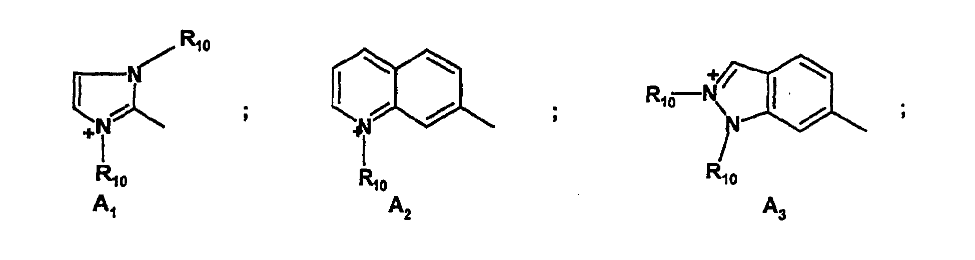 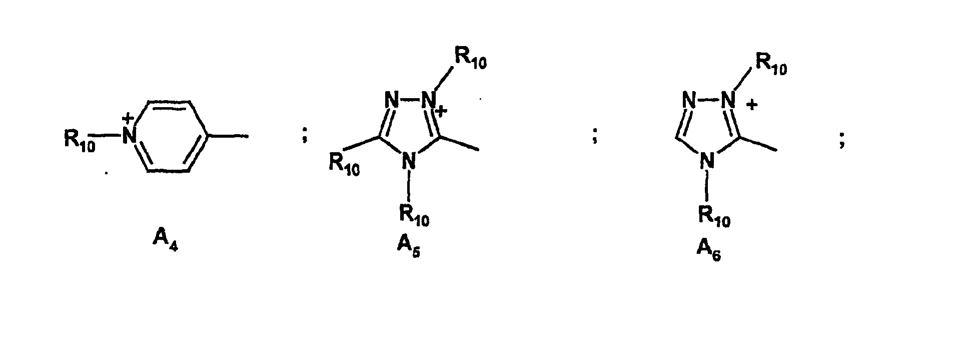 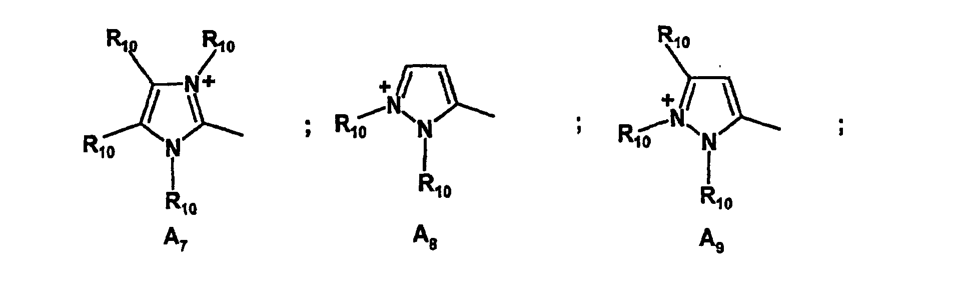 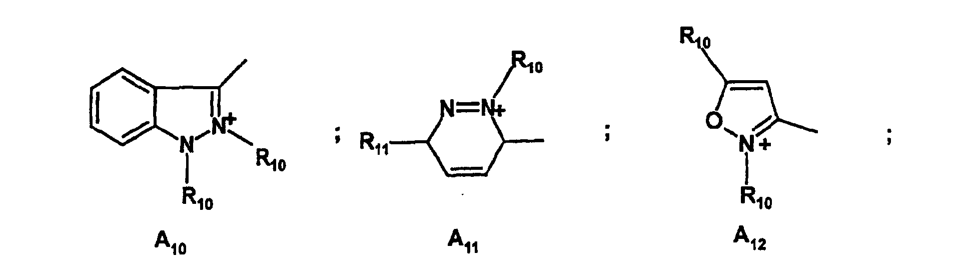 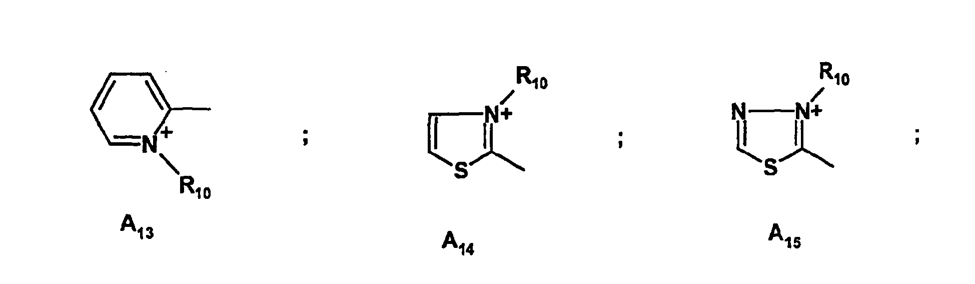 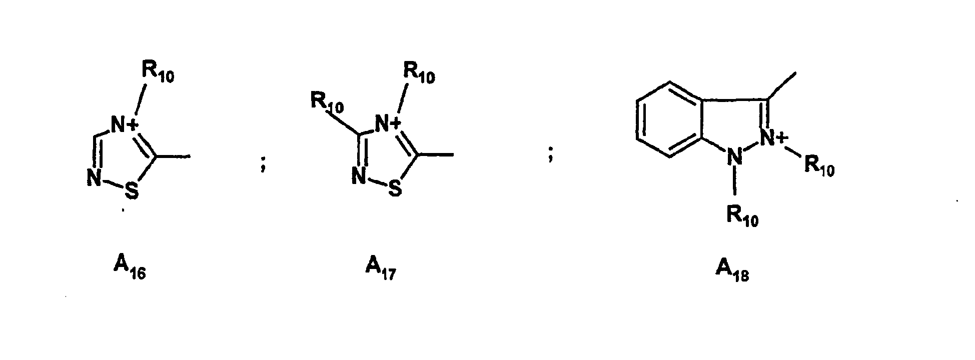 und 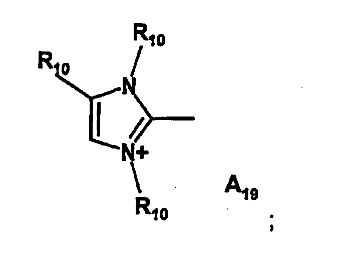
worin die Gruppe Rio eine C₁₋₄-Alkylgruppe bedeutet, die mit einer Hydroxygruppe substituiert sein kann, und R₁₁ eine C₁₋₄-Alkoxygruppe ist, mit der Maßgabe, dass
(i) die Gruppen R₇ und R₈ nicht gleichzeitig ein Wasserstoffatom bedeuten, wenn D die Gruppe -CH ist, die Gruppe A A₄ oder A₁₃ bedeutet und R₉ von Alkoxy verschieden ist;
(ii) die Gruppen R₇ und R₈ nicht gleichzeitig die Methylgruppe bedeuten, wenn D ein Stickstoffatom ist und die Gruppe A A₆ bedeutet;
(c) Verbindungen der folgenden Formel (VI): 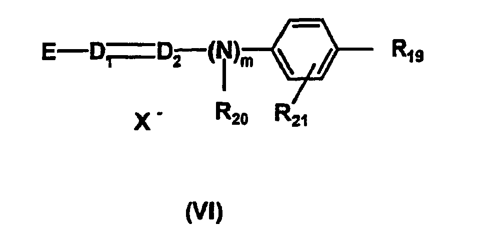 worin bedeuten:
R₁₉ ein Wasserstoffatom, eine C₁₋₄-Alkoxygruppe, ein Halogenatom, wie Brom, Chlor, Iod oder Fluor, oder eine Aminogruppe,
R₂₀ ein Wasserstoffatom oder eine C₁₋₄-Alkylgruppe oder R₂₀ bildet mit einem Kohlenstoffatom des Benzolrings einen Heterocyclus, der gegebenenfalls sauerstoffhaltig und/oder mit einer oder mehreren C₁₋₄-Alkylgruppen substituiert ist;
R₂₁ ein Wasserstoffatom oder ein Halogenatom, wie Brom, Chlor, Iod oder Fluor,
D₁ und D₂, die gleich oder verschieden sind, ein Stickstoffatom oder die Gruppe -CH,
m = 0 oder 1,
mit der Maßgabe, dass die Gruppen D₁ und D₂ gleichzeitig -CH bedeuten und m = 0, wenn R₁₉ eine unsubstituierte Aminogruppe ist,
X- ein Anion, das vorzugsweise unter Chlorid, Methylsulfat und Acetat ausgewählt ist,
E eine Gruppe, die unter den folgenden Strukturen E1 bis E8 ausgewählt ist: 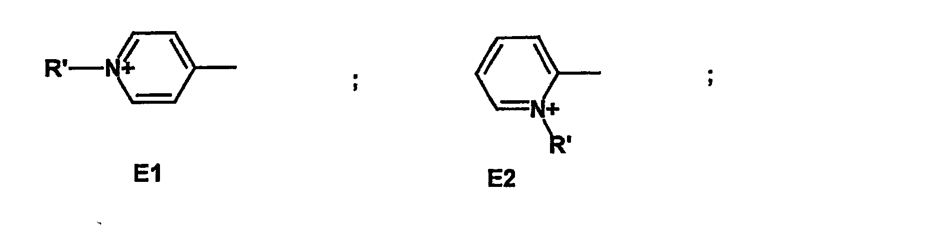 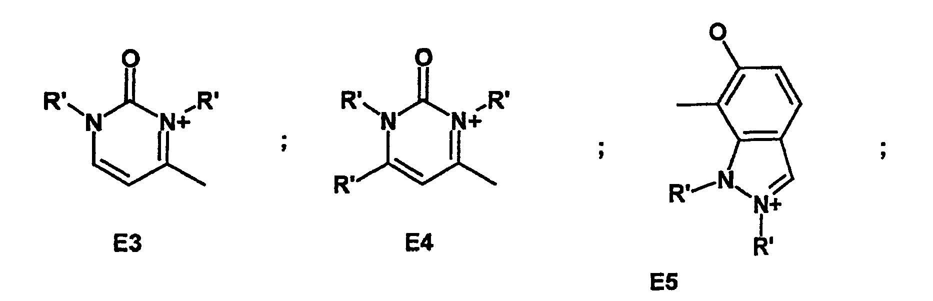 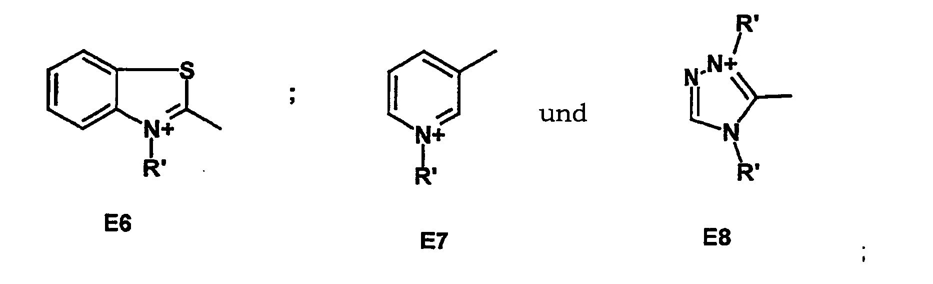
wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 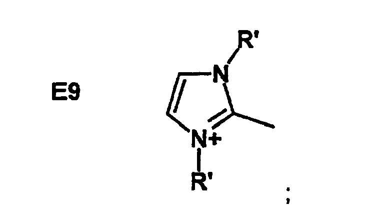 wobei R' eine C₁₋₄-Alkylgruppe bedeutet.

2. Verwendung nach Anspruch 1, **dadurch gekennzeichnet, dass** es sich bei dem kationischen Farbstoff um Basic Blue 99 handelt.

3. Verwendung nach Anspruch 1, **dadurch gekennzeichnet, dass** die kationischen Farbstoffe der Formel (IV) unter den Verbindungen der folgenden Strukturen (IV1) bis (IV54) ausgewählt sind:

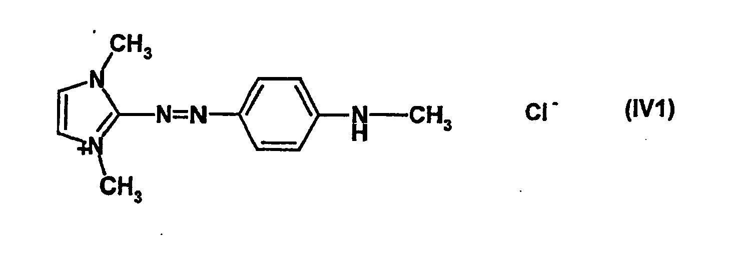

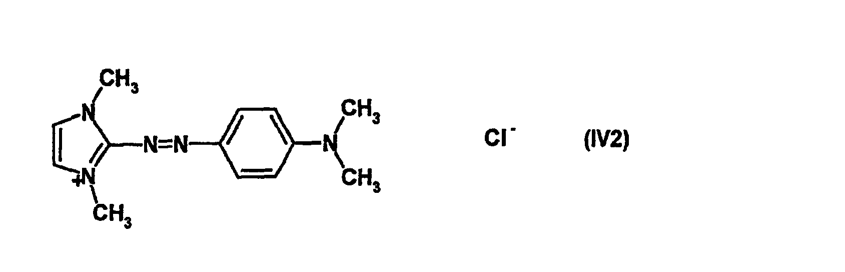

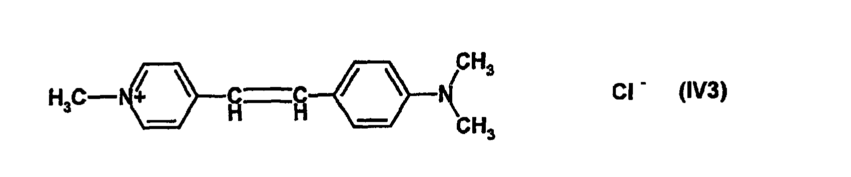

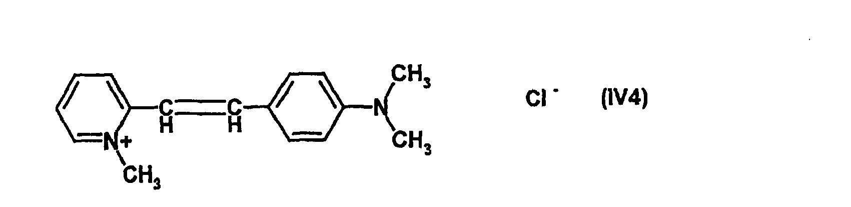

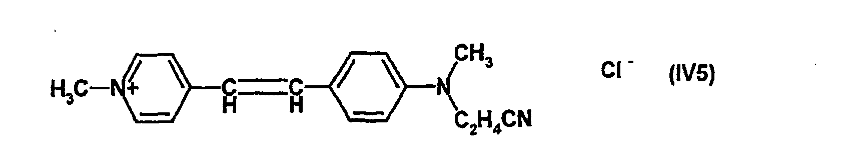

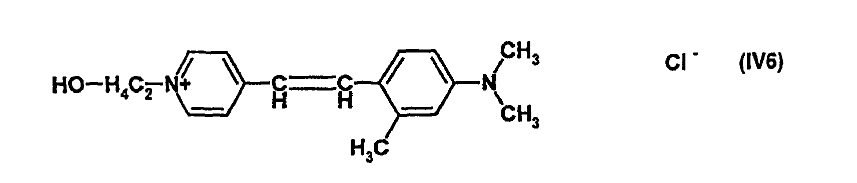

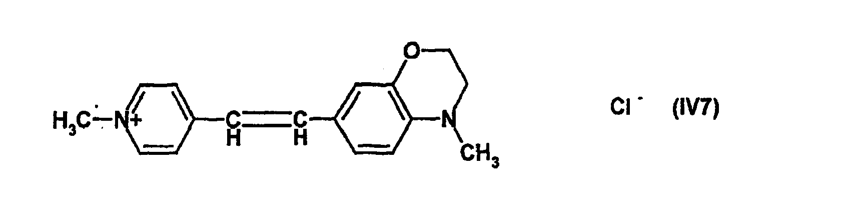

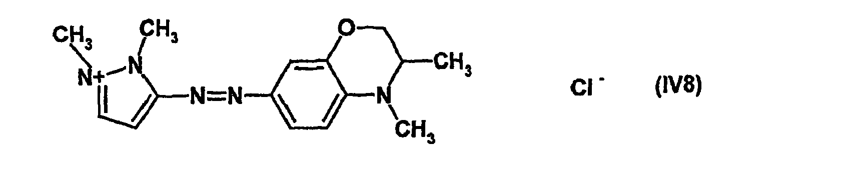

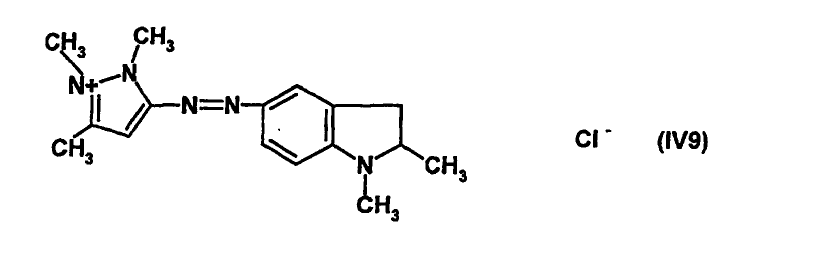

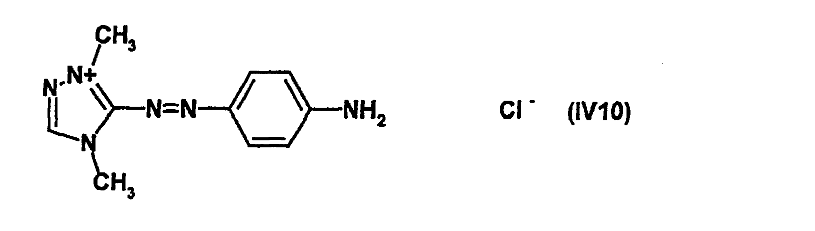

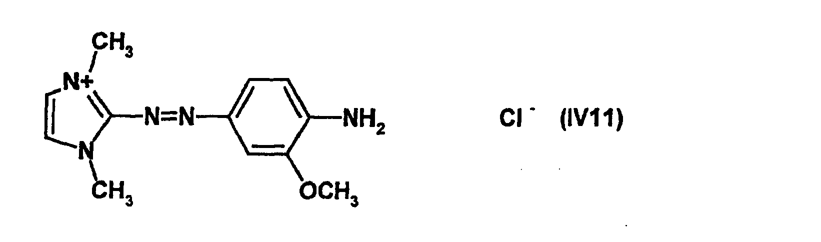

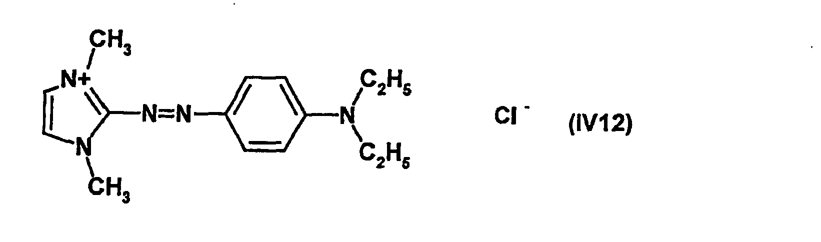

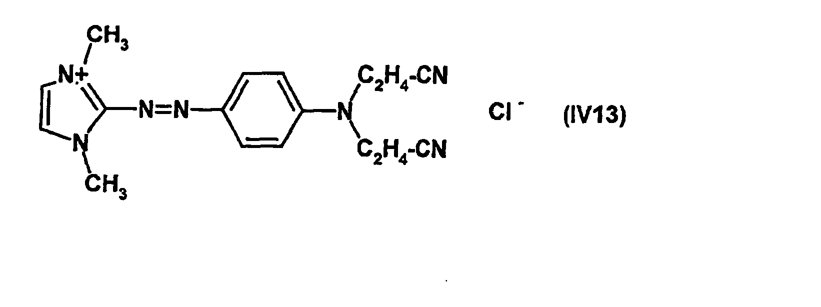

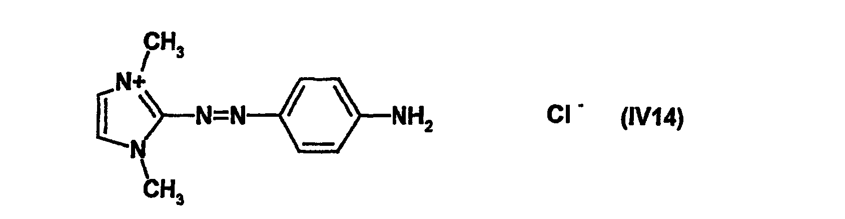

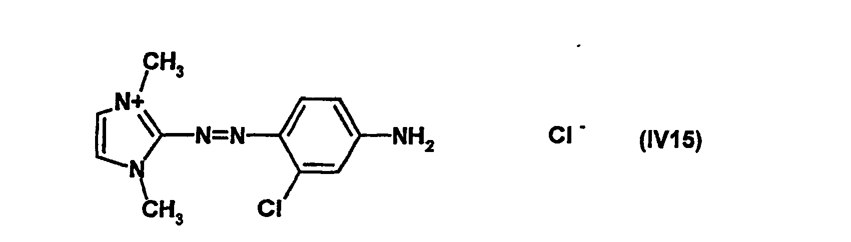

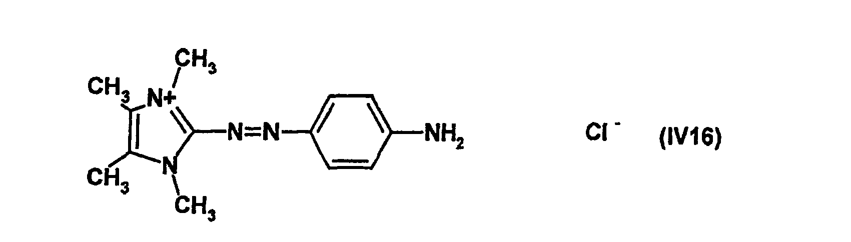

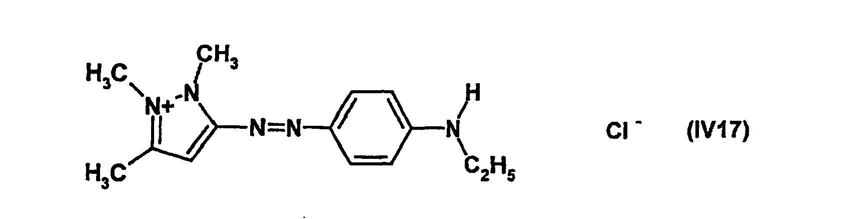

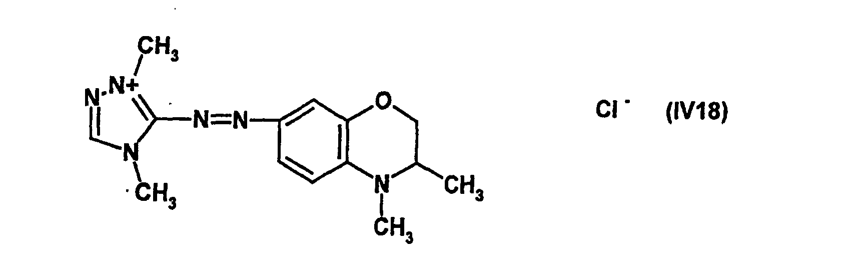

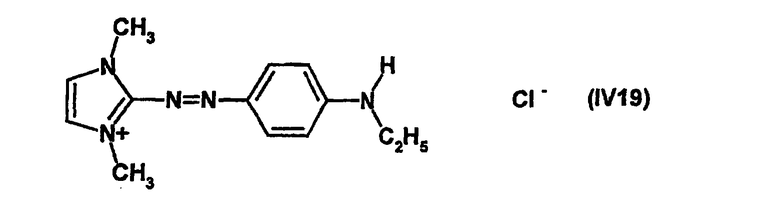

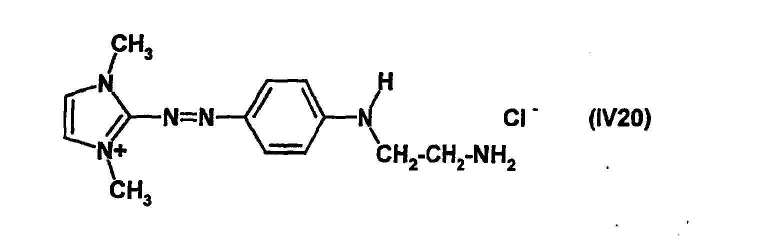

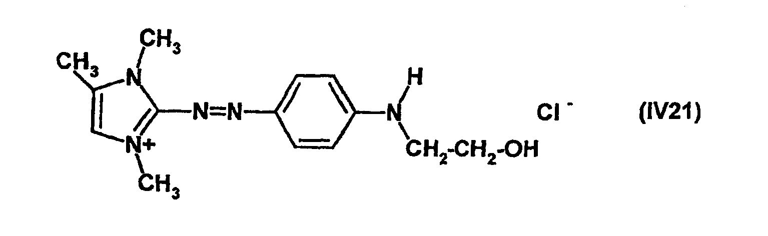

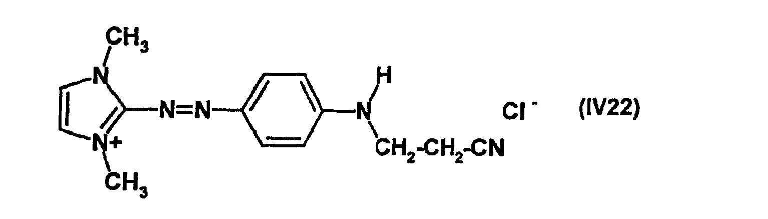

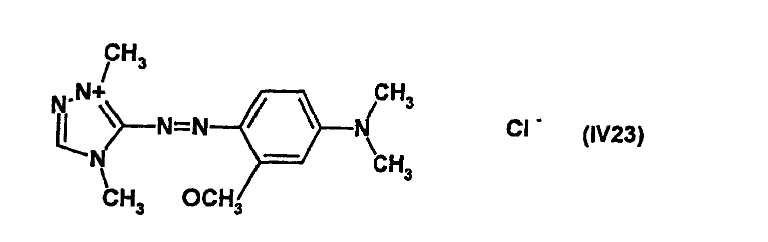

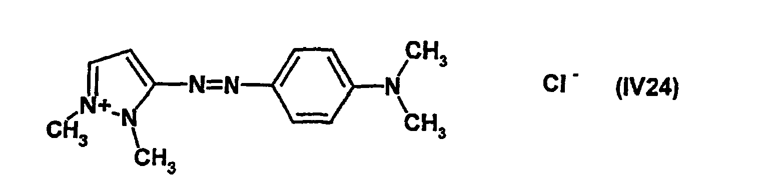

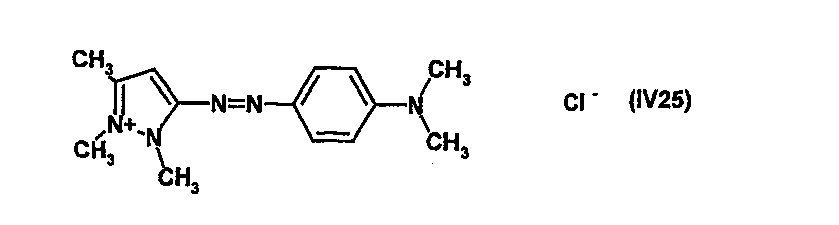

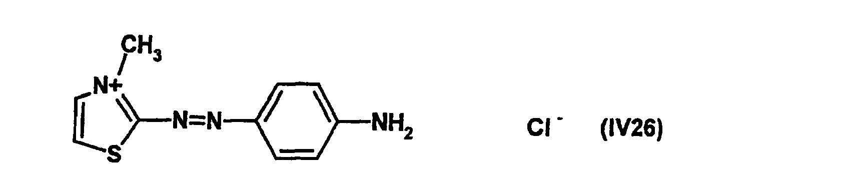

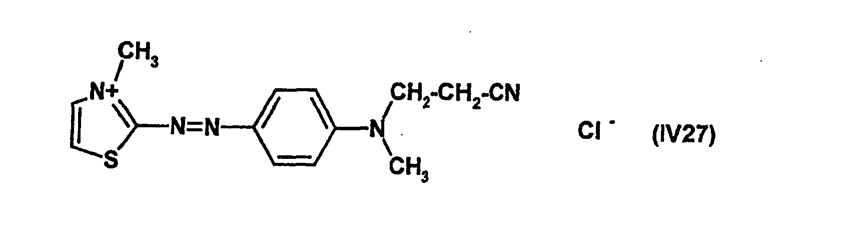

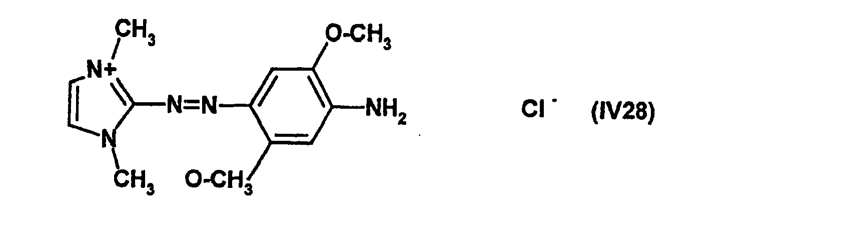

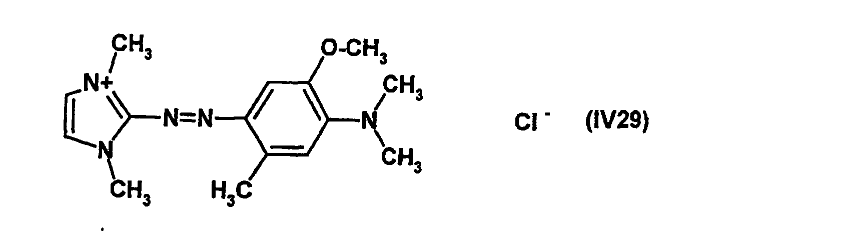

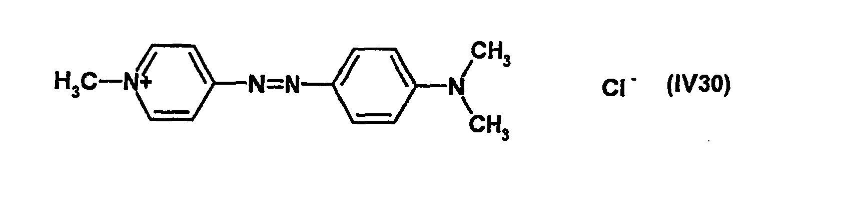

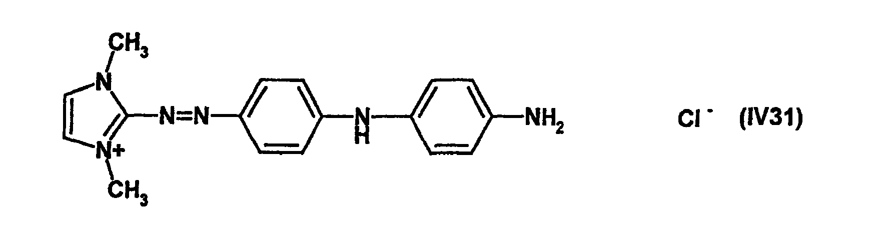

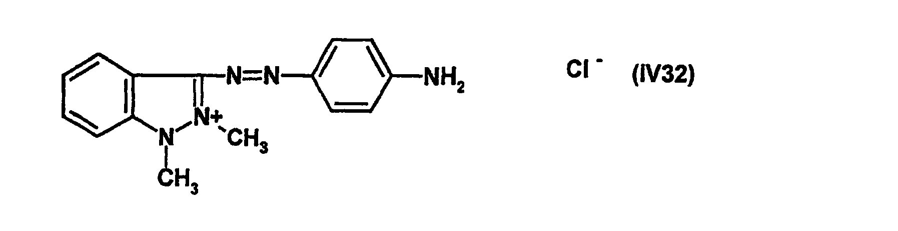

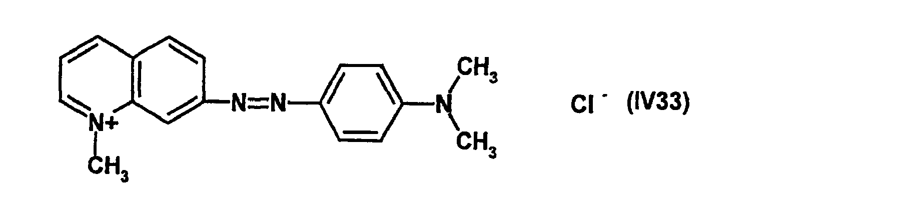

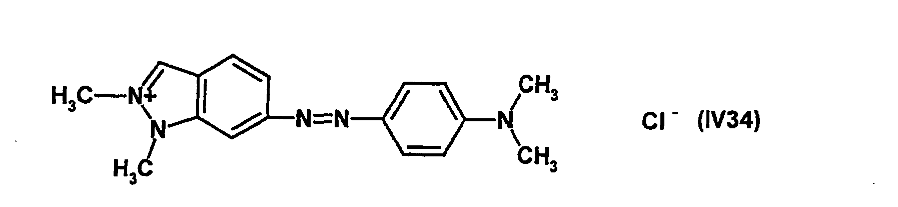

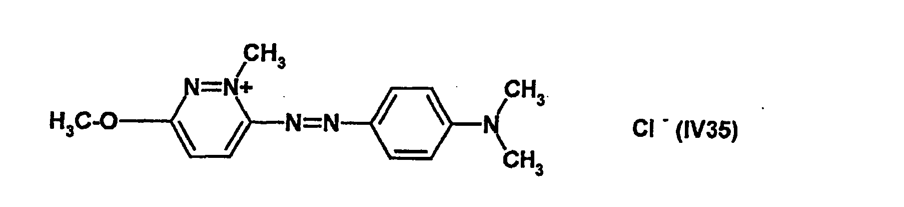

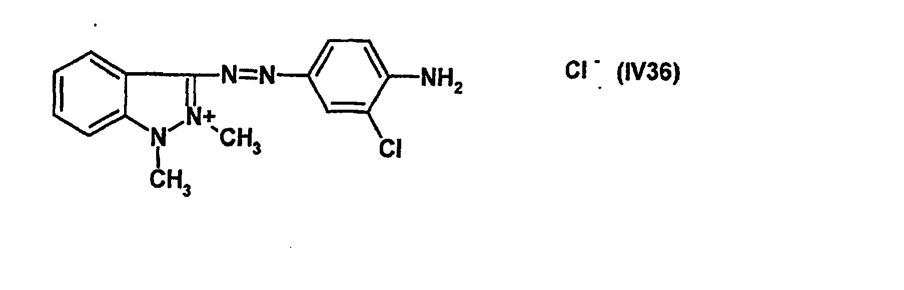

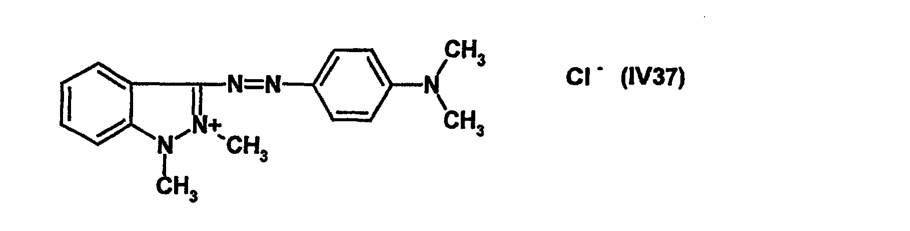

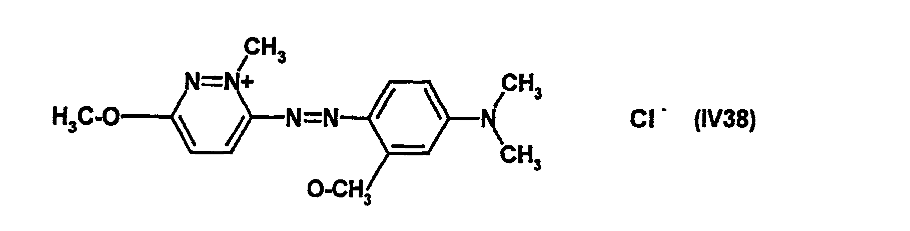

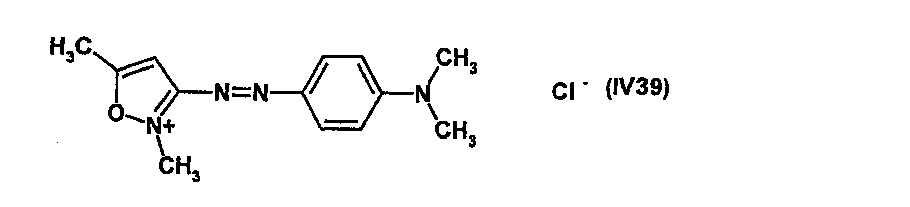

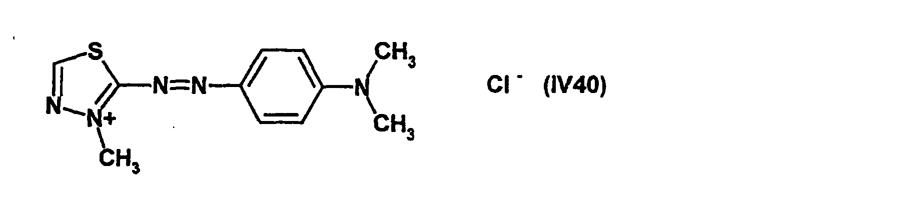

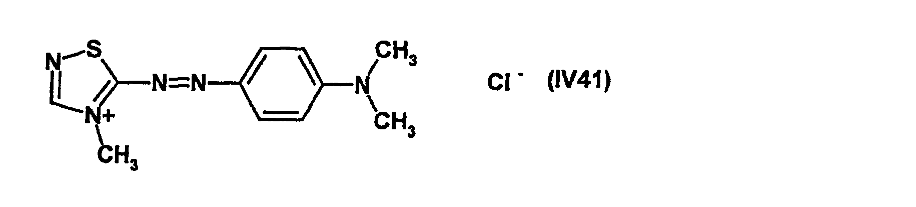

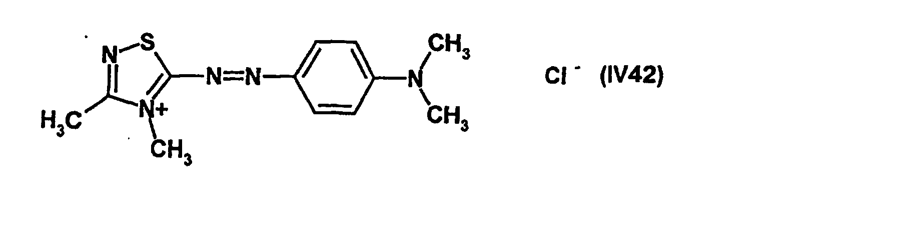

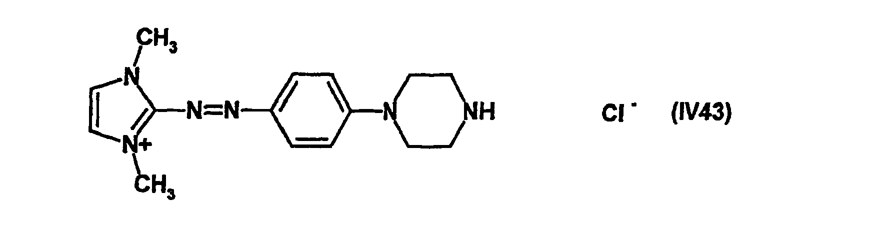

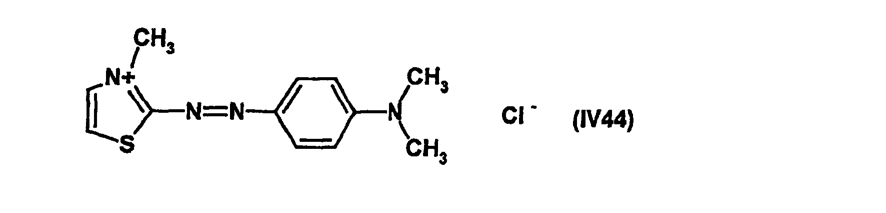

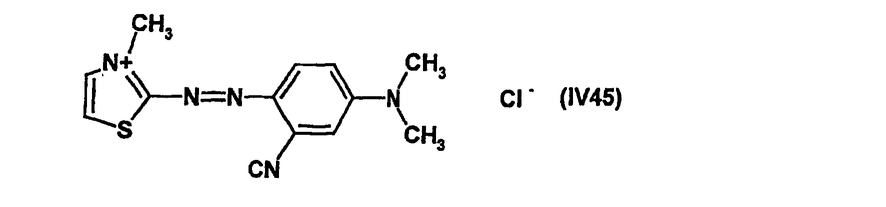

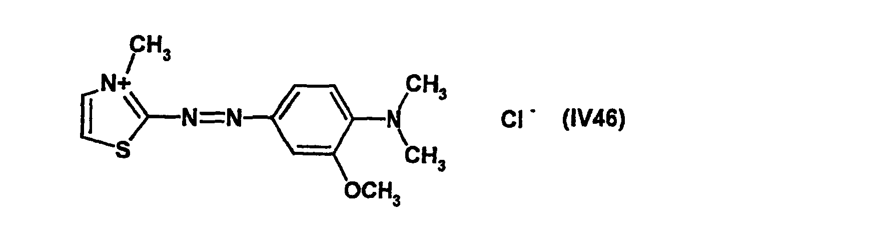

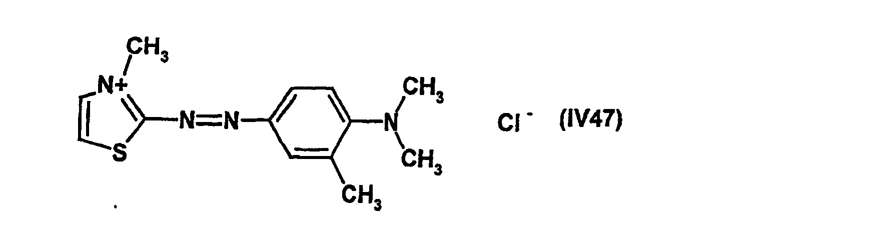

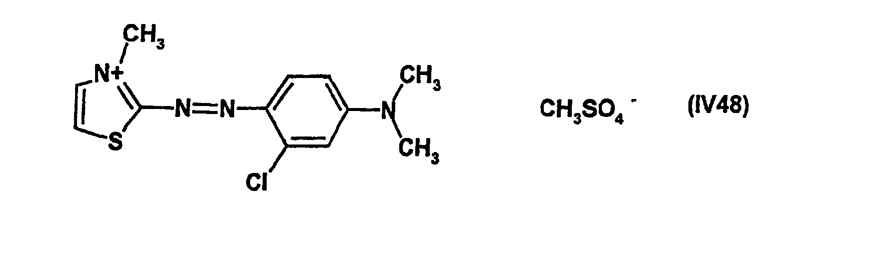

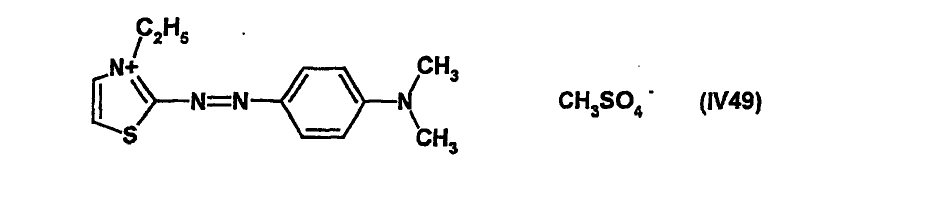

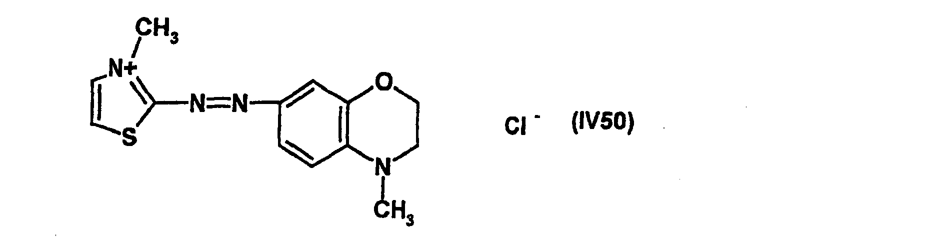

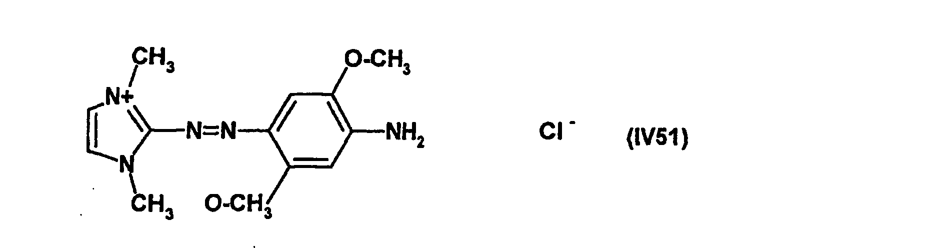

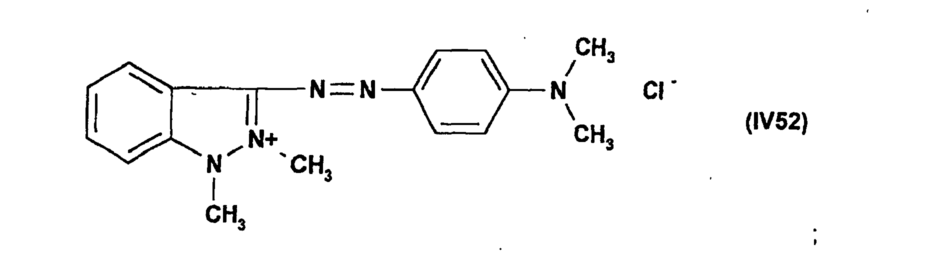

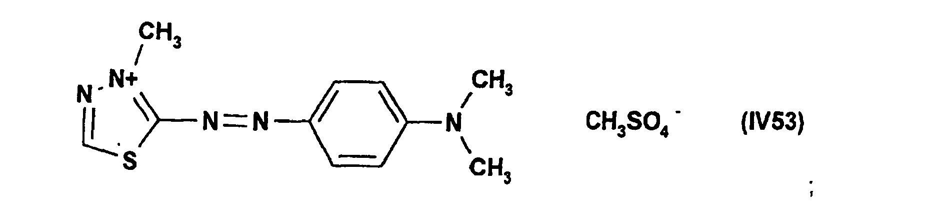

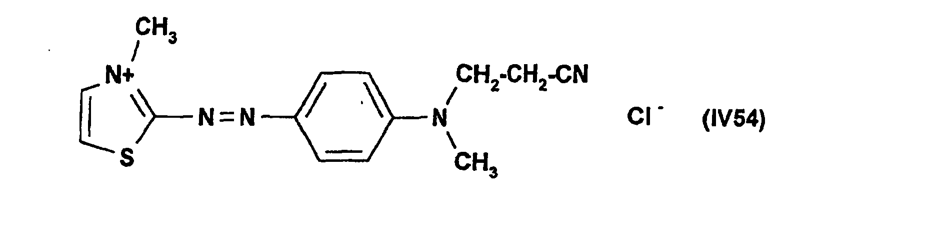

4. Verwendung nach Anspruch 3, **dadurch gekennzeichnet, dass** die kationischen Farbstoffe der Formel (IV) den Strukturen (IV1), (IV2), (IV14) und (IV31) entsprechen.

5. Verwendung nach Anspruch 1, **dadurch gekennzeichnet, dass** die kationischen Farbstoffe der Formel (VI) unter den Verbindungen der folgenden Strukturen (VI1) bis (VI 18) ausgewählt sind:

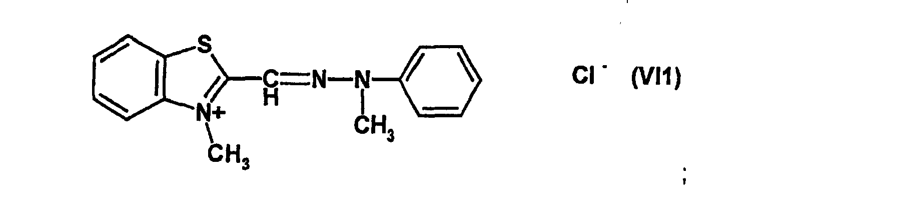

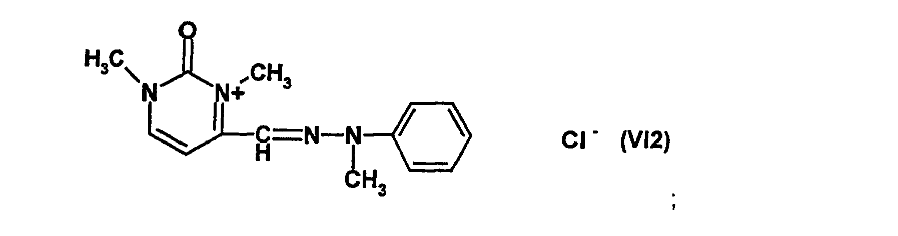

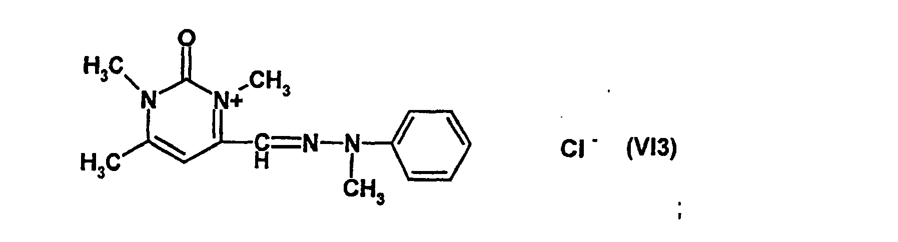

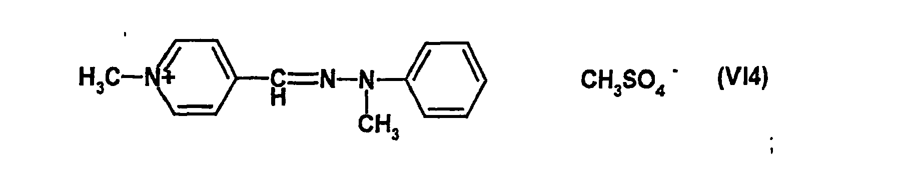

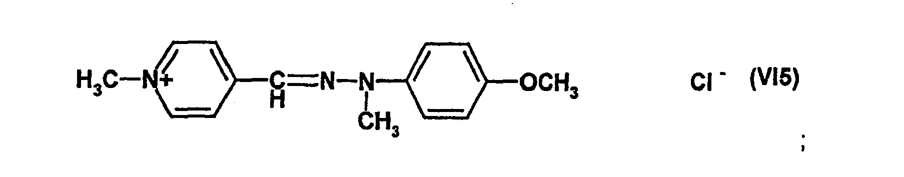

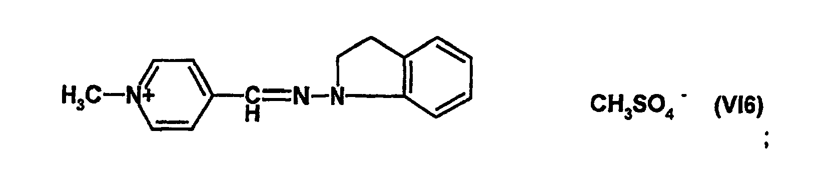

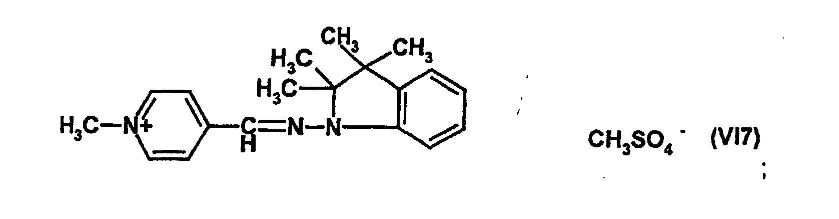

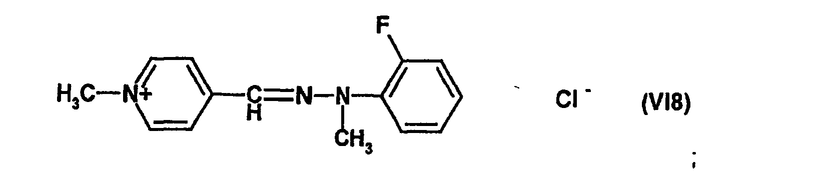

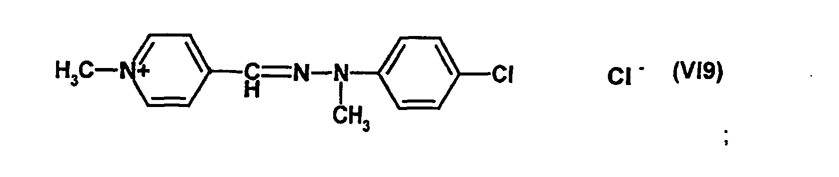

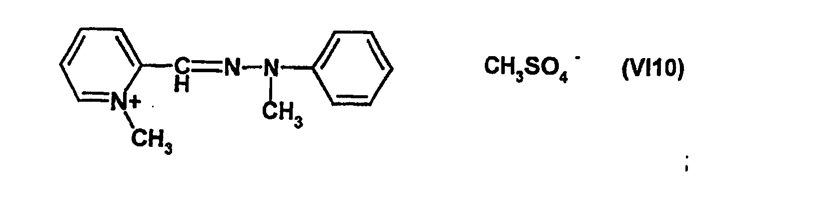

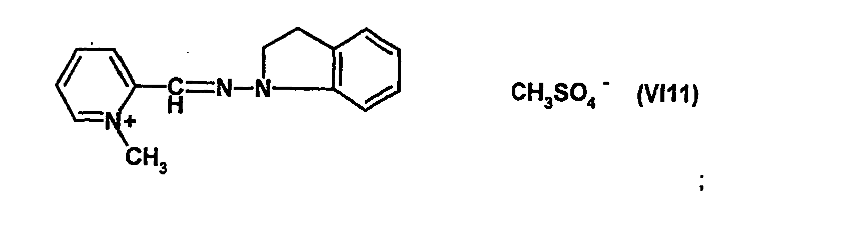

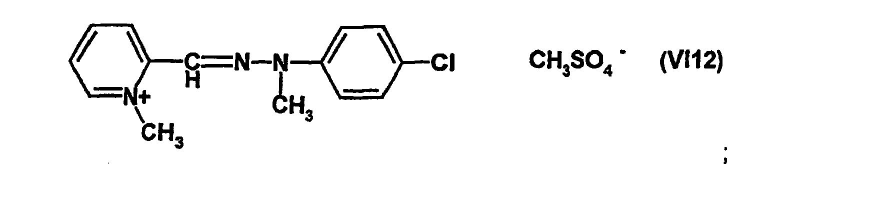

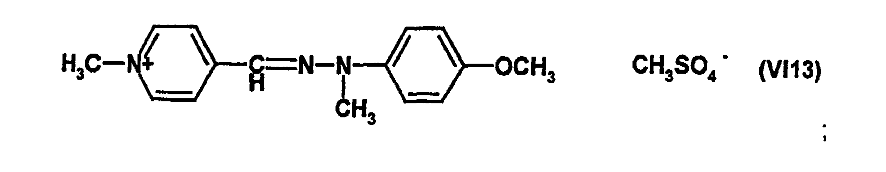

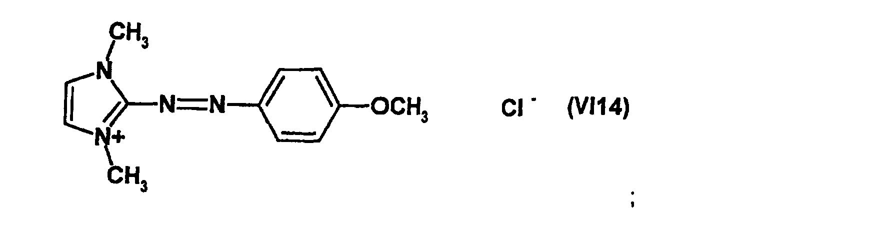

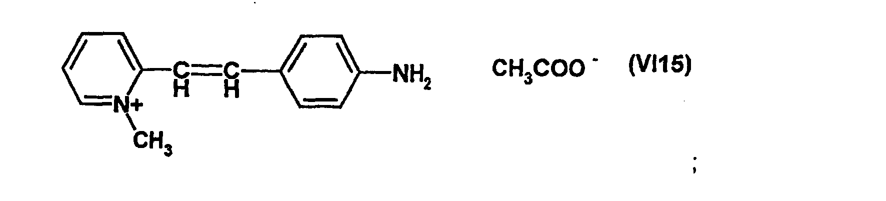

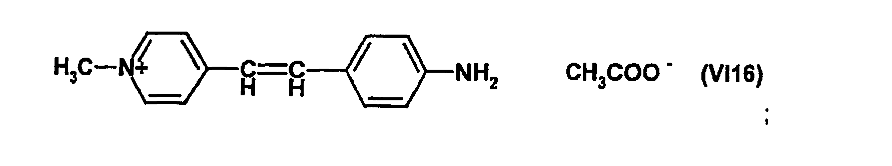

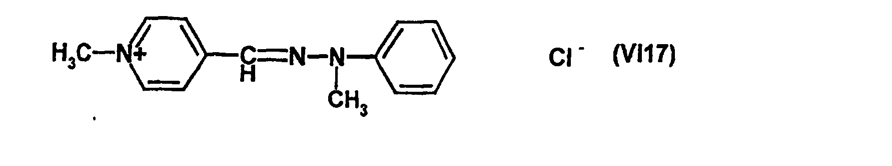

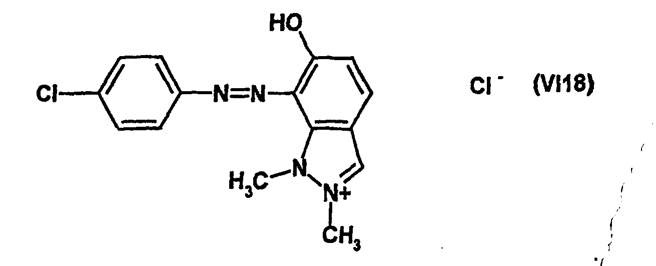

6. Verwendung nach Anspruch 5, **dadurch gekennzeichnet, dass** die kationischen Farbstoffe der Formel (VI) den Strukturen (VI4), (VI5) und (VI13) entsprechen.

7. Zusammensetzung für die Direktfärbung von Keratinfasern und insbesondere menschlichen Keratinfasern, wie Haaren, die keinen selbstoxidierfähigen Farbstoff enthält, **dadurch gekennzeichnet, dass** sie in einem zum Färben geeigneten Medium eine Kombination aus mindestens einem Farbstoff der Formeln (I) bis (III) und mindestens einem Farbstoff der Formeln (IV) oder (VI) enthält.

8. Zusammensetzung nach Anspruch 7, **dadurch gekennzeichnet, dass** der oder die kationische(n) Farbstoff(e) der Formeln (I), (II), (III) in einer von Konzentration 0,001 bis 10 Gew.-% des Gesamtgewichts der Zusammensetzung enthalten sind.

9. Zusammensetzung nach Anspruch 8, **dadurch gekennzeichnet, dass** der oder die kationische(n) Farbstoff(e) der Formeln (I), (II), (III) in einer von Konzentration 0,005 bis 5 Gew.-% des Gesamtgewichts der Zusammensetzung enthalten sind.

10. Zusammensetzung nach Anspruch 7, **dadurch gekennzeichnet, dass** der oder die kationische(n) Farbstoff(e) der Formeln (IV), (VI) 0,001 bis 10 Gew.-% des Gesamtgewichts der Zusammensetzung ausmachen.

11. Zusammensetzung nach Anspruch 10, **dadurch gekennzeichnet, dass** der oder die kationische(n) Farbstoff(e) der Formeln (IV), (VI) 0,001 bis 10 Gew.-% des Gesamtgewichts der Zusammensetzung ausmachen.

12. Zusammensetzung nach einem der Ansprüche 7 bis 11, **dadurch gekennzeichnet, dass** das zum Färben geeignete Medium (oder der Träger) aus Wasser oder einem Gemisch von Wasser und mindestens einem organischen Lösungsmittel besteht.

13. Zusammensetzung nach einem der Ansprüche 7 bis 12, **dadurch gekennzeichnet, dass** sie einen pH-Wert im Bereich von 2 bis 11 und vorzugsweise 5 bis 10 aufweist.

14. Zusammensetzung nach einem der Ansprüche 7 bis 13, **dadurch gekennzeichnet, dass** sie Zusatzstoffe enthält, die unter den Antioxidantien, Penetrationsmitteln, Maskierungsmitteln, Verdickungsmitteln, Parfums, Puffern, Dispergiermitteln, grenzflächenaktiven Stoffen, Polymeren, Filmbildnern, Ceramiden, Konservierungsmitteln, Filtern und Trübungsmitteln ausgewählt sind.

15. Verfahren zum direkten Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 7 bis 14 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

16. Verfahren zum direkten Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 7 bis 14 während einer Zeitspanne, die zur Bildung der gewünschten Färbung ausreichend ist, aufgebracht wird, ohne dass nochmals gespült wird.

17. Vorrichtung mit mehreren Abteilungen oder "Kit" zum Färben mit mehreren Abteilungen, **dadurch gekennzeichnet, dass** eine erste Abteilung mindestens einen in Anspruch 1 definierten kationischen Farbstoff (I) bis (III) und eine andere Abteilung mindestens einen in den Ansprüchen 1 und 3 bis 6 definierten kationischen Farbstoff der Formeln (IV) oder (VI) enthält.

18. Vorrichtung mit mehreren Abteilungen oder "Kit" zum Färben mit mehreren Abteilungen, **dadurch gekennzeichnet, dass** eine erste Abteilung eine pulverförmige Zusammensetzung enthält, die die Kombination aus mindestens einem in Anspruch 1 definierten kationischen Farbstoff (I) bis (III) und mindestens einem in den Ansprüchen 1 und 3 bis 6 definierten kationischen Farbstoff der Formeln (IV) oder (VI) enthält, und eine andere Abteilung eine wässrige Zusammensetzung enthält, die als Farbmittelträger dient.
